(19) Europäisches Patentamt
European Patent Office
Office européen des brevets

(11) **EP 3 423 080 B1**

(12) **EUROPEAN PATENT SPECIFICATION**

(45) Date of publication and mention
of the grant of the patent:
**24.01.2024 Bulletin 2024/04**

(51) International Patent Classification (IPC):
*A61K 38/17* [(2006.01)]   *A61P 25/28* [(2006.01)]
*A61P 25/08* [(2006.01)]   *A61P 9/10* [(2006.01)]
*A61K 38/45* [(2006.01)]   *A61P 9/00* [(2006.01)]
*A61P 25/00* [(2006.01)]

(21) Application number: **17759009.8**

(22) Date of filing: **01.03.2017**

(52) Cooperative Patent Classification (CPC):
**A61K 38/1716; A61K 38/45; A61P 9/00;**
**A61P 9/10; A61P 25/00; A61P 25/08; A61P 25/28;**
**C12Y 207/11024**

(86) International application number:
**PCT/AU2017/050180**

(87) International publication number:
**WO 2017/147654 (08.09.2017 Gazette 2017/36)**

(54) **PHOSPHORYLATED TAU AND P38GAMMA FOR TREATING A NEUROLOGICAL CONDITION**

PHOSPHORYLIERTEM TAU UND P38GAMMA ZUR BEHANDLUNG NEUROLOGISCHER
ERKRANKUNGEN

TAU PHOSPHORYLÉE ET DE P38 GAMMA POUR TRAITER UNE AFFECTION NEUROLOGIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.03.2016 AU 2016900764**

(43) Date of publication of application:
**09.01.2019 Bulletin 2019/02**

(73) Proprietor: **Macquarie University**
**New South Wales 2109 (AU)**

(72) Inventors:
• **ITTNER, Lars Matthias**
**Chatswood, New South Wales 2067 (AU)**
• **ITTNER, Arne Anselm**
**Randwick, New South Wales 2031 (AU)**

(74) Representative: **Nederlandsch Octrooibureau**
**P.O. Box 29720**
**2502 LS The Hague (NL)**

(56) References cited:
WO-A1-2008/124066    WO-A1-2013/086583
WO-A2-2007/062167    WO-A2-2007/094867
WO-A2-2012/009442

• ITTNER ARNE ET AL: "Site-specific
phosphorylation of tau inhibits amyloid-[beta]
toxicity in Alzheimer's mice.", SCIENCE (NEW
YORK, N.Y.) 18 11 2016, vol. 354, no. 6314, 18
November 2016 (2016-11-18), pages 904-908,
XP002794161, ISSN: 1095-9203
• BUEE-SCHERRE, V. et al.: "Phosphorylation of
microtubule-associated protein tau by
stress-activated protein kinases in intact cells",
FEBS Letters, vol. 515, 2002, pages 151-154,
XP004347759,
• GOEDERT, M. et al.: "Phosphorylation of
microtubule-associated protein tau by stress-
activated protein kinases", FEBS Letters, vol.
409, 1997, pages 57-62, XP000906954,
• SABIO, G. et al.: "Stress- and mitogen-induced
phosphorylation of the synapse- associated
protein SAP90/PSD-95 by activation of
SAPK3/p38y and ERKI/ERK2", Biochem. J., vol.
380, 2004, pages 19-30, XP003008876,
• LONG, D. et al.: "p38g mitogen-activated protein
kinase suppresses chondrocyte production of
MMP-13 in response to catabolic stimulation",
Osteoarthritis and Cartilage, vol. 18, 2010, pages
1203-1210, XP027255886,

- RANKIN, C. et al.: "Pseudo-phosphorylation of tau at Ser202 and Thr205 affects tau filament formation", Molecular Brain Research, vol. 138, 2005, pages 84-93, XP027784716,
- JEGANATHAN, S. et al.: "Proline-directed Pseudo-phosphorylation at AT 8 and PHF1 Epitopes Induces a Compaction of the Paperclip Folding of Tau and Generates a Pathological ( MC -1) Conformation", The Journal of Biological Chemistry, vol. 283, no. 46, 14 November 2008 (2008-11-14), pages 32066-32076, XP055575942, DOI: 10.1074/jbc.M805300200
- POOLER, A. et al.: "Dynamic association of tau with neuronal membranes is regulated by phosphorylation", Neurobiology of Aging, vol. 33, 2012, pages 431.e27-431.e38, XP028397295,
- SUN, Q. et al.: "Pseudo-hyperphosphorylation causing AD-like changes in tau has significant effects on its polymerization", Biochemistry, vol. 48, no. 25, 2009, pages 6002-6011, XP055412283,
- TENREIRO, S. et al.: "Protein phosphorylation in neurodegeneration: friend or foe?", Frontiers in Molecular Neuroscience, vol. 7, 2014, pages 1-30, XP055412284,
- ITTNER, L. et al.: "Dendritic function of tau mediates amyloid-beta toxicity in Alzheimer's disease mouse models", Cell, vol. 142, no. 3, 2010, pages 387-97, XP028931189,

Remarks:
    The complete document including Reference Table(s) and the Sequence Listing(s) can be downloaded from the EPO website

## Description

### Field

The present invention relates to an agent which elevates p38γ activity, or the activity of a variant of p38γ, in neurons of a subject, for use in treating or preventing a neurological condition mediated by a tau-dependent signalling complex in neurons of a subject, to a vector for treating or preventing such neurological conditions, and to compositions comprising a vector for treating such conditions. The invention is defined by the claims.

### Background

Excitotoxicity of neurons is a pathological process by which neurons are damaged or killed by excessive stimulation. Such stimulation occurs when glutamatergic receptors, such as, for example, NMDA-type receptors (NR), are overactivated by neurotransmitters such as, for example, glutamic acid. Excitotoxicity can also be induced by excitotoxins such as amyloid-β (Aβ).

Excitotoxicity is believed to play a prominent role in neurological conditions such as various forms of neurodegenerative disease including Alzheimer's disease (AD), frontotemporal dementia, Huntington's disease, Parkinson's disease. Excitotoxicity is also associated with epilepsy, and neuronal damage which occurs following stroke.

Alzheimer's disease (AD) is the most prevalent form of dementia and is the most common neurodegenerative disease. AD is estimated to affect as many as 1% of adults 60 years of age and over.

AD is characterised by brain atrophy, neural loss, extracellular Aβ plaques, and intracellular neurofibrillary tangle (NFTs) containing aberrantly phosphorylated tau.

Tau is an axonal protein that, under non-pathological conditions, regulates microtubule stability and microtubule dependent processes. Tau has also been found to reside in a post-synaptic signalling complex that mediates Aβ-induced excitotoxicity, and potentially other excitotoxicity. In AD, tau becomes aberrantly phosphorylated, and accumulates in the somatodendritic compartments of neurons, aggregates and eventually forms neurofibrilar tangles (NFT). Progression of NFT pathology throughout the brain correlates with disease progression in Alzheimer's disease.

The prevailing theory in AD is that Aβ triggers toxic events including tau phosphorylation causing neuronal dysfunction and death. In support, depleting tau prevents Aβ toxicity in AD mouse and cell culture models. Aβ-toxicity in AD is therefore considered in the art to be mediated by phosphorylated tau in the pathogenesis of AD.

It would be advantageous to provide alternative methods of treating AD and other neurological conditions.

### Summary

The invention is defined by the claims. The inventors have found that, contrary to the teaching in the art, phosphorylation of tau at particular amino acid residues causes disruption of tau-dependent signalling complexes, and prevents or reduces excitotoxicity and Aβ-induced toxicity.

### Description of the Drawings

**Figure 1A** is a schematic diagram showing the domain structure of p38 MAP kinases including a dendrogram showing degree of similarity. As can be seen, *p38γ* has a unique C-terminal PDZ interaction motif.

**Figure 1B** shows the results of polymerase chain reaction (PCR) on genomic DNA from mice with targeted alleles for *p38α, p38β, p38γ* and *p38δ.* f, floxed allele, -, knockout allele, +, wild-type allele.

**Figure 1C** shows the results of western blots of cortical extracts of control mice (f/f or +/+) confirmed expression of *p38α,* p38β and p38γ, but not p38δ in brains. Antibody specificity was shown by probing extracts of mice with individual knockout or p38 MAPKs. Δneu, neuron-specific knockout of p38α. GAPDH showed equal loading. BM, bone marrow.

**Figure 2A** are graphs showing reduced seizure latency (left) and linear regression slopes (right) of p38γ^+/+ and p38γ^-/- mice injected with 30mg/kg PTZ. Mean seizure severity was markedly increased in *p38γ*^-/- compared to *p38γ*^+/+ mice injected with 30mg/kg PTZ (**$P < 0.01$; ****$P < 0.0001$; n=10-12).

**Figure 2B** are photographs showing co-localization of p38γ and post-synaptic PSD-95 (arrows), but not pre-synaptic synaptophysin (Syp) in neurons. Scale bar, 1pm.

**Figure 2C** is a graph showing early mortality in APP23.*p38γ*^+/+ (n=62) was further augmented in APP23.*p38γ*^-/- (n=43) mice, while *p38γ*^+/+ (n=49) and *p38γ*^-/- (n=48) mice presented with normal survival (****$P < 0.0001$, ***$P < 0.001$).

Figures 2D-F show the spatial working memory deficits in APP23.$p38\gamma^{+/+}$ (n=10), and more so APP23.$p38\gamma^{-/-}$ (n=8) compared to $p38\gamma^{+/+}$ (n=10) and $p38\gamma^{-/-}$ (n=10) mice using Morris-water-maze (MWM) (**$P < 0.01$; *$P < 0.05$).

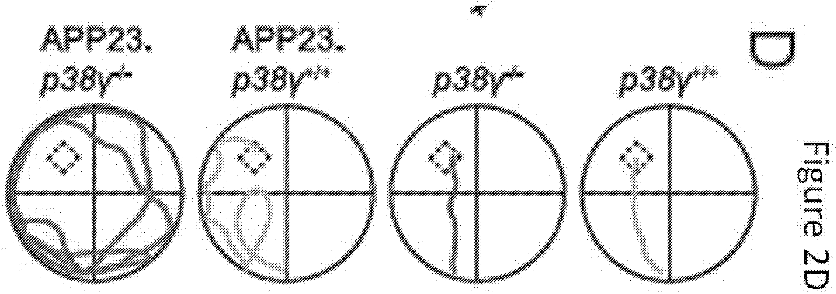

**Figure 2D** is representative MWM path traces. Dashed squares, location of hidden platform.

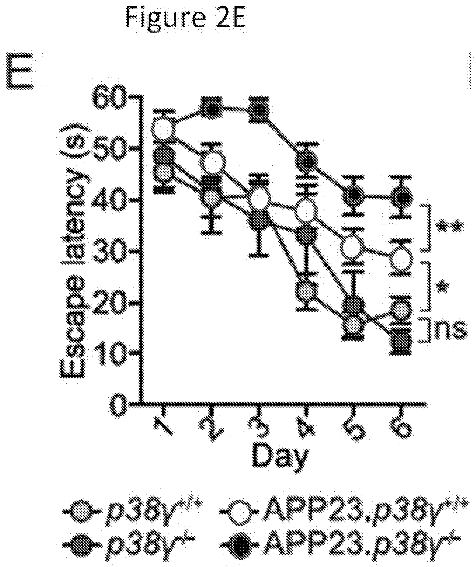

**Figure 2E** is a graph showing escape latency was increased in APP23.$p38\gamma^{+/+}$, and more so in APP23.$p38\gamma^{-/-}$ mice, but comparable to $p38\gamma^{+/+}$ and $p38\gamma^{-/-}$ mice.

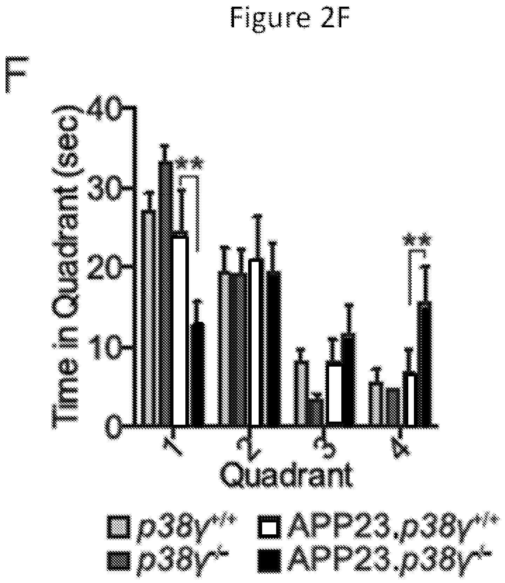

**Figure 2F** is a graph showing the time in quadrant (seconds) in a MWM test of p38$\gamma^{+/+}$, p38$\gamma^{-/-}$, APP23p38$\gamma^{+/+}$ and APP23p38$\gamma^{-/-}$ mice. APP23.$p38\gamma^{-/-}$ mice spent less time in the targeted (Q1) and more time in the opposite quadrant (Q4) during probe trials, compared to APP23.$p38\gamma^{+/+}$, $p38\gamma^{+/+}$ and $p38\gamma^{-/-}$ mice.

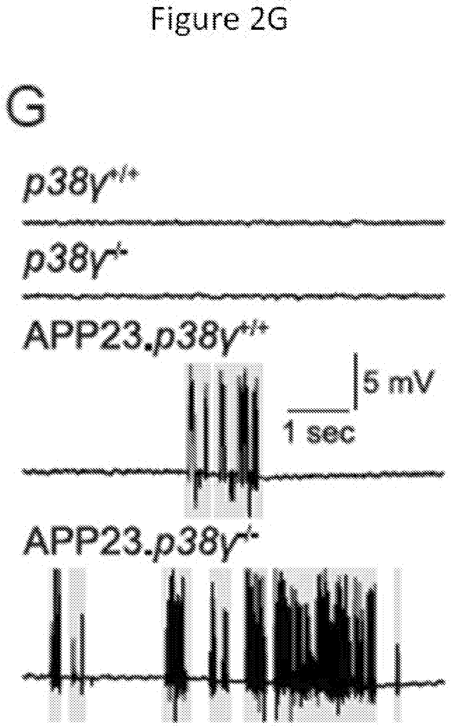

**Figure 2G** shows representative EEG traces of APP23.$p38\gamma^{+/+}$, APP23.$p38\gamma^{-/-}$, $p38\gamma^{+/+}$ and $p38\gamma^{-/-}$ mice, with bouts of hypersynchronicity (green) in APP23.$p38\gamma^{+/+}$ and APP23.$p38\gamma^{-/-}$, but not $p38\gamma^{+/+}$ and $p38\gamma^{-/-}$ mice.

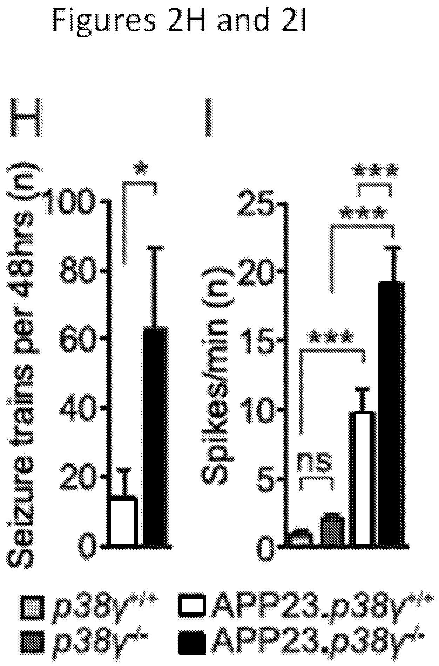

**Figure 2H** is a graph showing markedly increased numbers of spike trains in APP23.$p38\gamma^{-/-}$ compared to APP23.$p38\gamma^{+/+}$ mice (n=6-8; **$P < 0.01$). No spike trains were detected in $p38\gamma^{+/+}$ and $p38\gamma^{-/-}$ recordings.

**Figure 2I** is a graph showing the number of spikes per minute was increased in APP23.$p38\gamma^{-/-}$ compared to APP23.$p38\gamma^{+/+}$ mice, but rare in $p38\gamma^{+/+}$ and $p38\gamma^{-/-}$ mice (n=6-8; ***$P < 0.001$ **$P < 0.01$, *$P < 0.05$).

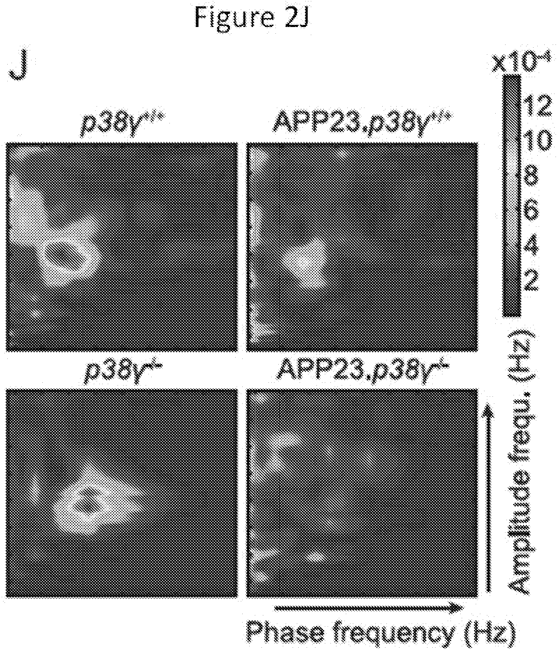

**Figure 2J** shows a representative phase-amplitude comodulograms computed for interictal hippocampal LFPs recordings showing reduced cross-frequency coupling (CFC) around 8 Hz in APP23.$p38\gamma^{+/+}$ compared to $p38\gamma^{+/+}$ and $p38\gamma^{-/-}$ mice. CFC at ~8Hz was virtually lost in APP23.$p38\gamma^{-/-}$ mice.

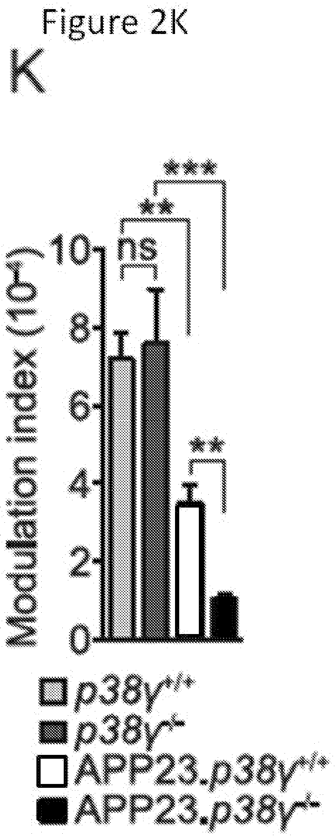

**Figure 2K** is a graph showing the modulation index computed for phase-amplitude distributions was reduced in APP23.$p38\gamma^{+/+}$ (n=8) and more so in APP23.$p38\gamma^{-/-}$ (n=8) compared to $p38\gamma^{+/+}$ (n=6) and $p38\gamma^{-/-}$ (n=6) mice (***$P < 0.001$, **$P < 0.01$).

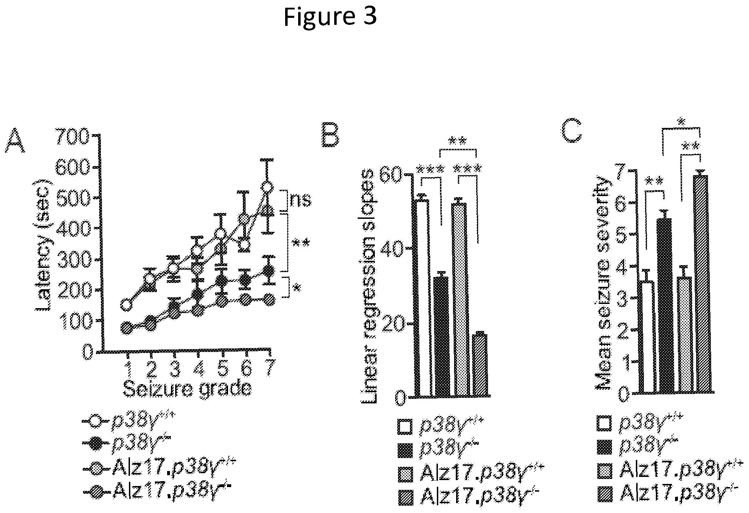

**Figure 3A** is a graph showing seizure latencies in p38$\gamma^{+/+}$, p38$\gamma^{-/-}$, Alz17.p38$\gamma^{+/+}$ and Alz17.p38$\gamma^{-/-}$ mice following i.p. administration of 30 mg/kg PTZ. Further reduction in seizure latencies following 30 mg/kg PTZ i.p. was observed in Alz17.$p38\gamma^{-/-}$ mice compared to those already reduced in $p38\gamma^{-/-}$ compared to $p38\gamma^{+/+}$ and Alz17.$p38\gamma^{+/+}$ mice (n=10-12; **$P < 0.01$; *$P < 0.05$; ns, not significant).

**Figure 3B** is a graph showing linear regression analysis of seizure latency curves in (3A) (n=10-12; ***$P < 0.001$; **$P < 0.01$).

**Figure 3C** is a graph showing further enhanced mean seizure severity after 30 mg/kg PTZ in Alz17.$p38\gamma^{-/-}$ mice compared to those already increased in $p38\gamma^{-/-}$ compared to $p38\gamma^{+/+}$ and Alz17.$p38\gamma^{+/+}$ mice (n=10-12; ***$P < 0.001$, **$P < 0.01$, *$P < 0.05$).

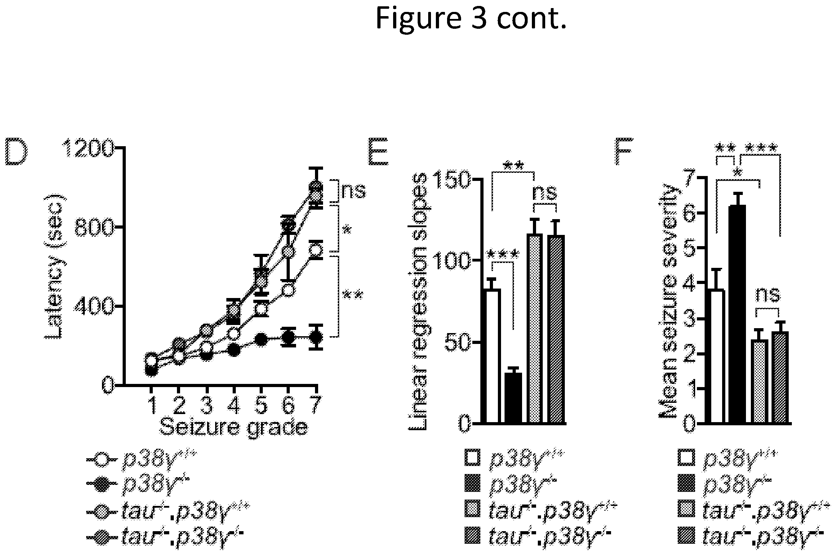

**Figure 3D** is a graph showing seizure latencies after 30mg/kg PTZ were profoundly reduced in $tau^{+/+}.p38\gamma^{-/-}$ compared to $tau^{+/+}.p38\gamma^{+/+}$ mice, and were markedly increased in both $tau^{-/-}.p38\gamma^{+/+}$ and $tau^{-/-}.p38\gamma^{-/-}$ mice (n=10-12; **$P < 0.01$; *$P < 0.05$).

**Figure 3E** is a graph showing a linear regression analysis of seizure latency curves in (D) (n=10-12; ***$P < 0.001$; **$P < 0.01$; ns, not significant)

**Figure 3F** is a graph showing mean seizure severity was increased in $tau^{+/+}.p38\gamma^{-/-}$ compared to $tau^{+/+}.p38\gamma^{+/+}$ mice, but was similarly reduced in $tau^{-/-}.p38\gamma^{+/+}$ and $tau^{-/-}.p38\gamma^{-/-}$ mice after 30 mg/kg PTZ injection (n=10-1; ***$P < 0.001$; **$P < 0.01$; *$P < 0.05$).

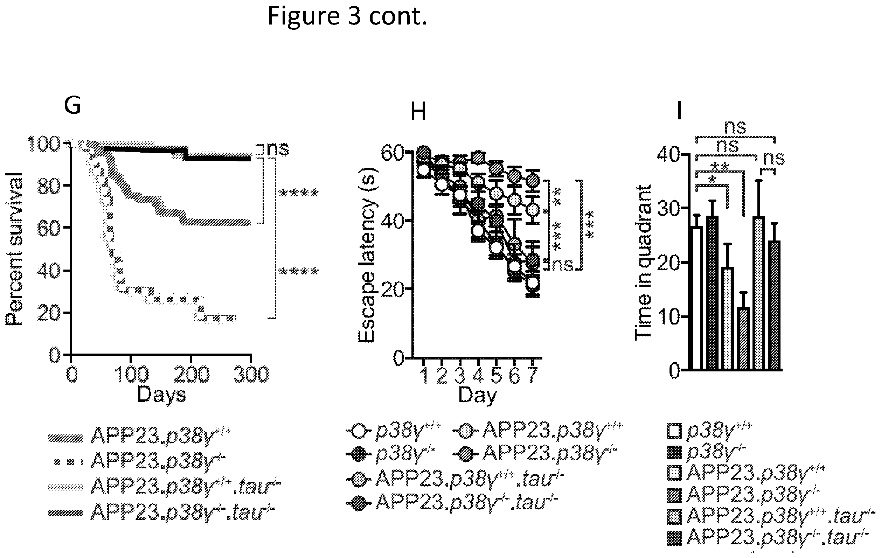

**Figure 3G** is a graph showing percent survival of APP23.p38$\gamma^{-/-}$.tau$^{-/-}$ mice compared with APP23.p38$\gamma^{+/+}$tau$^{-/-}$, APP23.p38$\gamma^{+/+}$ and APP23.p38$\gamma^{-/-}$ mice over 300 days.

**Figure 3H** is a graph showing escape latency of p38$\gamma^{+/+}$, p38$\gamma^{-/-}$,APP23.$p38\gamma^{+/+}$, APP23.p38$\gamma^{-/-}$, APP23.p38$\gamma^{+/+}$.tau$^{-/-}$ and APP23.$p38\gamma^{-/-}$.tau$^{-/-}$ mice following Morris Water Maze (MWM) test.

**Figure 3I** is a graph showing time in quadrant during MWM test for $p38\gamma^{+/+}$, p38$\gamma^{-/-}$,APP23.$p38\gamma^{+/+}$, APP23.$p38\gamma^{-/-}$, APP23.p38$\gamma^{+/+}$.tau$^{-/-}$ and APP23.$p38\gamma^{-/-}$.tau$^{-/-}$ mice.

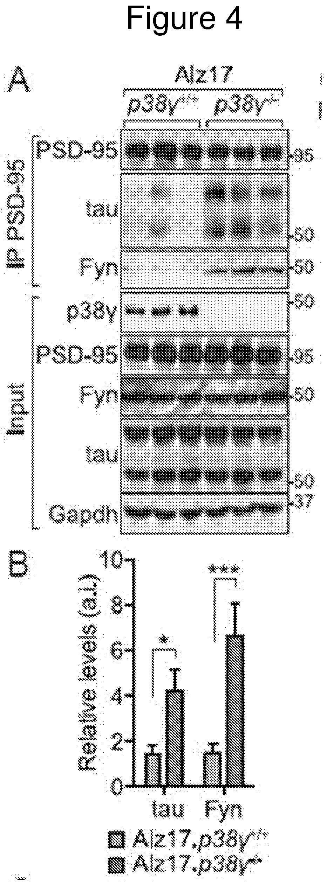

**Figure 4A** is a photograph showing that more PSD-95/tau/Fyn complexes were immunoprecipitated from Alz17.$p38\gamma^{-/-}$ than Alz17.$p38\gamma^{+/+}$ brains, despite comparable total levels of PSD-95, tau and Fyn. GAPDH confirmed equal loading.

**Figure 4B** is a graph showing quantification of tau and Fyn bound to PSD-95 detected in (4A) (n=6; ***$P < 0.001$; *$P < 0.05$).

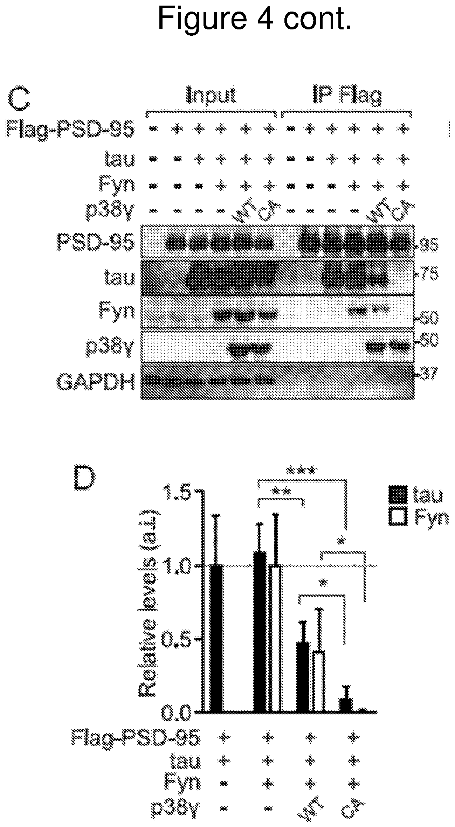

**Figure 4C** is a photograph showing the results of immunoprecipitation (IP) of PSD-95/tau/Fyn complexes from cells transfected with FLAG-PSD-95, tau and Fyn. Co-transfection of wild-type p38$\gamma$ (WT) mitigated, and of constitutive active p38y (CA) abolished, PSD-95/tau/Fyn interaction.

**Figure 4D** is a graph showing quantification of tau and Fyn bound to PSD-95 detected in (C) (n=6; ***$P < 0.001$; **$P < 0.01$; *$P < 0.05$).

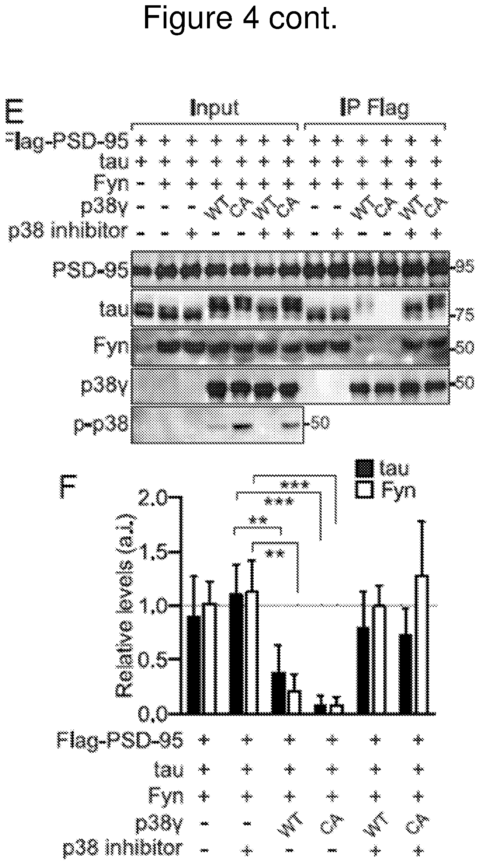

**Figure 4E** shows that p38$\gamma$ WT and CA p38$\gamma$ failed to disrupt PSD-95/tau/Fyn complexes immunoprecipitated from cells in the presence of p38 inhibitor.

**Figure 4F** is a graph showing quantification of tau and Fyn bound to PSD-95 detected in (E) (n=6; ***$P < 0.001$; **$P < 0.01$; *$P < 0.05$).

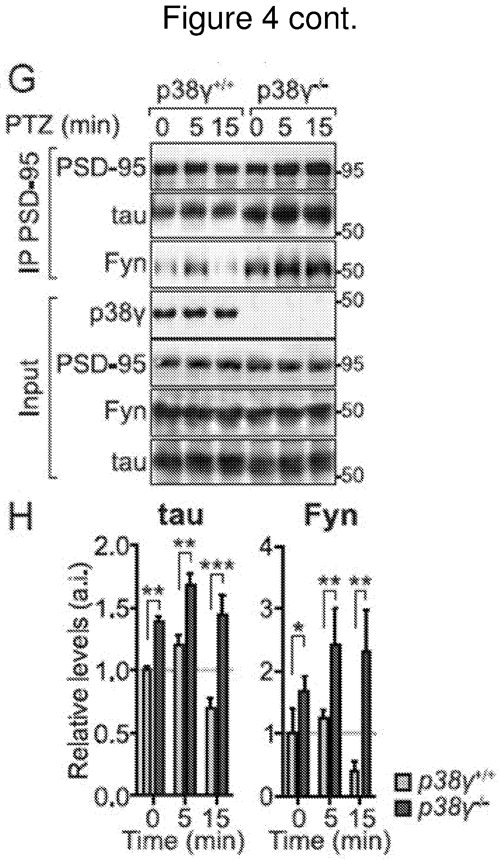

**Figure 4G** shows that consistently more PSD-95/tau/Fyn complexes were immunoprecipitated from cortical lysates of *p38γ^-/-* than *p38γ^+/+* mice 0, 5 and 15 minutes after injection with 30 mg/kg PTZ.

**Figure 4H** is a graph showing quantification of tau and Fyn bound to PSD-95 detected in (4G) (n=6; ***$P < 0.001$; **$P < 0.01$; *$P < 0.05$).

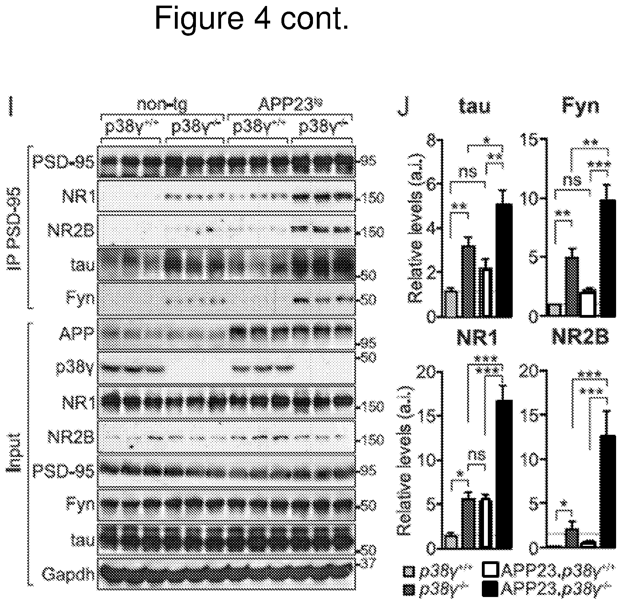

**Figure 4I** shows more tau, Fyn, NMDA receptor subunits 1 (NR1) and 2B (NR2B) we immunoprecipitated in complexes with PSD-95 from brains of *p38γ^-/-* than *p38γ^+/+* mice. This was further enhanced in APP23.*p38γ^-/-* compared to APP23.*p38γ^+/+* mice. Total levels of APP (22C11), PSD-95, tau, Fyn, NR1, NR2B and p38γ were, however, comparable in *p38γ^-/-*, *p38γ^+/+*, APP23.*p38γ^-/-* and APP23.*p38γ^+/+* mice.

**Figure 4J** is a graph showing quantification of tau, Fyn, NR1 and NR2B bound to PSD-95 detected in (4I) (n = 6-8; ***$P < 0.001$; **$P < 0.01$; *$P < 0.05$).

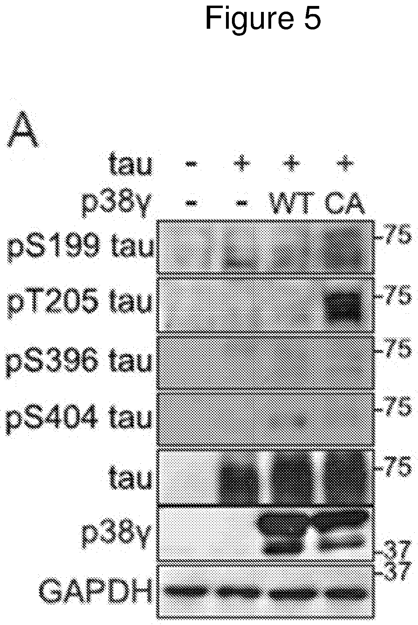

**Figure 5A** shows cells transfected with tau and wild-type (WT) or constitutive active (CA) p38γ are predominantly being phosphorylated at T205 and less at S199, but virtually not at S396 and S404. GAPDH showed equal loading.

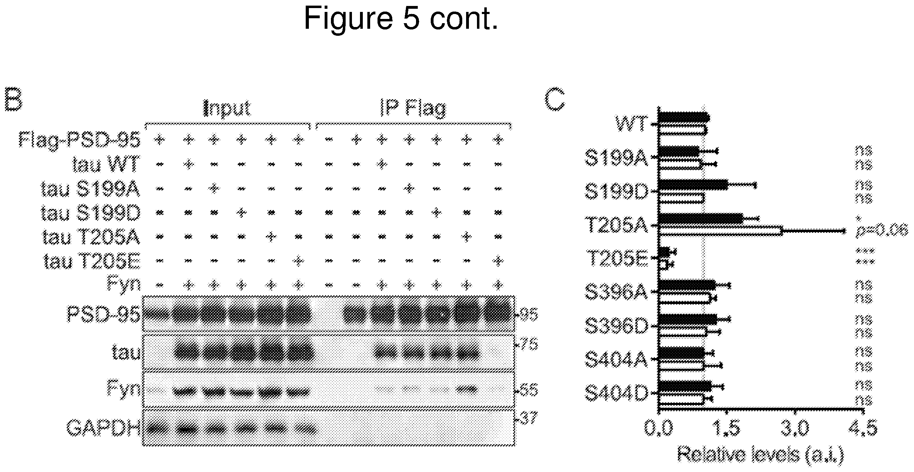

**Figure 5B** shows the results of immunoprecipitation of PSD95/tau/Fyn complexes from cells co-transfected with PSD95, Fyn and wild-type or mutant human tau (S199A, S199D, T205A, T205E). The results show that mimicking phosphorylation at T205 (T205E) quantitatively disrupted the interaction of PSD95, Fyn and tau, while the tau variant T205A increased it. Mutation of S199 had no effect on PSD-95/tau/Fyn complexes.

**Figure 5C** is a graph showing quantification of tau and Fyn bound to PSD-95 detected in (Fig. 5B) (n=6; ***$P < 0.001$; *$P < 0.05$; ns, not significant).

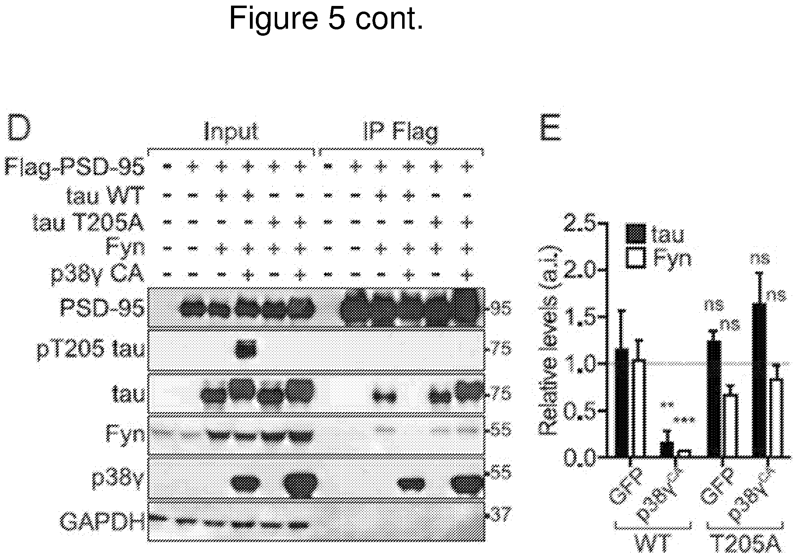

**Figure 5D** shows the results of imnmunoprecipitation of PSD95/tau/Fyn complexes from cells co-transfected with PSD95, Fyn, wild-type or mutant human tau, with or without p38γ^CA. Co-expression of PSD-95, Fyn and WT tau with p38γ^CA abolished PSD-95/tau/Fyn complex formation, while transfection of T205A tau completely prevented the effects of p38γ^CA on PSD-95/T205A tau/Fyn interaction.

**Figure 5E** is a graph showing quantification of tau and Fyn bound to PSD-95 detected in Figure 5D (n=4; ***$P < 0.001$; **$P < 0.01$).

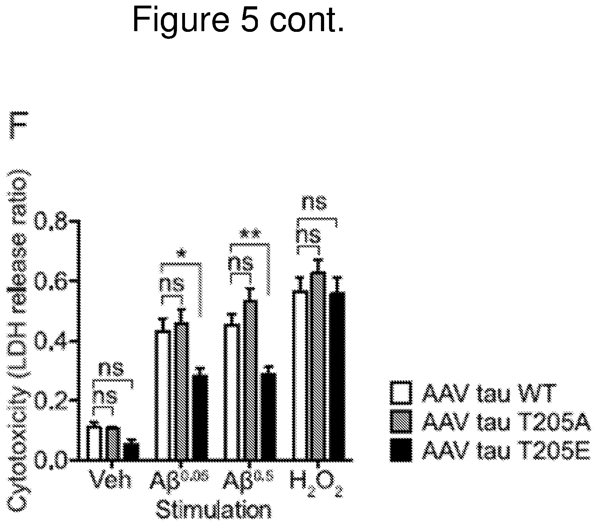

**Figure 5F** is a graph showing the effect of tau variants on Aβ-induced toxicity as determined by LDH release in hippocampal neurons. Aβ (0.05 or 0.5μM)-induced toxicity (measured by LDH release) was reduced in T205E compared to WT and T205A tau-expressing neurons. Cytotoxicity induced by $H_2O_2$ (3uM) was similar for all tau variants. (n=6 independent experiments; **$P < 0.01$; *$P < 0.05$).

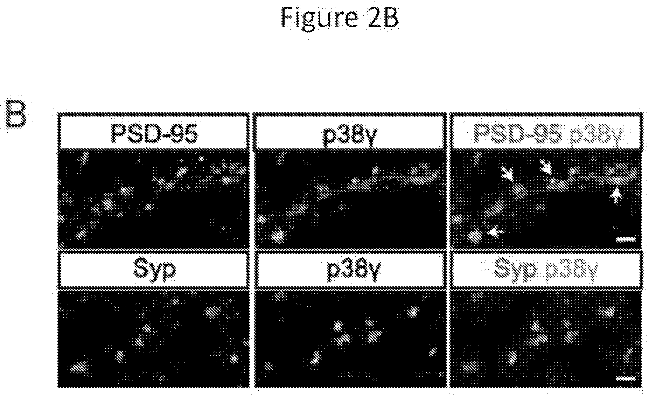

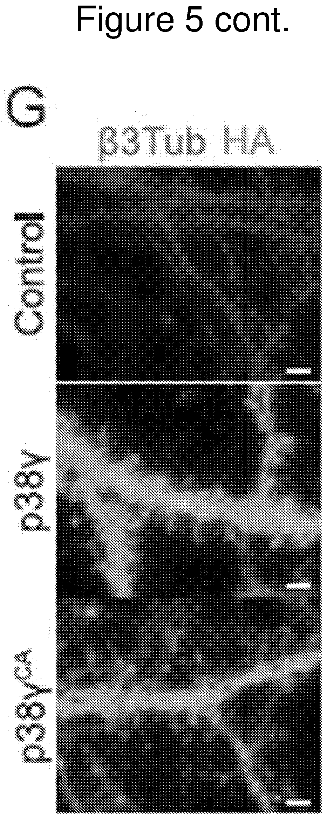

**Figure 5G** is an image showing localization of p38γ and p38γ^CA in cultured hippocampal neurons. Both, AAV-expressed WT and constitutive active (CA) p38γ localized to dendritic spines in cultured hippocampal neurons (β3Tub, β3-tubulin), similar to endogenous p38γ (see Fig. 2B). Control neurons expressed AAV18 GFP. Scale bar, 1 um.

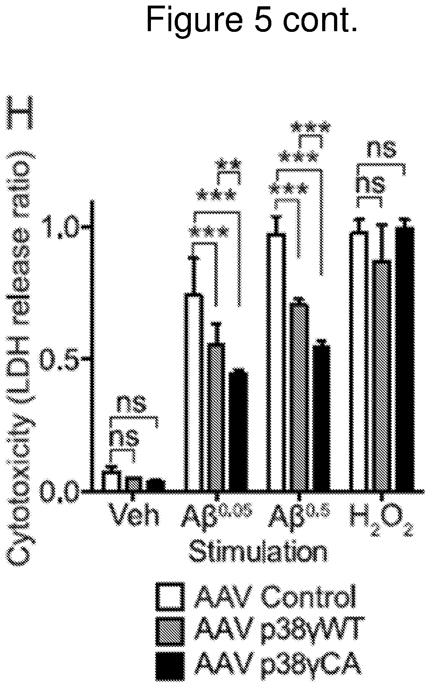

**Figure 5H** is a graph showing expression of p38γ WT and more so of p38γ^CA reduced toxicity induced by Aβ (0.05 or 0.5uM) but not $H_2O_2$ (3μM) in hippocampal neurons, determined by LDH release. (n=6 independent experiments; ***$P < 0.001$; **$P < 0.01$).

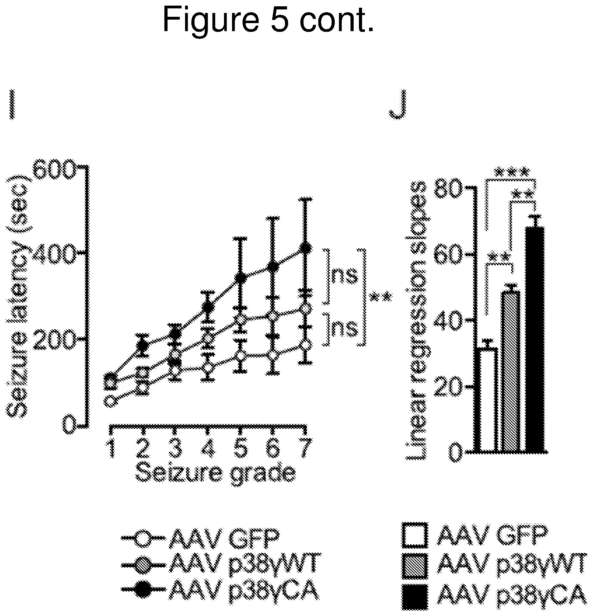

**Figure 5I** is a graph showing expression of p38γ and more so of p38γ^CA in C57B1/6 brains increased seizure latencies after administration of PTZ (50mg/kg i.p.) compared to mice that received AAV-GFP (n=8-10; **$P < 0.01$).

**Figure 5J** is a graph showing the results of linear regression analysis of seizure latency curves in Figure 5I (n=8-10;. ***$P < 0.001$; **$P < 0.01$).

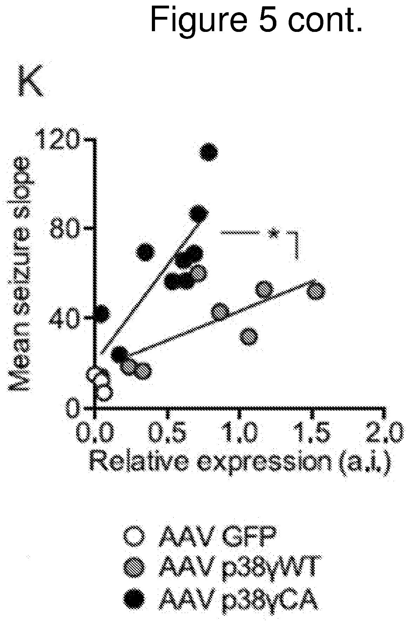

**Figure 5K** is a graph showing the degree of improvement in seizure latencies (linear regression slopes) mediated by expression of both WT and CA p38γ positively correlated with level of p38γ expression in individual mice challenged with 50mg/kg PTZ (n=8-10; *$P < 0.05$).

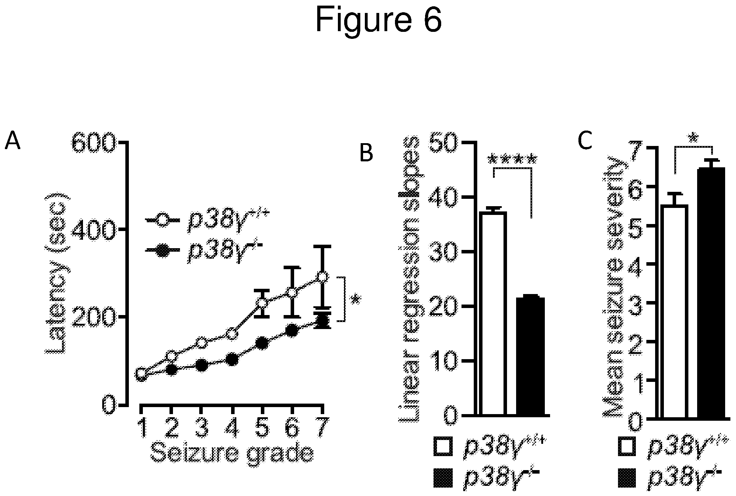

**Figure 6A** is a graph showing the susceptibility of p38γ^-/- knockout mice and p38γ^+/+ control mice to excitotoxic seizures induced following i.p injection of 50mg/kg body weight pentylenetetrazole (PTZ). Seizure latency was reduced in *p38γ* knockout *(p38γ^-/-)* as compared to control *(p38y+/+)* mice following 50mg/kg PTZ (*$P < 0.05$; n=9-10).

**Figure 6B** is a graph showing the results of linear regression of analysis of seizure latency curves in Figure 6A (****$P < 0.0001$; n=9-10).

**Figure 6C** is a graph showing mean seizure severity in p38γ^+/+ and p38γ^-/- mice following i.p. administration of 50 mg/kg PTZ. Mean seizure severity was reduced in p38γ^+/+ mice compared to p38γ^-/- mice (*$P<0.05$) .

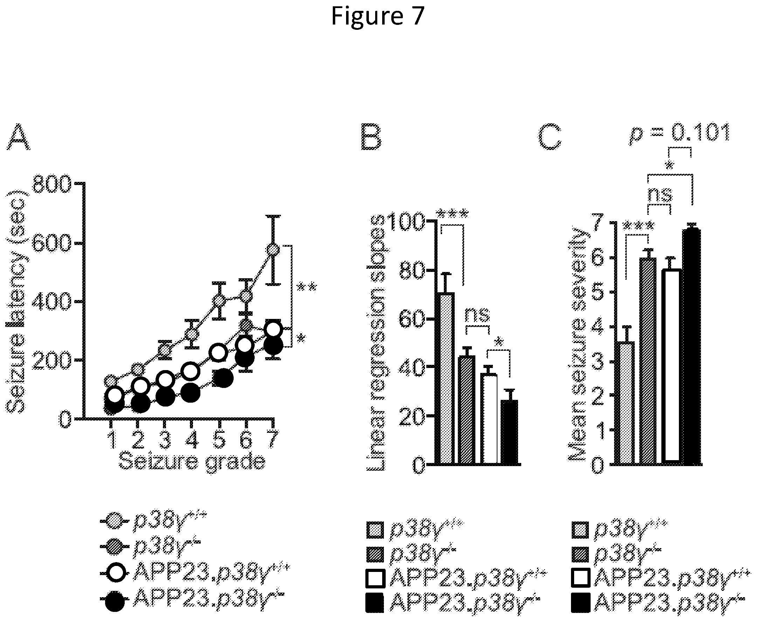

**Figure 7A** is a graph showing the susceptibility of p38γ^-/- knockout APP23 mice and p38γ^+/+ APP23 mice to excitotoxic seizures induced following i.p injection of 30mg/kg body weight PTZ. p38γ^-/- and APP23.p38γ^+/+ presented similar reduced seizure latencies compared to non-transgenic p38γ^+/+ mice after PTZ injection. The seizure latency was even further reduced in APP23 p38γ^-/- mice (n=10-12; **$P < 0.01$; *$P < 0.05$).

**Figure 7B** is a graph showing the results of linear regression analysis of seizure latency curves in Figure 7A (n=10-12; ***$P < 0.001$; *$P < 0.05$).

Figure **7C** is a graph showing mean seizure severity following PTZ administration (30mg/kg BW i.p.). Seizure severity was significantly increased in *p38γ^-/-* and APP23.*p38γ^+/+* compared to non-transgenic *p38γ^+/+* mice (n=10-12; ***$P < 0.001$; *$P < 0.05$). APP23.*p38γ^-/-* mice showed a trend to even further enhanced seizure.

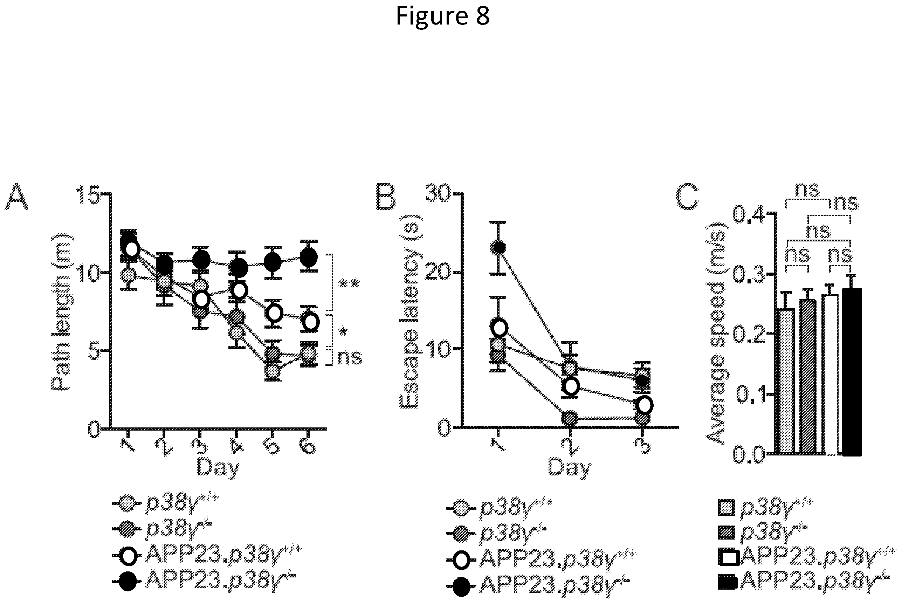

**Figure 8A** is a graph showing the length of swim paths of *p38γ^+/+*, p38γ^-/-, APP23.p38γ^+/+ and APP23.p38γ^-/- *mice* in a Morris-water-maze (MWM) to assess memory impairment. Longer swim paths indicated memory acquisition deficits in *APP23.p38γ$^{+/+}$*, that were worse in APP23.p38γ$^{-/-}$ mice, compared to normal learning in *p38γ$^{+/+}$* and *p38γ$^{-/-}$* mice (**$P < 0.01$; *$P < 0.05$; ns, not significant).

**Figure 8B** is a graph showing escape latencies, and **Figure 8C** is a graph showing average speeds, of p38γ$^{+/+}$, p38γ$^{-/-}$, APP23.p38γ$^{+/+}$ and APP23.p38γ$^{-/-}$ *mice* in the Morris-water-maze (MWM). Escape latencies and average speeds were similar using visual cued platform, confirming visual and motor competency.

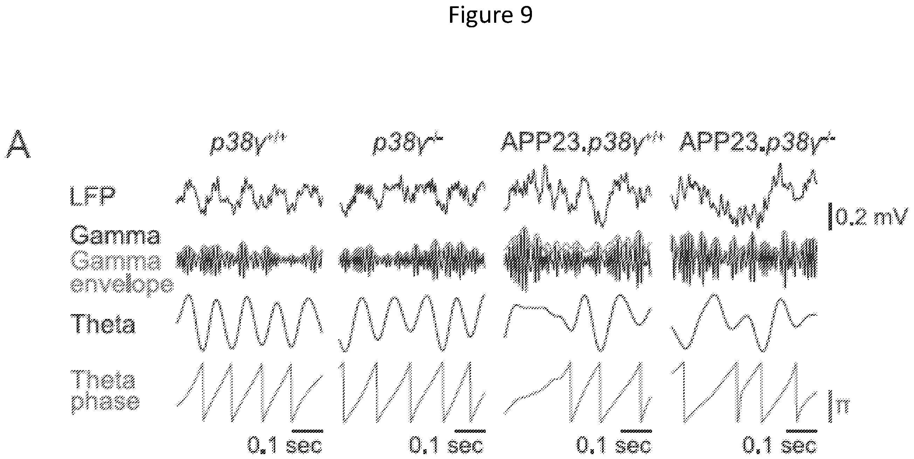

**Figure 9A** is a diagram of a representative raw interictal EEG (LFP), band pass filtered signals for theta (4-12 Hz) and gamma (25-100 Hz) oscillations, gamma amplitude envelope and theta phase in APP23.*p38γ$^{+/+}$* and APP23.*p38γ$^{-/-}$* and non-transgenic control *p38γ$^{+/+}$* and *p38γ$^{-/-}$* mice.

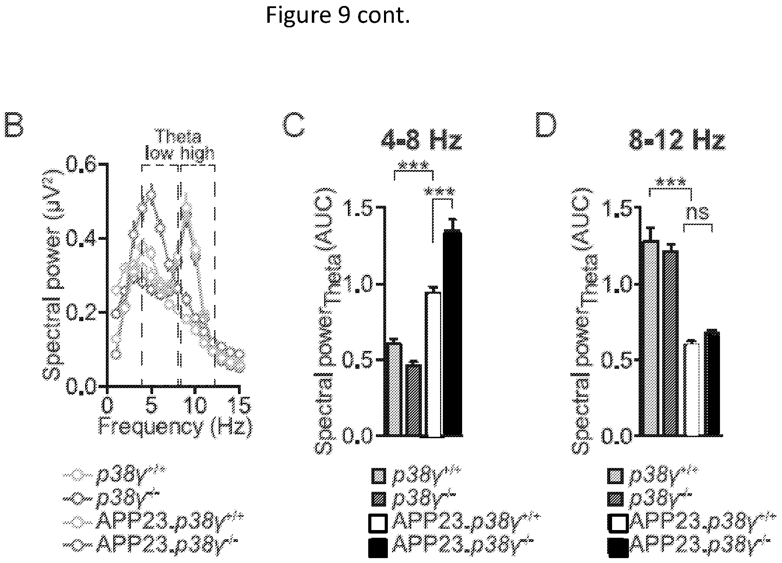

**Figure 9B** is a graph showing spectral power analysis of interictal EEGs showed a shift to lower theta frequencies in APP23.*p38γ$^{+/+}$* and APP23.*p38γ$^{-/-}$* compared to *p38γ$^{+/+}$* and *p38γ$^{-/-}$* recordings (n=6-8). Dashed boxes mark low and high theta bands.

**Figure 9C** is a graph showing quantification (area-under-curve, AUC) of spectral power of low frequency theta (4-8 Hz) in APP23.*p38γ$^{+/+}$* and more so in APP23.*p38γ$^{-/-}$* compared to *p38γ$^{+/+}$* and *p38γ$^{-/-}$* recordings (***$P < 0.001$).

**Figure 9D** is a graph showing that spectral power of high frequency theta power (8-12 Hz) in Figure 9B was decreased in APP23.*p38γ$^{+/+}$* and APP23.*p38γ$^{-/-}$* compared to *p38γ$^{+/+}$* and *p38γ$^{-/-}$* recordings (***$P < 0.001$; ns, not significant). Note that aberrant power of high frequency theta (8-12 Hz) in APP23 mice was not affected by deletion of *p38y.*

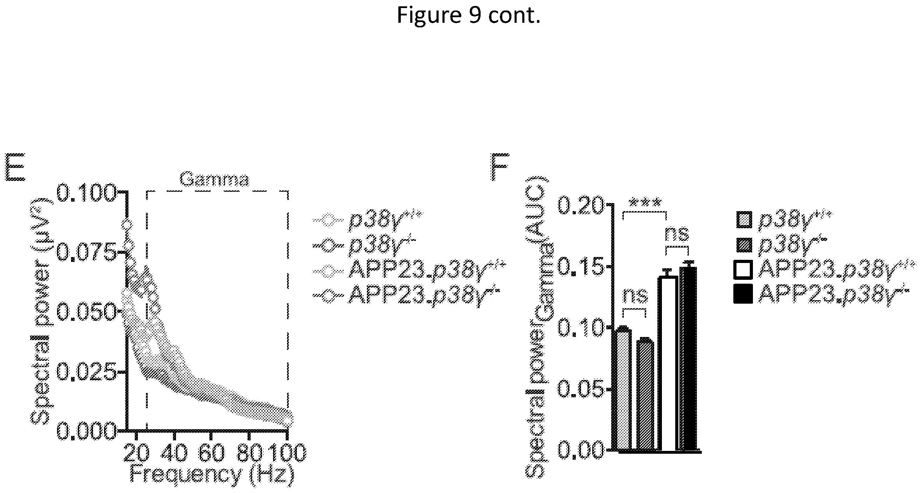

**Figure 9E** is a graph showing gamma spectral power analysis of interictal EEG in APP23.*p38γ$^{+/+}$*, APP23.*p38γ$^{-/-}$*, *p38γ$^{+/+}$* and *p38γ$^{-/-}$* recordings (n=6-8) . Dashed boxes mark gamma band.

**Figure 9F** is a graph showing quantification (AUC) of the graph shown in Figure 9E. The results showed increased spectral power of gamma (25-100 Hz) in APP23.*p38γ$^{+/+}$* and APP23.*p38γ$^{-/-}$* compared to *p38γ$^{+/+}$* and *p38γ$^{-/-}$* recordings (***$P < 0.001$).

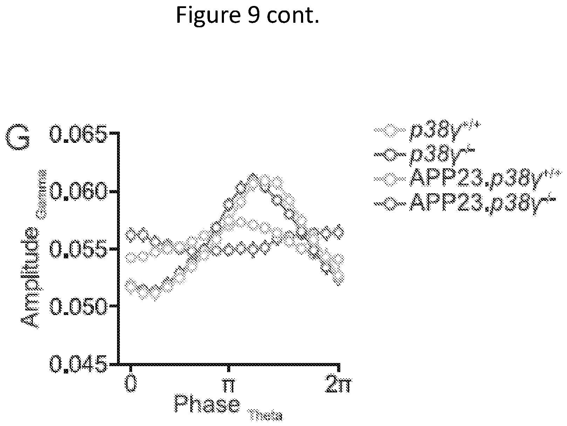

**Figure 9G** is a graph showing phase-amplitude plot computed for interictal hippocampal LFPs recordings showing a reduction in APP23.*p38γ$^{+/+}$* (n=8) and loss in APP23.*p38γ$^{-/-}$* (n=8) of phase-amplitude coupling (CFC) compared to *p38γ$^{+/+}$* (n=6) and *p38y$^{-/-}$* (n=6) mice.

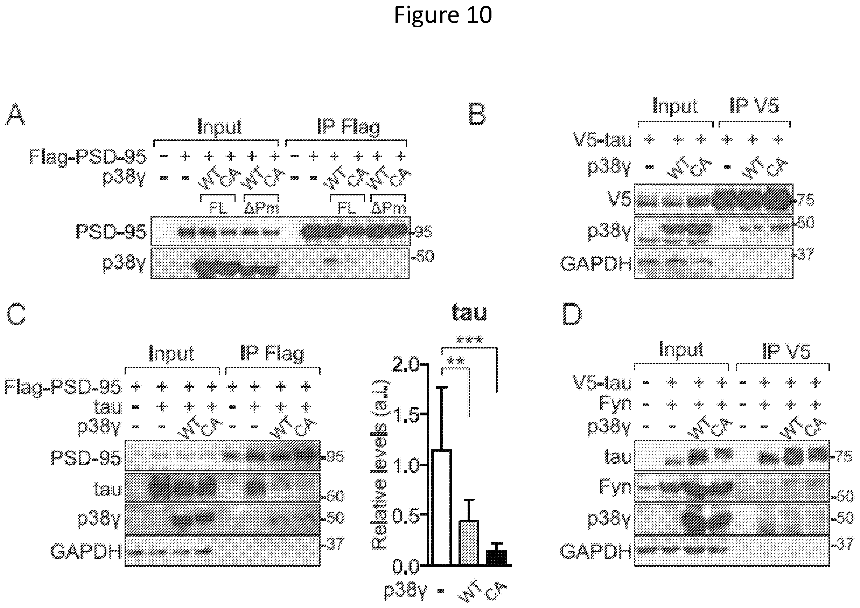

**Figure 10A** shows immunoblots in which both full-length (FL) WT and CA p38γ precipitated together with PSD-95 from cells transfected with PSD-95 and p38γ variants. Notable, deletion of the C-terminal PDZ-binding motif (ΔPm) in both WT and CA p38γ abolished the interaction with PSD-95.

**Figure 10B** shows immunoblots in which both WT and CA p38γ precipitated together with tau from cells transfected with V5-tagged tau and p38γ variants. GAPDH confirmed equal loading.

**Figure 10C** shows immunoprecipitation (IP) of PSD-95/tau complexes from cells transfected with PSD-95 and tau (hTau40). Co-transfection of wild-type p38γ (WT) mitigated and of constitutive active p38γ (CA) abolished PSD-95/tau interaction. GAPDH confirmed equal loading. Figure 10C also shows a graph showing quantification of tau bound to PSD-95 as detected in IPs (n=5; ***$P < 0.001$; **$P < 0.01$).

**Figure 10D** is an immunoblot showing Fyn and both, WT and CA p38γ precipitated together with tau from cells transfected with V5-tagged tau, Fyn and p38γ variants. GAPDH confirmed equal loading.

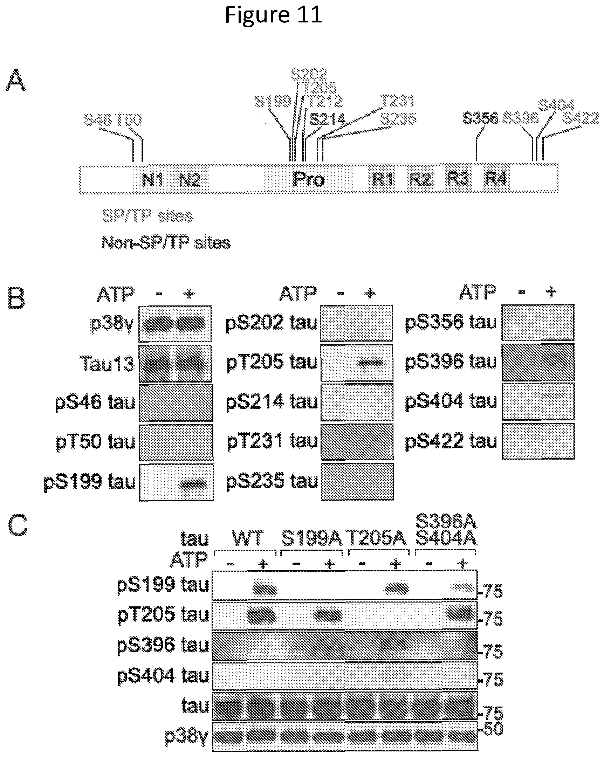

**Figure 11A** is schematic diagram of tau domains and major phosphorylation sites, including non-SP/TP and SP/TP sites. N1/N2: N-terminal inserts encoded by exons 2/3; Pro: proline-rich domain; R1-4: microtubule-binding repeats.

**Figure 11B** is the results of an *in vitro* kinase assay using recombinant tau and p38γ in absence (-) or presence (+) of adenosinetriphosphate (ATP) and followed by immunoblotting for p38y, tau (Tau13) and phosphorylation site specific antibodies showed phosphorylation of tau at S199, T205, S396 and S404, but not other sites tested by p38γ.

**Figure 11C** is the results of an in vitro kinase assay using recombinant wild-type tau or variants with indicated serines/threonines mutated to Alanine and p38γ in absence (-) or presence (+) of ATP which confirmed site-specific phosphorylation of S199, T205, S396 and S404 by p38γ.

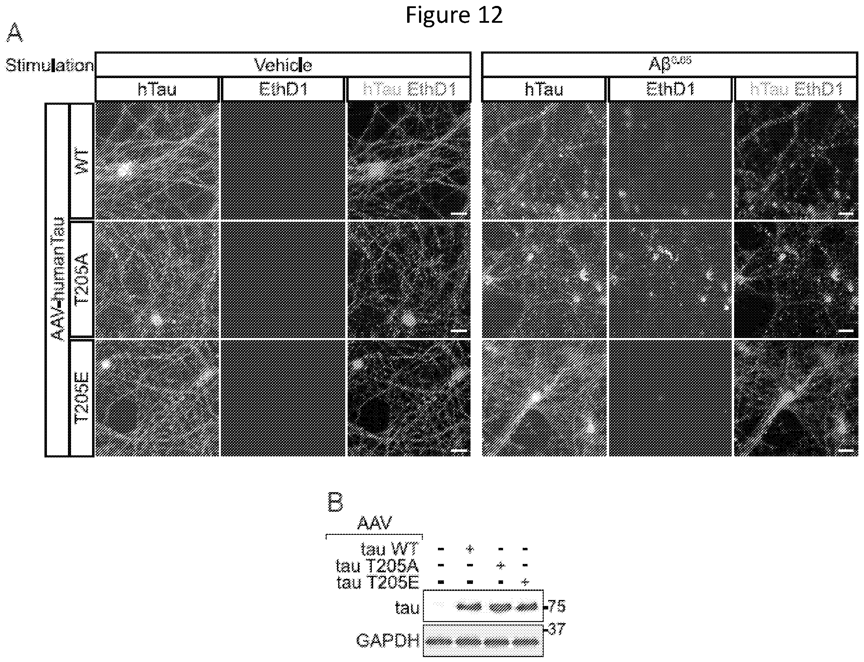

**Figure 12A** shows hippocampal neurons with adeno-associated virus (AAV)-mediated expression of human wildtype (WT), T205A or T205E mutant tau which were exposed to 0.05 μM Aβ42 or vehicle. Cytotoxicity was detected 24 later by EthD1 uptake in WT and T205A, but not T205E tau expressing neurons. Scale bar, 10 μm.

**Figure 12B** shows immunoblots in which similar expression of WT, T205A and T205E tau was observed in hippocampal neurons. GAPDH confirmed equal loading.

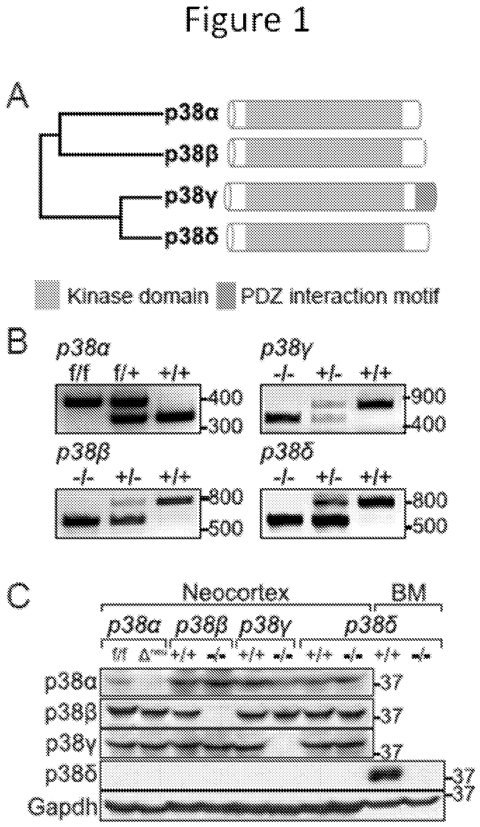

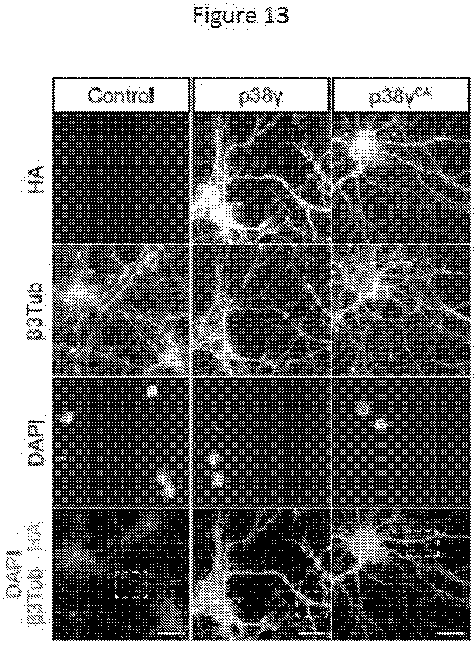

**Figure 13** shows lower magnification of cells shown in Figure 5G: Both, AAV-expressed WT and constitutive active (CA) p38γ localized to dendritic spines in cultured hippocampal neurons (β3Tub, β3-tubulin), similar to endogenous p38γ (see Figure 1). Control neurons expressed AAV-GFP. Scale bar, 10pm. Broken lines indicated optical fields shown at higher magnification in Figure 5G.

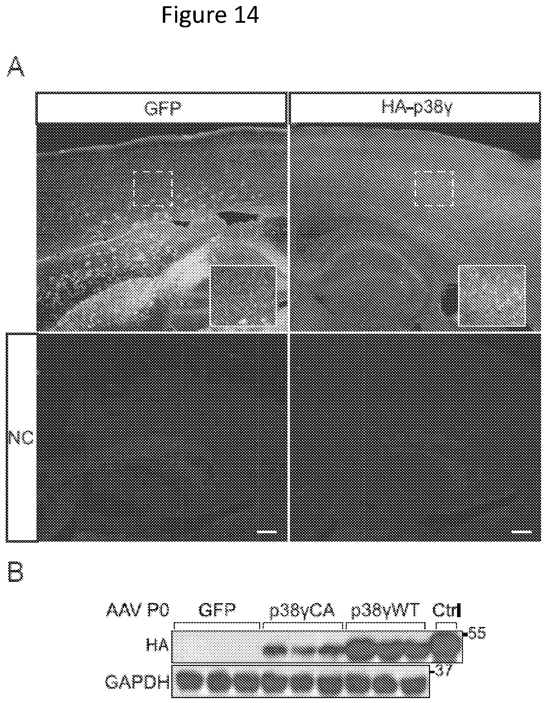

**Figure 14A** shows forbrains of mice infected with AAV constructs. Brains show widespread AAV-mediated expression of GFP or HA-p38γ. NC, negative control. Scale bar, 250 μm. Broken lines indicate insets.

**Figure 14B** is an immunoblot of cortical lysates of mice intracranially injected with AAV carrying GFP, HA-tagged p38γ or HA-tagged p38γCA which shows higher expression of p38γ than p38γCA. GAPDH confirmed equal loading. Ctrl, lysate from cells transfected with HA-p38γ.

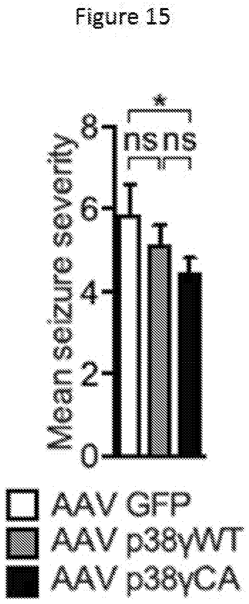

**Figure 15** is a graph showing mean seizure severity was significantly reduced in C57Bl/6 mice with AAV-mediated expression of p38γCA challenged with PTZ (50mg/kg i.p.) compared to GFP-expressing controls (n=8-10; *$P < 0.05$).

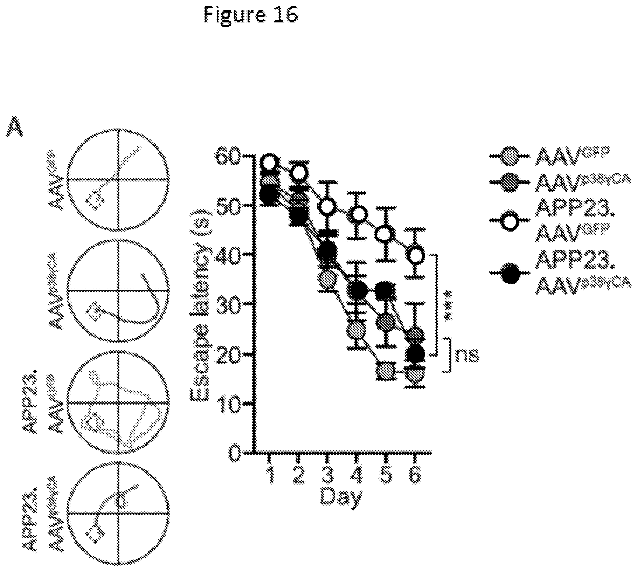

**Figures 16-18** show the spatial working memory deficits in APP23.AAV$^{GFP}$, AAV$^{GFP}$, AAV$^{p38γCA}$, and APP23.AAV$^{p38γCA}$ mice using Morris-water-maze (MWM).

**Figure 16A** is representative MWM path traces for APP23.AAV$^{GFP}$, AAV$^{GFP}$, AAV$^{p38γCA}$, and APP23.AAV$^{p38γCA}$ mice. Dashed squares is the location of hidden platform. Also shown is a graph showing that escape latency was decreased in AAV$^{GFP}$, AAV$^{p38γCA}$, and APP23.AAV$^{p38γCA}$ as compared to APP23.AAV$^{GFP}$ mice.

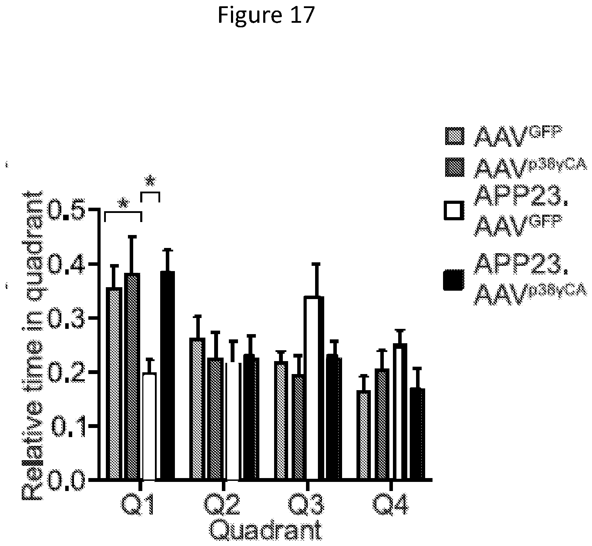

**Figure 17** is a graph showing AAV$^{p38γCA}$ mice spent more time in the targeted (Q1) and less time in the opposite quadrant (Q4) during probe trials, compared to APP23.AAV$^{GFP}$ mice.

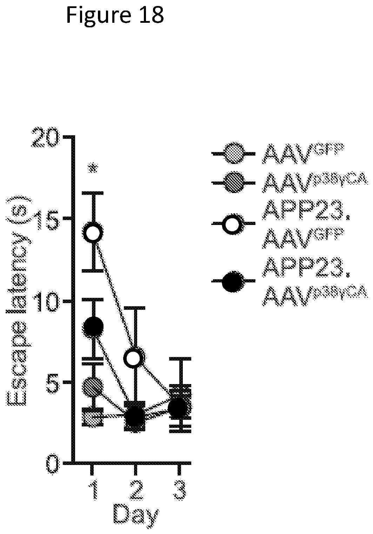

**Figure 18** is a graph showing escape latency over 3 days was decreased in AAV$^{GFP}$, AAV$^{p38γCA}$, and APP23.AAV$^{p38γCA}$ as compared to APP23.AAV$^{GFP}$ mice.

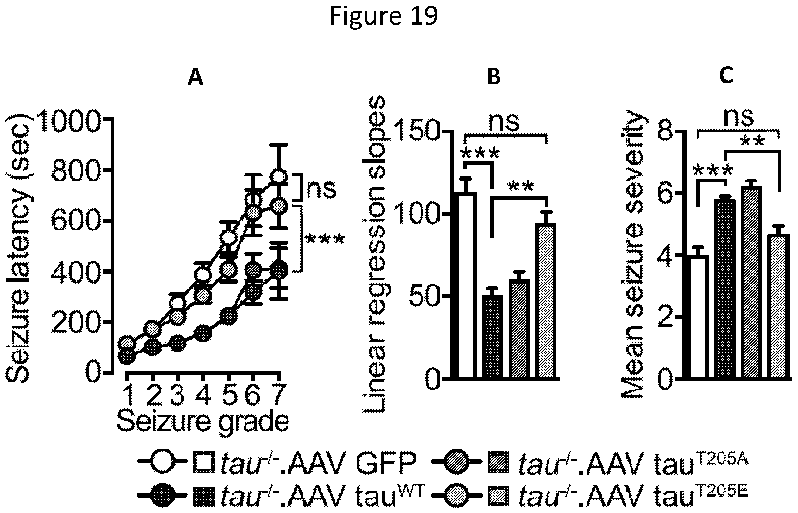

**Figure 19** is graphs showing effect of AAV mediated expression of tau wild type (tau$^{-/-}$.AAV tau$^{WT}$), GFP (tau$^{-/-}$.AAV GFP), tauT205A (tau$^{-/-}$.AAV tau$^{T205A}$), or tau T205E (tau$^{-/-}$.AAV tau$^{T205E}$) in tau$^{-/-}$ mice on (A) seizure latency and seizure grade ((B) is a linear regression of the slopes of (A)); and (C) mean seizure severity, following PTZ-induced seizures by administration of 50mg/kg of PTZ.

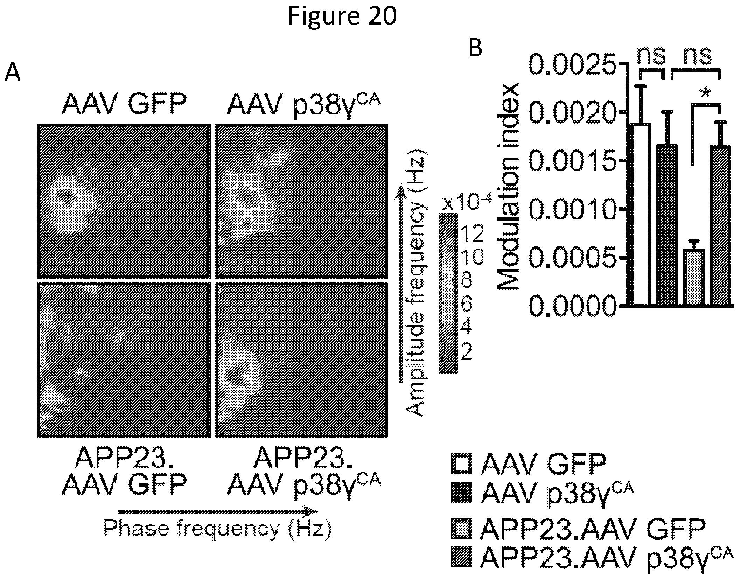

**Figure 20** is (A) an image of cross-frequency coupling (CFC); and (B) is a graph showing the modulation index, in APP23.AAVp38γ$^{CA}$ mice compared with APP23.AAVGFP, AAV.GFP and AAV.p38γ$^{CA}$ mice (n=5 to 6) (left) .*P<0.05; ns: not significant. Error bars indicate SEM.

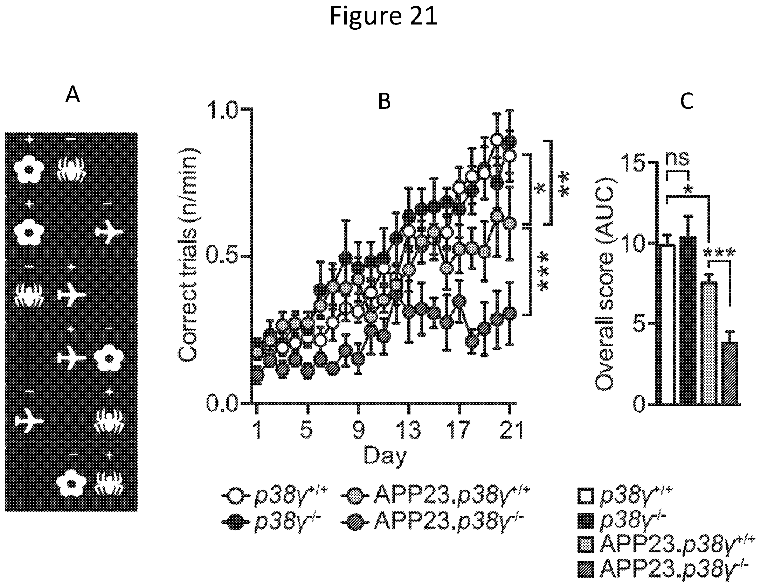

Figure 21 (A) shows stimulus image-location pairing possibilities in differential paired associate learning (dPAL) task in Bussey-Saksida touchscreen operant chamber used in pPAL trial in (B). + indicates the correct image location pairing and - indicates the incorect pairing. The 6 image location pairings were randomised across trials; (B) is a graph of the number of correct pPAL trials over time in APP23.p38γ$^{+/+}$, APP23.p38γ$^{-/-}$, p38γ$^{+/+}$ and p38γ$^{-/-}$ mice during touchscreen operant chamber testing; and (C) is a graph showing area under the curve analysis of correct trials per minute curves in (B), (n=8-10); ***P<0.001, **P<0.01, *P<0.05, ns: not significant; (left) two-way ANOVA: F(3,941)=60.90; a=0.05; SAidak post-hoc; (right) one-way ANOVA: F(3,41)=11.43; a=0.05; Sidak post-hoc).

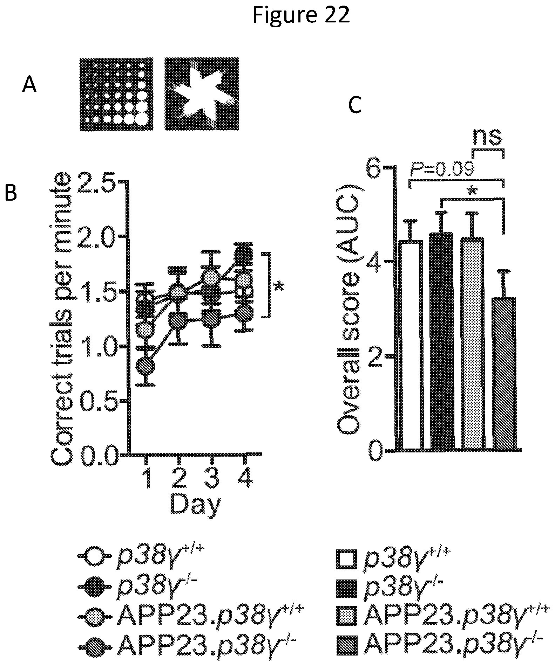

Figure 22 A-C shows the results of a pairwise discrimination task in Bussey-Saksida touchscreen operant chamber which shows minor impairment of discrimination memory in APP23.p38g-/- mice. (A) shows the stimulus used for the analysis of the pairwise discrimination task; (B) shows a graph of the number of correct trials per minute for consecutive testing days for APP23.p38γ$^{+/+}$, APP23.p38γ$^{-/-}$, p38γ$^{+/+}$ and p38γ$^{-/-}$ mice (n=8; *P<0.05 for APP23.p38γ$^{-/-}$ vs p38γ$^{-/-}$; a=0.05; F(3,100)=3.561; 2-way ANOVA with Sidak's multiple comparisons post-hoc test); and (C) is a graph showing area under the curve (AUC) analysis of correct trials per minute curves in (B) (n=8; *P<0.05 for APP23.p38γ$^{-/-}$ vs p38γ$^{-/-}$ α=0.05; F(3,28)=2.984; ANOVA with Sidak's multiple comparisons post-hoc test).

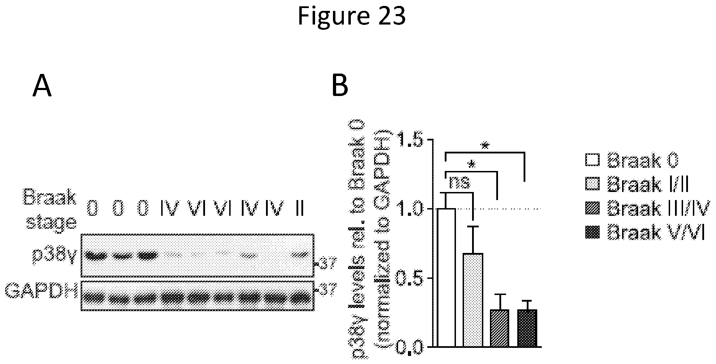

**Figure 23** is (A) an image of a representative Western blot of brain extracts from human controls (Braak 0) and humans suffering from Alzheimer's Disease at different neuropathological disease stages (Braak I-VI) set out in Table 3, (B) is a graph showing the levels of p38y in the western blot in (A) normalised to GAPDH, both (A) and (B) showing markedly reduced levels of p38y as AD advances, and a trend towards reduction in early disease stages,(n=4-5/group; *P<0.05; ns, not significant; a=0.05; F(3,13)=5.435; ANOVA with Sidak's multiple comparisons post-hoc test).

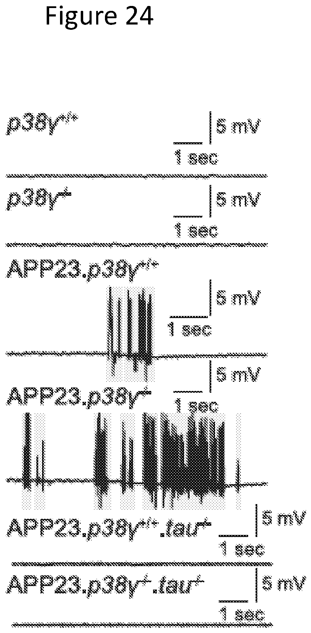

**Figure 24** is an image of representative EEG (LFP) traces in 4 month-old non-transgenic control *p38γ$^{+/+}$* and *p38γ$^{-/-}$*, and *APP23.p38γ$^{+/+}$*, APP23.*p38γ$^{-/-}$*, APP23.*p38γ$^{+/+}$.tau$^{-/-}$* and APP23.*p38γ* /-. tau-/- mice. Note that deletion of tau results in absent hypersynchronous activity (grey boxes).

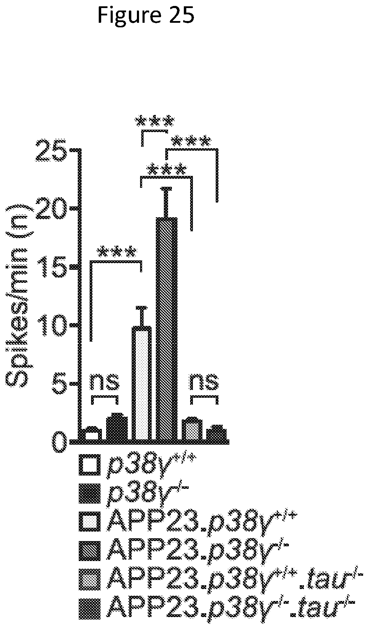

**Figure 25** is a graph showing numbers of hypersynchronous epileptiform activity (spikes per minute) in *p38γ$^{+/+}$*,*p38γ$^{-/-}$*, *APP23.p38γ$^{+/+}$*, APP23.*p38γ$^{-/-}$*, APP23.*p38γ$^{+/+}$. tau$^{-/-}$* and APP23.*p38γ$^{-/-}$.tau$^{-/-}$* mice. Hypersynchronous epileptiform activity in APP23.*p38γ$^{+/+}$. tau$^{-/-}$* and APP23.*p38γ$^{-/-}$. tau$^{-/-}$ mice* were similar to levels seen in nontransgenic control *p38γ$^{+/+}$* and *p38γ$^{-/-}$* mice (n=6-8; ***P < 0.001; ns, not significant; α=0.05; F(5, 223)=45.12; ANOVA with Sidak's multiple comparisons post-hoc test).

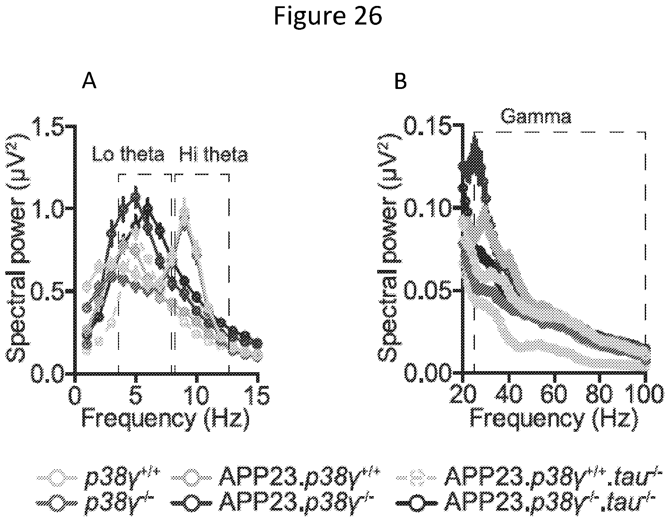

**Figure 26** is graphs showing (A) spectral power analysis of theta frequencies (4 - 12 Hz) in interictal sections of *APP23.p38γ$^{+/+}$*, APP23.*p38γ$^{-/-}$*, *APP23.p38γ$^{+/+}$. tau$^{-/-}$*, APP23.*p38γ$^{-/-}$. tau$^{-/-}$*, *p38γ$^{+/+}$* and *p38γ$^{-/-}$* recordings (n=6-8) . Note that theta shift to lower theta frequencies (4 - 8 Hz) in APP23 recordings was not reversed upon deletion of *tau* in APP23.*p38γ$^{+/+}$. tau$^{-/-}$* and APP23.*p38γ$^{-/-}$. tau-/-*. Dashed boxes mark low and high theta bands; and (B) gamma spectral power (25 - 100 Hz) of interictal sections of APP23.*p38γ$^{+/+}$* and more so APP23.*p38γ$^{-/-}$* was reverted in APP23.p38γ$^{+/+}$. tau$^{-/-}$ and APP23.*p38γ$^{-/-}$.tau$^{-/-}$* to levels of *p38γ$^{+/+}$* and *p38γ$^{-/-}$* recordings (n=6-8). Dashed boxes mark gamma band.

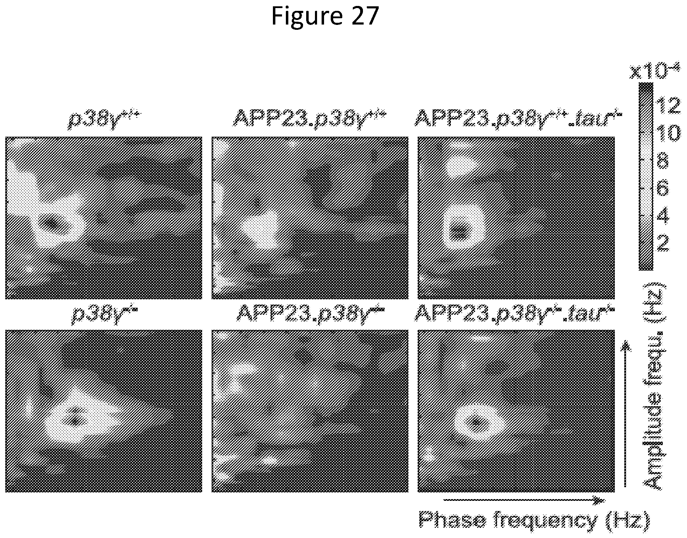

**Figure 27** is an image of a representative phase-amplitude comodulograms of interictal hippocampal LFPs recordings showed reduced and virtually lost cross-frequency coupling (~8Hz) in APP23.*p38γ$^{+/+}$* and APP23.*p38γ$^{-/-}$* respectively compared to *p38γ$^{+/+}$* and *p38γ$^{-/-}$* mice. Deletion of *tau* resulted in restored cross-frequency coupling in both APP23.p38γ$^{+/+}$. *tau$^{-/-}$* and APP23.*p38γ$^{-/-}$. tau$^{-/-}$* recordings (n=6-8) .

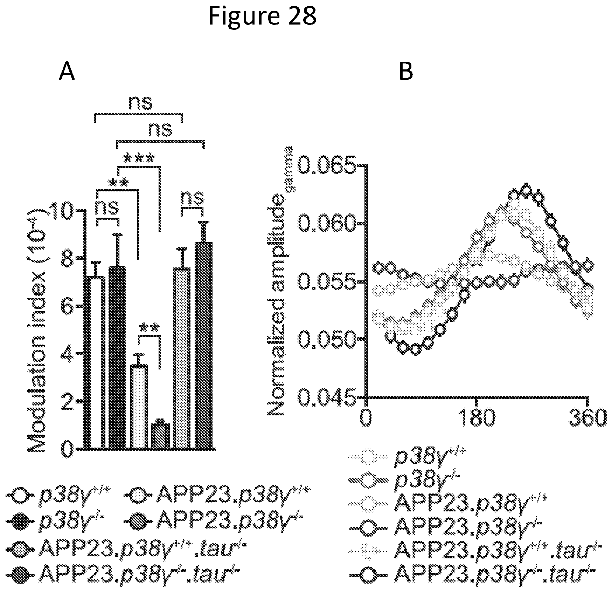

**Figure 28** are graphs showing (A) the averaged modulation index for coupling of theta phase and gamma amplitude in recordings from *APP23.p38γ+/+,* APP23.p38γ-/-, APP23.*p38γ+/+. tau-/-,* APP23.p38γ-/-. *tau-/-, p38γ+/+* and *p38γ-/-* mice (n=6-8; ***P < 0.001; **P < 0.01; ns, not significant; n=6-8; a=0.05; F(5, 111)=17.31; ANOVA with Sidak's multiple comparisons post-hoc test). Deletion of *tau* resulted in restored and similar levels of cross-frequency coupling in both APP23.*p38γ+/+.tau-/-* and APP23.*p38γ-/-.tau-/-* mice; and (B) a Phase-amplitude plot showing the relation of gamma amplitude across the theta phase computed for interictal hippocampal LFPs shows reduction in APP23.*p38γ+/+* (n=8) and loss in APP23.*p38γ-/-* (n=8) of phase-amplitude coupling (CFC) compared to *p38γ+/+* (n=6) and *p38y-/-* (n=6) mice. However, deletion of *tau* results in restored coupling across the theta phase in both APP23.*p38γ+/+.tau-/-* and APP23.*p38γ-/-.tau-/-* recordings (α=0.05; F(5, 2790)=0.003418; 2-way ANOVA with Sidak's multiple comparisons post-hoc test per phase bin).

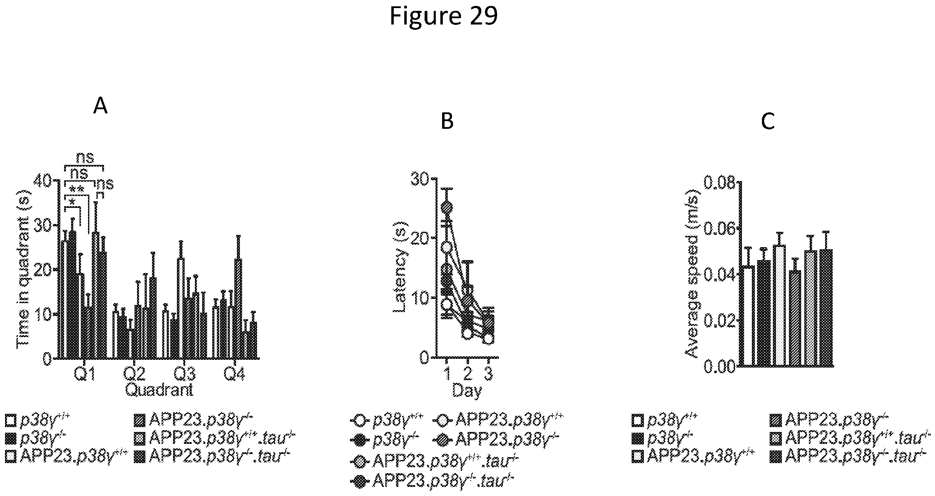

Figure 29 (A) to (C) is graphs showing details on effects of genetic deletion of tau on memory impairment in 12-month-old p38γ+/+, p38γ-/-, APP23. p38γ+/+, APP23.*p38γ-/-,* APP23.p38gγ-/-.tau-/- and APP23.p38γ+/+.tau-/- mice using the Morris water maze paradigm. (A) is a graph showing time in all 4 water maze quadrants (Q1-4) during probe trials (n=6-8; **P < 0.01; *P< 0.05; ns, not significant; a=0.05; F(5, 184)=0.002783; 2-way ANOVA with Sidak's multiple comparisons post-hoc test). (B) is a graph showing escape latencies were similar during visual cued platform testing, confirming visual competency (n=6-8; **P<0.01 (APP23.*p38γ-/-.tau-/-* vs APP23.*p38γ-/-* in trial 1); ns, not significant (P=0.5092; APP23.*p38γ-/-.tau-/-* vs APP23.*p38γ-/-* in trial 3); n=6-10; α=0.05; F(5, 145)=7.091; 2-way ANOVA with Sidak's multiple comparisons post-hoc test per trial). (C) is a graph showing Average swimming speeds were similar between APP23.*p38γ+/+*, APP23.*p38γ-/-*, APP23.p38γ+/+.*tau-/-*, APP23.*p38γ-/-.tau-/-, p38γ+/+* and *p38γ-/-* mice during MWM testing, confirming motor competence (n=6-8; a=0.05; F(5, 169)=0.4651; ANOVA with Sidak's multiple comparisons post-hoc test).

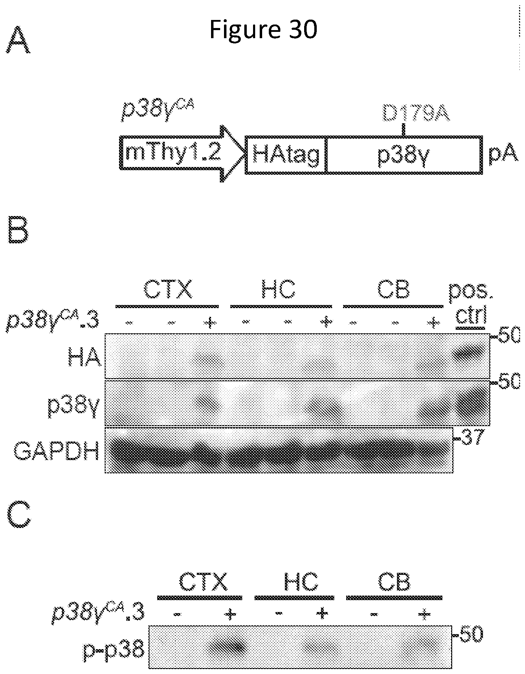

Figure 30 (A) is a schematic of the transgene construct use for the generation of *p38γCA,* mice by pronuclear injection into C57Bl/6 oocytes. HA-tagged p38γ containing the D179A mutation that renders it constitutively active was expressed under control of a neuronspecific murine Thy1.2 (mThy1.2) promoter, and followed by a bovine growth hormone poly-adenylation (pA) sequence. (B) Immunoblots of cortical (CTX), hippocampal (HC) and cerebellar (CB) brain extracts from non-transgenic (-) and of the *p38γCA*.3 (+) transgenic mouse line confirmed expression of HA-tagged p38γCA. HA-p38γCA expressed in 293T cells was used as a positive control. (C) Image showing immunoprecipitation of p38γ from nontransgenic (-) and of *p38γCA* (+) brains of transgenic p38γCA mice revealed active p38γ in all of *p38γCA* samples, as detected with an antibody to phosphorylated p38, indicating that the transgenic mice express active p38y.

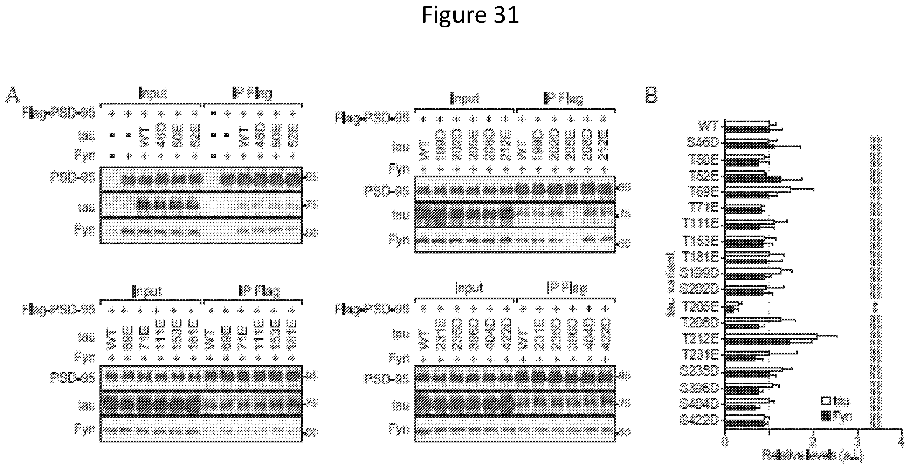

**Figure 31** is (A) representative western blots of co-immunopreciptation of mutated tau variants with PSD-95/tau/Fyn complexes in 293T cells, co-expressing individual tau variants together with PSD-95 and Fyn. Only the T205E tau variant abolished complex formation with PSD-95 and Fyn.; and (B) is a graph showing quantification of 4 independent experiments as shown in (A). The PSD-95/tau/Fyn complex formation was only significantly disrupted in the presence of the T205E tau variant (n=4; *P < 0.05 (for WT vs T205E); ns, not significant; a=0.05; F(18, 79)=1.003; ANOVA with Sidak's multiple comparisons test).

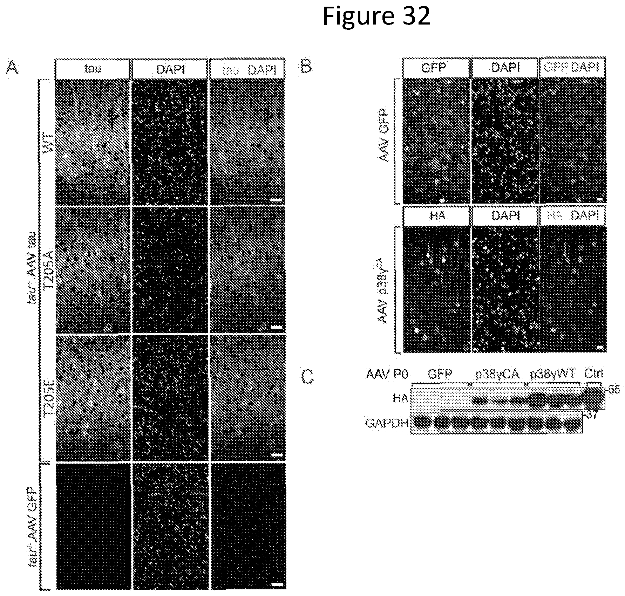

**Figure 32** is (A) an image showing AAV-delivered WT tau, T205A and T205E (as indicated) is broadly expressed in the cortex of 4 month-old *tau-/-* mice injected intracranially at postnatal day 0. No tau was detected in *tau-/-* brains injected with AAV GFP (tau-/-.AAVGFP). DAPI, nuclei. Scale bar, 50 μm; and (B) is an image showing staining of GFP or HA showing widespread neuronal AAV-mediated expression of GFP or HA-p38γ in brains of mice. Scale bar, 25 um; and (C) is an immunoblot of cortical lysates of mice intracranially injected on postnatal day 0 with AAV carrying GFP, HA-tagged p38γ or HA-tagged p38γCA. HA-tagged p38y showed higher expression of p38γ than p38γCA. GAPDH confirmed equal loading. Ctrl, lysate from cells transfected with HA-p38γ.

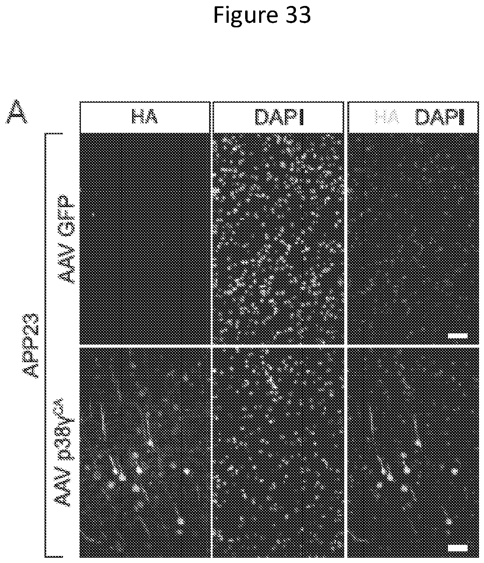

**Figure 33** is an immunofluorescence image showing that AAV-delivered *p38γCA* is broadly expressed in murine cortex of 6 month-old APP23 mice injected intracranially with AAV at postnatal day 0. Immunofluorescence staining for HA showed expression of HA-tagged p38γCA throughout the cortex. DAPI, nuclei. Scale bar, 50 μm.

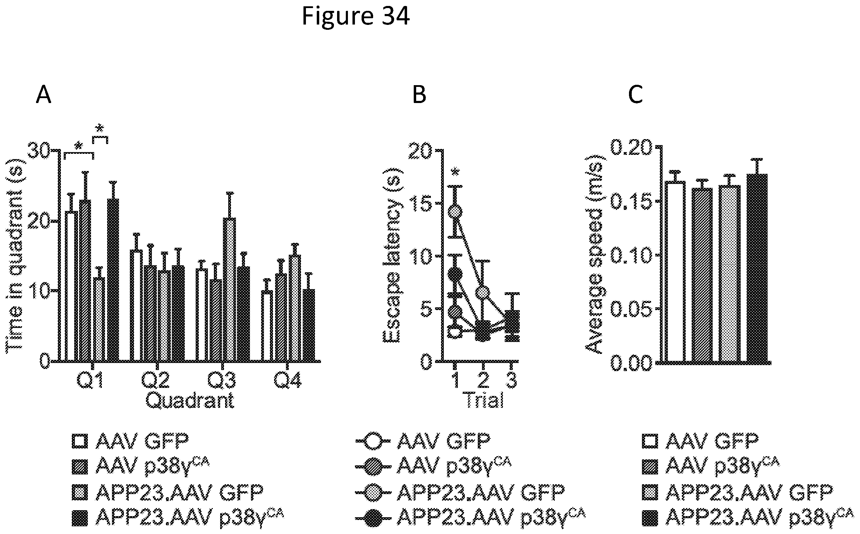

**Figure 34** is graphs showing the results of Morris Water Maze testing of WT or APP23 mice expressing AAV-delivered GFP or p38γCA, in which (A) is a graph showing time in quadrant of mice, and shows that APP23 mice expressing AAV-delivered *p38γCA* (APP23.AAVp38γCA) show consolidated memory as compared with APP23 expressing control AAV (APP23.AAVGFP) when performing MWM probe trials. Time in all 4 water maze quadrants (Q1-4) during probe trials (day 7) is shown for APP23.AAVp38γCA, APP23.AAVGFP and non-transgenic AAVGFP, AAVp38γCA controls. APP23.AAVp38γCA mice spend significantly more time in target quadrant Q1 as compared with APP23.AAVGFP mice. Note that AAVp38γCA mice show similar memory performance as AAVGFP mice (*P<0.05 (APP23.AAVp38γCA vs APP23.AAVGFP in Q1; F(3, 84)=3.494); n=6-10; a=0.05; F(3, 108)=4.454; 2-way ANOVA with Sidak's multiple comparisons post-hoc test per quadrant); (B) shows that APP23.AAVp38γCA, APP23.AAVGFP, AAVGFP, AAVp38γCA showed similar escape latencies after 3 visual cued trials in the MWM, indicating normal visuosensory function and motor-coordination competency (*P<0.05 (APP23.AAVp38γCA vs APP23.AAVGFP in trial 1; F(3, 84)=3.494); ns, not significant (P=0.5092; APP23.AAVp38γCA vs APP23.AAVGFP in trial 3); n=6-10; α=0.05; F(3, 84)=0.07474; 2-way ANOVA with Sidak's multiple comparisons post-hoc test per trial); and (C) shows that Average swimming speeds were similar between APP23.AAVp38$\gamma^{CA}$, APP23.AAVGFP, AAVGFP and AAVp38$\gamma^{CA}$ mice during MWM testing, confirming motor competency ($P$=0.8389; n=6-10; a=0.05; F(3, 68)=0.2811; ANOVA with Sidak's multiple comparisons post-hoc test).

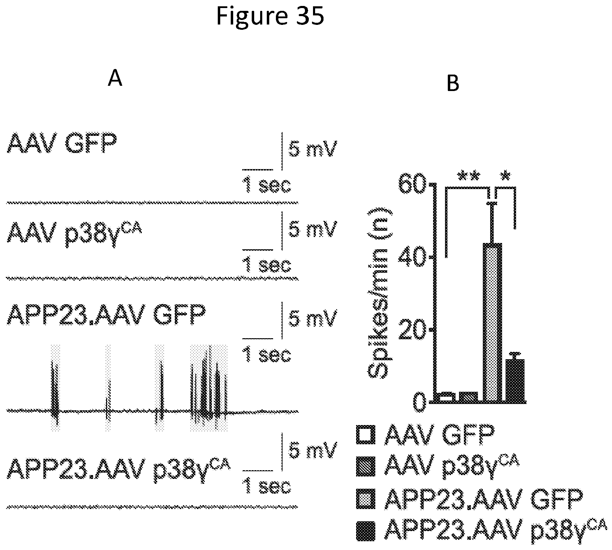

**Figure 35** shows (A) reduced spontaneous spikes in EEG recording from APP23.AAVp38$\gamma^{CA}$ mice compared to APP23.AAVGFP mice, and no spikes in EEG recording from AAVp38$\gamma^{CA}$ or AAVGFP-treated wild-type mice (n=5-6; **$P$<0.01, *$P$<0.05; one-way ANOVA: F(3, 68)=301.1; a=0.05; Sidak post-hoc); and (B) is a graph showing the spikes/min for AAV.GFP, AAVp38$\gamma^{CA}$, APP23.AAV.GFP and APP23.AAVp38$\gamma^{CA}$ mice, showing reduced spikes/min for APP23.AAVp38$\gamma^{CA}$ compared to APP23.AAVGFP mice.

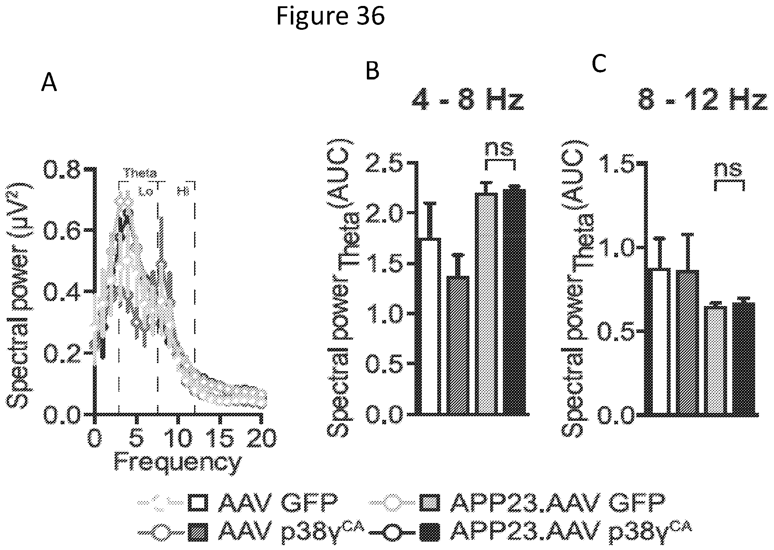

**Figure 36** shows Theta oscillation power changes of APP23 mice at 4-8 (B) and 8-12Hz (C) were not affected by AAVp38$\gamma^{CA}$ expression, with comparable levels in APP23.AAVp38$\gamma^{CA}$ and APP23.AAVGFP recordings (*$P$<0.05; n=5-6; a=0.05; F(3, 47)=3.038; ANOVA with Sidak's multiple comparisons post-hoc test).

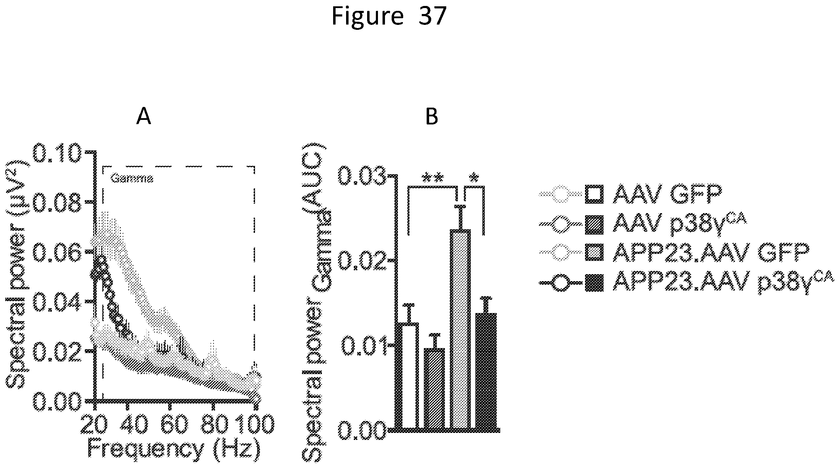

**Figure 37** shows gamma oscillation power (25-100Hz) in APP23.AAVp38$\gamma^{CA}$ mouse recordings was significantly reduced compared with APP23.AAVGFP mouse recordings. (**$P$<0.01; *$P$<0.05; n=5-6; a=0.05; F(3, 26)=6.930; ANOVA with Sidak's multiple comparisons post-hoc test).

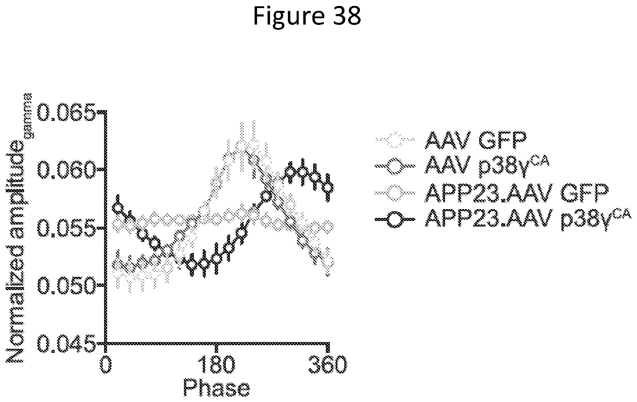

**Figure 38** is a graph showing that AAV-delivered $p38\gamma^{CA}$ results in normal cross-frequency coupling in EEG recordings of APP23 mice. Phase-amplitude correlation showed strong coupling of gamma amplitude along theta phase in APP23.AAVp38$\gamma^{CA}$ and in AAVGFP and AAVp38$\gamma^{CA}$ recordings, yet not in recordings from APP23.AAVGFP mice (**$P$<0.01 (APP23.AAVp38$\gamma^{CA}$ vs APP23.AAVGFP); a=0.05; F(3, 20)=4.793; 2-way ANOVA with Sidak's multiple comparisons post-hoc test per phase bin).

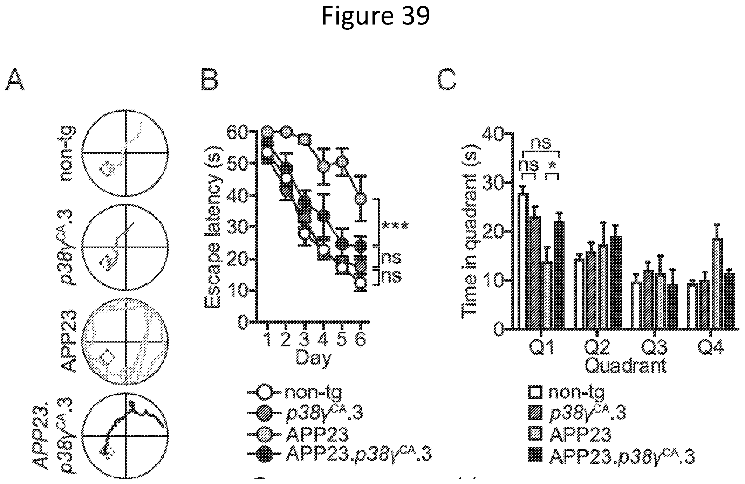

Figure 39 A to E shows active neuronal p38$\gamma$ protects APP23 mice from developing impaired memory function as tested by Morris water maze (MWM). (A) is representative traces of swim paths of WT (non tg), p38$\gamma^{CA}$.3, APP23 and APP23.p38$\gamma^{CA}$.3 mice in the MWM test showing that APP23.$p38\gamma^{CA}$ mice swim shorter paths in the Morris water maze test (day 5) as compared with APP23 mice, indicative of non-impaired learning/memory in these mice. $p38\gamma^{CA}$ single transgenic mice showed similar swim path lengths as non-transgenic controls, suggesting that active neuronal p38$\gamma$ does not affect learning functions on a wild-type background. Representative swim path traces are shown (n=6-12). (B) is a graph of escape latencies over 6 days, and shows that, consistent with shorter swim paths, escape latencies in APP23.$p38\gamma^{CA}$ mice were significantly lower than in APP23 mice, and similar to escape latencies seen in $p38\gamma^{CA}$ and non-transgenic mice (*$P$<0.05; n=6-12; a=0.05; F(3, 168)=4.454; 2-way ANOVA with Sidak's multiple comparisons post-hoc test). (C) is a graph of time in quadrant, and shows that APP23.$p38\gamma^{CA}$ mice spent significantly more time in the target quadrant during probe trials than APP23 mice, indicating consolidated memory in APP23.$p38\gamma^{CA}$ mice, yet not in APP23 mice. APP23.$p38\gamma^{CA}$ mice, single transgenic $p38\gamma^{CA}$ and non-transgenic mice spent similar time in the target quadrant (*$P$<0.05 F(3, 116)=7.028); n=6-12; $\alpha$=0.05; 2-way ANOVA with Sidak's multiple comparisons post-hoc test per quadrant). (D) is a graph showing that escape latencies converged to similar levels after 3 visual cued trials in all experimental groups, indicating normal visuo-sensory function and motor-coordination in APP23.$p38\gamma^{CA}$, APP23, $p38\gamma^{CA}$ and non-transgenic mice (*$P$<0.05 (APP23.$p38\gamma^{CA}$ vs APP23 in trial 1; F(3, 87)=3.690); ns, not significant ($P$=0.7190; APP23.$p38\gamma^{CA}$ vs APP23 in trial 3); n=6-12; a=0.05; F(3, 87)=0.369; 2-way ANOVA with Sidak's multiple comparisons post-hoc test per trial). (E) is a graph showing that average swimming speeds were similar in APP23.$p38\gamma^{CA}$, APP23, $p38\gamma^{CA}$ and nontransgenic mice during MWM testing, confirming motor competency ($P$=0.3221; n=6-12; $\alpha$=0.05; F(3, 62)=1.187; ANOVA with Sidak's multiple comparisons post-hoc test).

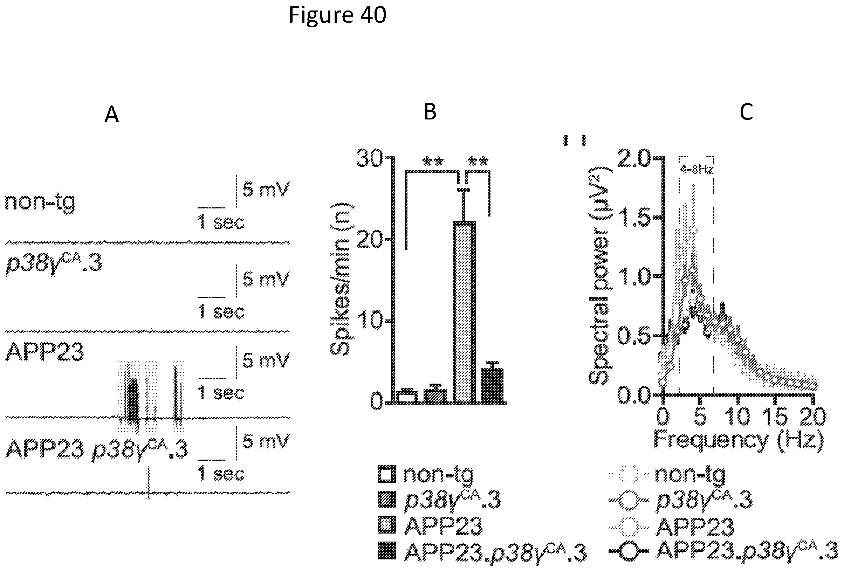

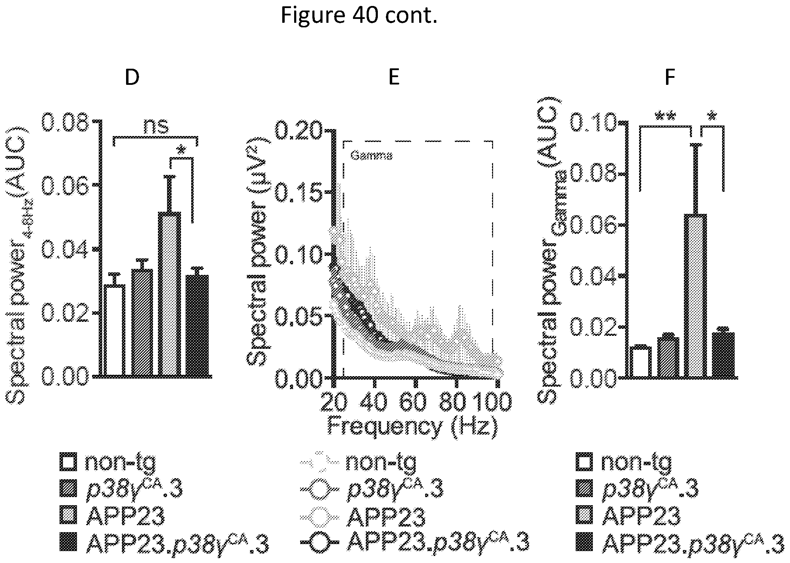

**Figure 40** is (A) EEG recordings from WT (non-tg), APP23 mice, single transgenic p38$\gamma^{CA}$.3, and APP23.p38$\gamma^{CA}$.3 mice, showing that APP23.$p38\gamma^{CA}$.3 mice exhibited markedly lower epileptiform activity than APP23 recordings. (n=4-5). (B) is a graph showing that significantly fewer hypersynchronous epileptiform discharges were found in recordings from APP23.$p38\gamma^{CA}$ mice compared with APP23 recordings. Single transgenic $p38\gamma^{CA}$ and non-transgenic control recordings did not show hypersynchronous activity (**$P$<0.01; n=4-5; a=0.05; F(3, 22)=11.38; ANOVA with Sidak's multiple comparisons post-hoc test). (C and D) are graphs showing increased theta oscillation power of APP23 was reduced to levels of $p38\gamma^{CA}$ and nontransgenic recordings in APP23.$p38\gamma^{CA}$ recordings. Specifically, the spectral distribution peak at 4-8Hz in APP23 power spectra was significantly lower in APP23.$p38\gamma^{CA}$ spectra (**$P$<0.01; *$P$<0.05; n=4-5; $\alpha$=0.05; F(3, 26)=6.930; ANOVA with Sidak's multiple comparisons post-hoc test). (E and F) are graphs showing increased gamma oscillation power (25-100Hz) of APP23 was reduced to levels of $p38\gamma^{CA}$ and non-transgenic recordings in APP23.$p38\gamma^{CA}$ recordings (**$P$<0.01; *$P$<0.05; n=4-5; a=0.05; F(3, 47)=4.761; ANOVA with Sidak's multiple comparisons post-hoc test).

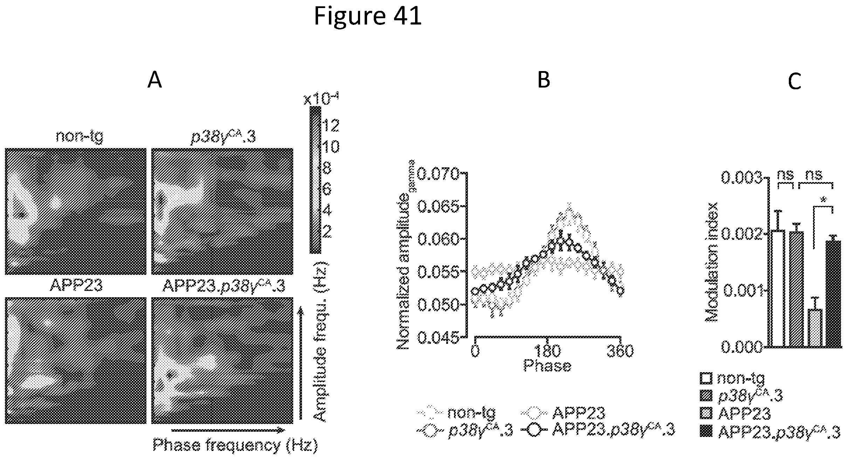

Figure 41 (A) is an image showing comodulogram analysis of cross-frequency coupling showed unaffected coupling of theta oscillations to gamma amplitude in recordings of APP23.$p38\gamma^{CA}$ mice in contrast to APP23 recordings. Representative comodulograms are shown (n=4-5) (B) is a graph in which phase-amplitude correlation showed strong coupling of gamma amplitude along theta phase in APP23.$p38\gamma^{CA}$ and in p38$\gamma^{CA}$ and non-transgenic recordings, yet not in recordings from APP23 mice. (**$P$<0.01 (APP23.AAVp38$\gamma^{CA}$ vs APP23.AAVGFP); n=4-5; $\alpha$=0.05; F(3, 162)=3.238; 2-way ANOVA with Sidak's multiple comparisons post-hoc test per phase bin). (C) is a graph showing average modulation index was significantly higher in APP23.$p38\gamma^{CA}$ recordings as compared with APP23 recordings and reached similar levels as in recordings from single transgenic $p38\gamma^{CA}$ and non-transgenic control mice (*$P$<0.05; n=4-5; a=0.05; F(3, 9)=6.370; ANOVA with Sidak's post-hoc test).

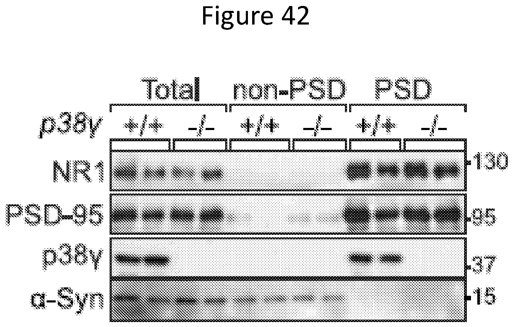

**Figure 42** is an immunoblot of extracts from dendritic spines of hippocampal neurons showing p38γ enriched with NR1 and PSD-95 in PSD fractions of p38y+/+ synaptosome preparations, yet not in non-PSD fractions ($\alpha$-syn; $\alpha$-synuclein).

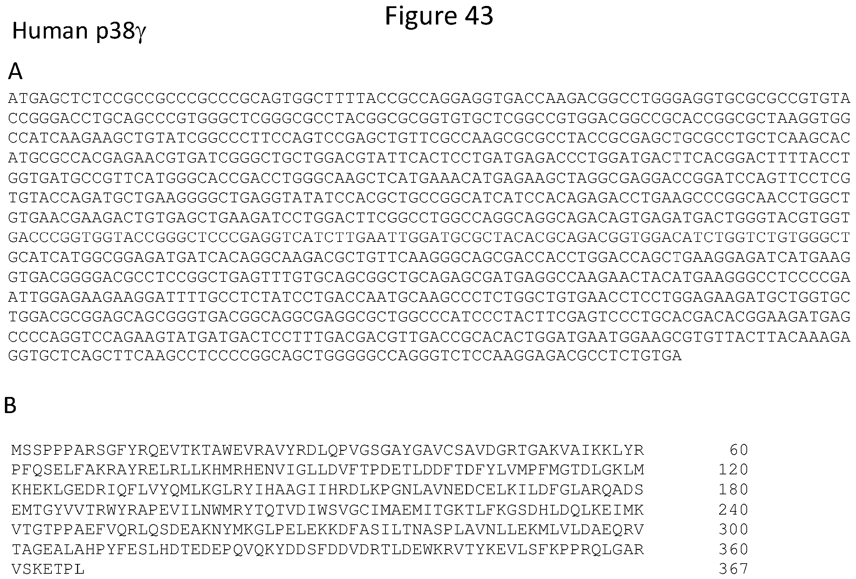

**Figure 43** shows (A) the nucleic acid sequence (SEQ ID NO: 1) and (B) the amino acid sequence (SEQ ID NO: 2) of full length human wild-type p38y.

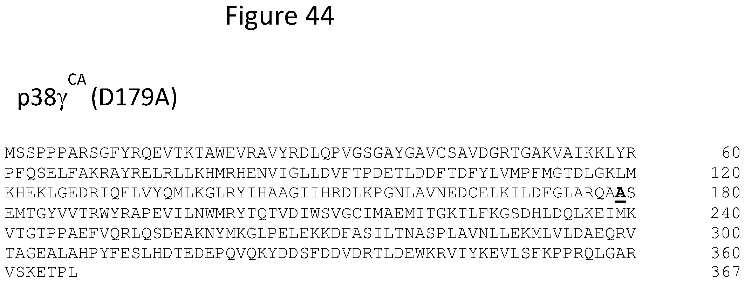

**Figure 44** shows the amino acid sequence of p38γ$^{CA}$ (SEQ ID NO: 3). The location of the mutation from D to A (D179A) is underlined.

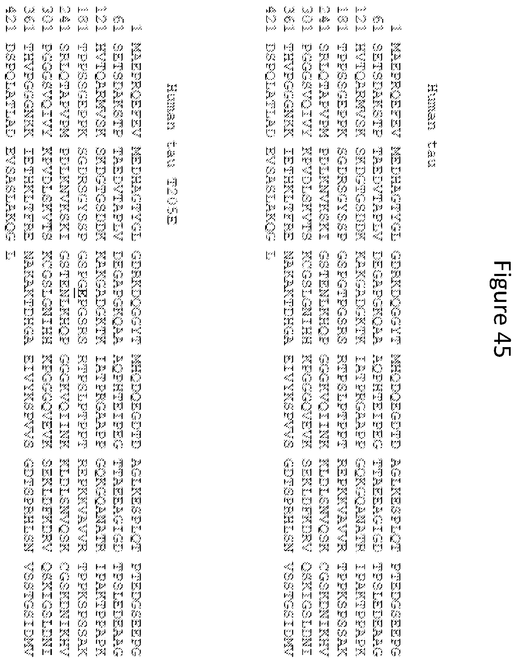

**Figure 45** shows the amino acid sequence of full length human tau (top) (SEQ ID NO: 4) and tau T205E (SEQ ID NO: 5) (bottom). The location of the mutation from T to E in tau T205E is underlined.

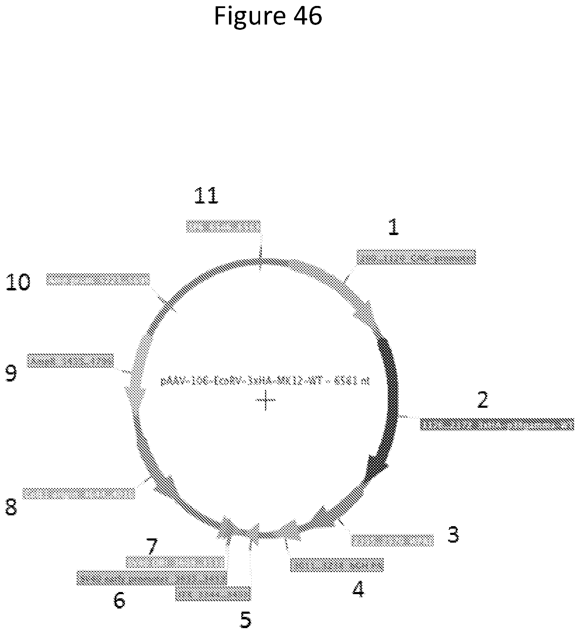

**Figure 46** is a map of adeno-associated viral vector pAM-CAG containing wild-type p38y coding sequence. 1. Position 200-1120, CAG-promoter; 2. Position 1176-2372, 3xHA-p38y-wt coding sequence; 3. Position 2395-2970, WPRE; 4. Position 3011-3238, bGH PA; 5. Position 3344-3453, ITR; 6. Position 3655-3459, SV40 promoter; 7. Position 3608-3531, SV40 ORI; 8. Position 4644-4016, ColE1 origin; 9. Position 5455-4796, AmpR; 10. Position 5723-5695, Amp prom; 11. Position 6546-6563, SP6.

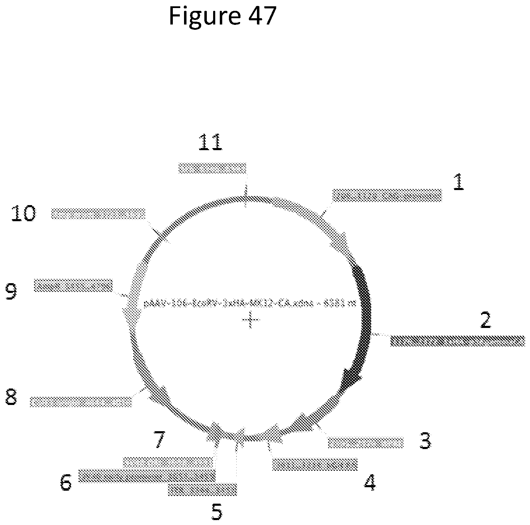

**Figure 47** is a map of adeno-associated viral vector pAM-CAG containing the coding sequence of p38γ$^{CA}$ (D179A) (constitutively active variant of p38γ). 1. Position 200-1120, CAG-promoter; 2. Position 1176-2372, 3xHA-p38γ$^{CA}$ coding sequence; 3. Position 2395-2970, WPRE; 4. Position 3011-3238, bGH PA; 5. Position 3344-3453, ITR; 6. Position 3655-3459, SV40 promoter; 7. Position 3608-3531, SV40 ORI; 8. Position 4644-4016, ColE1 origin; 9. Position 5455-4796, AmpR; 10. Position 5723-5695, Amp prom; 11. Position 6546-6563, SP6.

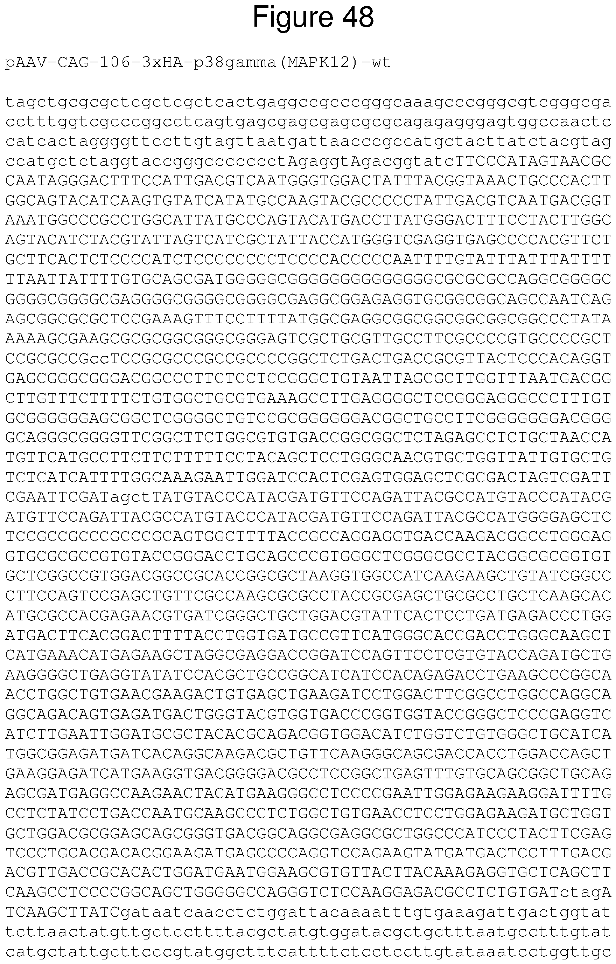

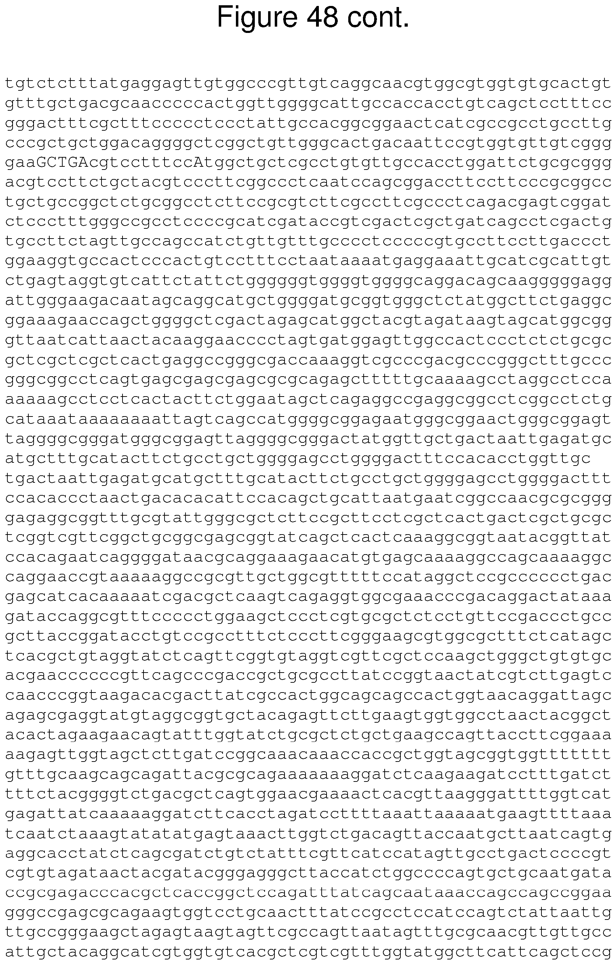

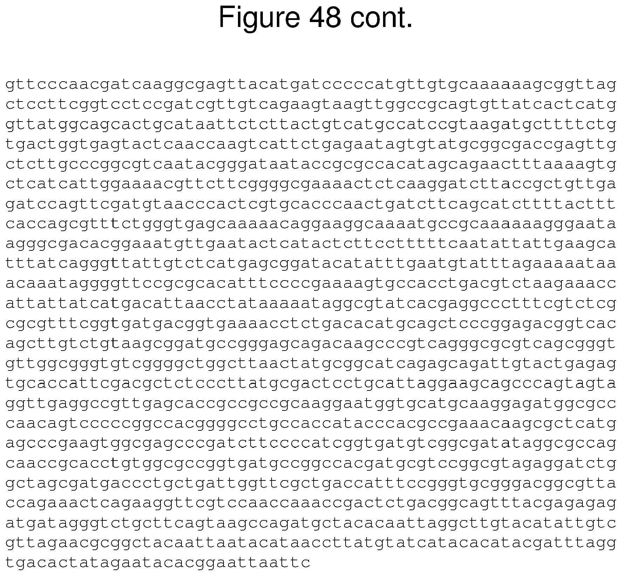

**Figure 48** is the nucleic acid sequence of adeno-associated viral vector pAM-CAG containing wild-type p38y coding sequence (SEQ ID NO: 6).

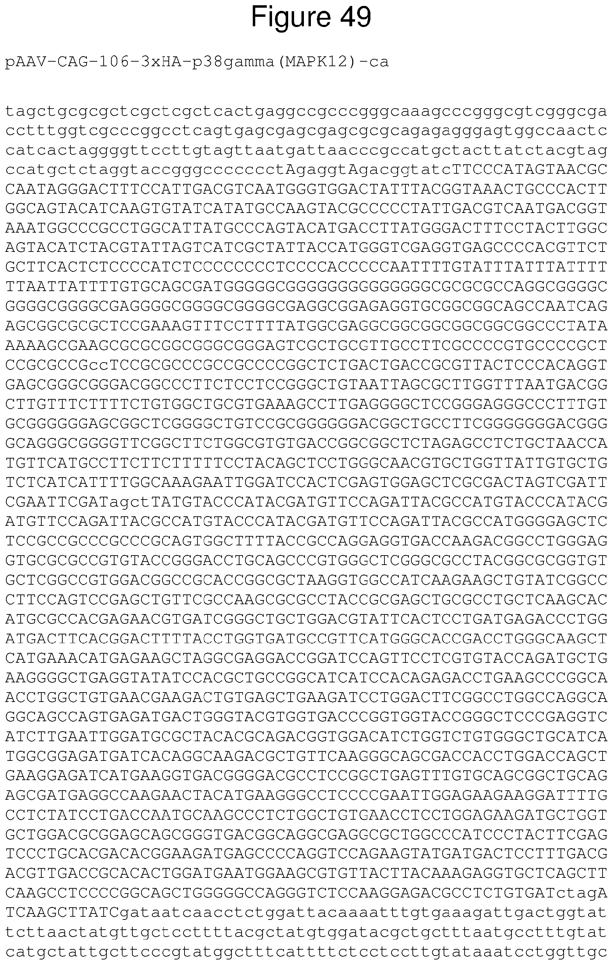

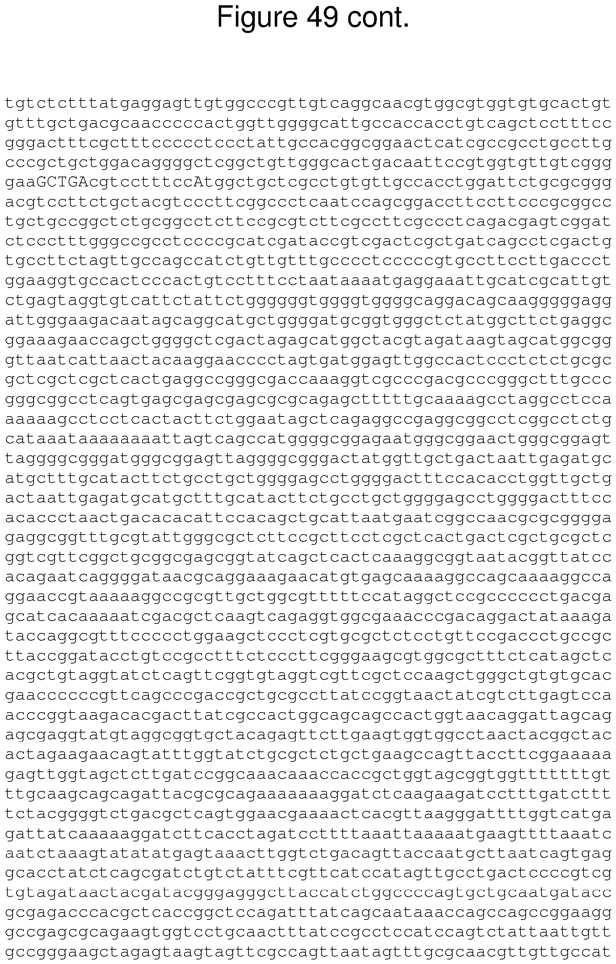

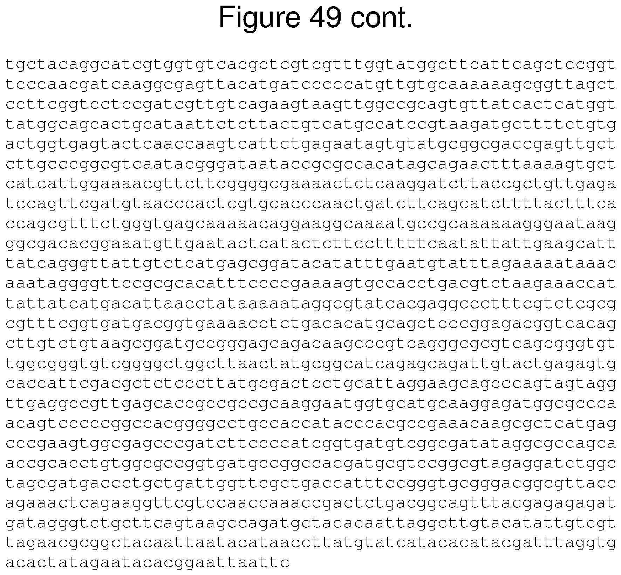

**Figure 49** is the nucleic acid sequence of adeno-associated viral vector pAM-CAG containing the coding sequence of p38γ$^{CA}$ (D179A) (constitutively active variant of p38y) (SEQ ID NO: 7).

**Detailed Description**

The method of treating or preventing a disease as described herein, is to be construed as an agent for use in treatment or prevention of a disease. More in particular, the method of treating or preventing a nenrological condition mediated by a tau-dependent signalling complex in neurons of a subject as described herein, is to be construed as an agent for use in treating or preventing a neurological condition mediated by a tau-dependent signalling complex in neurons of a subject.

The present invention relates to an agent which elevates p38γ activity, or the activity of a variant of p38γ, in neurons of a subject, for use in treating or preventing a neurological condition mediated by a tau-dependent signalling complex in neurons of a subject. The inventors have found that promoting phosphorylation of one or more amino acid residues of tau, wherein the phosphorylation of the amino acid residues causes disruption of the tau-dependent signalling complex in neurons of the subject, or introducing a variant of tau that causes disruption of the tau-dependent signalling complex in neurons of the subject, can be used to treat or prevent neurological conditions mediated by a tau-dependent signalling complex, such as AD.

A tau-dependent signalling complex is a post-synaptic signalling complex, typically associated with the N-methyl-D aspartate receptor (NMDA receptor), which can mediate excitotoxicity in neurons. A signalling complex is a complex of proteins which are involved in transduction of a signal in a cell. A tau dependent signalling complex requires tau in order to transduce the signal. The tau-dependent signalling complex typically comprises tau as a component of the complex.

Excitotoxicity refers to the process by which neurons are damaged or killed by excessive stimulation of glutamatergic receptors, such as NMDA receptors, and is mediated via signalling complexes in the postsynaptic space. Neural damage from excitotoxicity is associated with a number of neurological conditions. Neural damage in stroke patients is believed to be caused, at least in part, by overactivation of glutamatergic receptors and associated signalling complexes by excessive amounts of extracellular glutamate that are released immediately following ischaemic stroke. Neural damage in epilepsy is also thought to result from excitotoxicity caused by overactivation of glutamatergic receptors and associated signalling complexes following release of glutamate during epileptic events.

The tau-dependent signalling complex is also thought to mediate amyloid-β (Aβ) toxicity in Alzheimer's disease (AD). In Alzheimer's disease (AD), amyloid-β (Aβ) has been shown to induce toxicity in neurons through a signalling complex comprising NMDA receptors, PSD-95, tau and FYN.

The tau-dependent signalling complex typically comprises tau. In one embodiment, the tau-dependent signalling complex comprises PSD-95 and tau. In one embodiment, the tau-dependent signalling complex comprises PSD-95, FYN and tau. Typically, the tau-dependent signalling complex comprises NMDA receptors, PSD-95, tau and FYN.

The neurological condition may be any neurological condition mediated by a tau-dependent signalling complex. Typically, the neurological condition is caused by neuronal damage from overactivation of the tau-dependent signalling complex. Examples of such conditions include, for example, Alzheimer's disease, frontotemporal dementia, amyotrophic lateral sclerosis, Huntington's disease, Parkinson's disease, neural damage from stroke and neural damage from epilepsy.

In one embodiment, the neurological condition is Alzheimer's disease.

In one embodiment, the neurological condition is stroke.

In one embodiment, the neurological condition is epilepsy.

In one embodiment, the method comprises treating the subject to promote phosphorylation of tau at T205, wherein the phosphorylation of the amino acid residues causes disruption of the tau-dependent signalling complex. As used herein, "disruption of the tau-dependent signalling complex" refers to an effect which prevents the tau-dependent signalling complex from mediating excitotoxicity and Aβ toxicity, and includes destabilising, dismantling or preventing formation of, the signalling complex. In one embodiment, the one or more amino acid residues of tau that are phosphorylated to cause disruption of the tau-dependent signalling complex are one or more amino acid residues that would be phosphorylated by the MAP kinase p38y. The amino acid residue of tau that is phosphorylated to cause disruption of the tau-dependent signalling complex is threonine at position 205 (T205). In one embodiment, the one or more amino acid residues of tau that is phosphorylated to cause disruption of the tau-dependent signalling complex is threonine at position 205 (T205) and one or more amino acid residues selected from the group consisting of serine at position 199 (S199), serine at position 396 (S396) and serine at position (S404). In various embodiments, the amino acid residues of tau that are phosphorylated to cause disruption of the tau-dependent signalling complex are: (a) T205; (b) T205, S199; (c) T205,S199,S396; (d) T205,S199,S396,S404; (e) T205,S199,S404; (f) T205,S396,S404; (g) T205,S396; or (h) T205,S404.

In one embodiment, the subject is treated to promote phosphorylation of tau at T205, wherein phosphorylation of the amino acid residues causes disruption of the tau-dependent signalling complex in neurons of the brain of the subject.

In one embodiment, the subject is treated by administering an agent that elevates tau that has been phosphorylated at one or more amino acid residues, wherein the phosphorylation of the amino acid residues causes disruption of the tau-dependent signalling complex.

The agent comprises, a nucleic acid sequence, a nucleic acid analogue, a protein, or a peptide. Typically, administration of the agent introduces the agent into neurons of the subject. More typically, administration of the agent introduces the agent into neurons of the brain of the subject.

In some embodiments, the agent comprises a nucleic acid sequence which is introduced into neurons of the subject. The nucleic acid is then transcribed and translated in the neurons.

In some embodiments, the agent can cross the blood-brain barrier, or can be formulated to cross the blood-brain barrier.

As used herein, a "subject" is a mammal. The mammal can be a human, non-human primate, sheep, mouse, rat, dog, cat, horse, cow, pig, or any other mammals which can suffer from a neurological condition mediated by a tau-dependent signalling complex in neurons. Typically, the subject is a human.

In one embodiment, the subject is treated by administering an agent that elevates p38y activity, or activity of a variant of p38y, in neurons of the subject. p38y, also known as ERK6, SAPK3 and MAPK12, is a mitogen activated protein kinase (MAP Kinase). In one embodiment, the p38y is from a mammal. For example, the p38y may be from a human, mouse, dog, cat, pig, cow, rat, non-human primate, goat, sheep. Typically, the p38y is human p38y. Wild type p38y is activated through phosphorylation of tyrosine and threonine residues in the motif TGY. Wild type p38y phosphorylates tau following activation. Activation of p38y is carried out by the MAP kinase kinases MKK3 and MKK6, which are in turn activated upon phosphorylation by the MAPK kinase MAP3K.

As described in the Examples, the inventors have found that phosphorylation of tau by p38y results in disruption of NR/PSD-95/tau/FYN complexes in cultured neurons and in a mouse model of Alzheimer's disease; limits Aβ-induced toxicity in cultured neurons in a mouse model of Alzheimer's disease; and reduces the severity of pentylenetetrazole (PTZ) induced seizures in a mouse model of excitotoxicity and epilepsy. The inventors have shown that by introducing p38y, or a constitutively active variant of p38y, into neurons of mice, NR/PSD-95/tau/FYN complexes in neurons are disrupted and Aβ-induced excitotoxicity is reduced in a mouse model of Alzheimer's disease, and the severity of pentylenetetrazole (PTZ) induced seizures in a mouse model of excitotoxicity and epilepsy is reduced.

An agent that elevates p38y activity, or the activity of a variant of p38y, in a neuron may be an agent that: (a) elevates the amount of p38y, typically the amount of active p38γ, in the neuron; and/or (b) elevates the amount of a variant of p38y, typically the amount of an active variant of p38γ, in the neuron; and/or (c) elevates the amount of p38y activation in the neuron; and/or (d) elevates the amount of activation of the variant of p38y in the neuron, if the variant if not an active variant. As used herein, "p38y activity" is an activity of activated p38ythat causes disruption of the tau-dependent signalling complex. Typically, the activity of activated p38y that causes disruption of the tau-dependent signalling complex is phosphorylation of tau at T205, and optionally phosphorylation of tau at one or more amino acid residues selected from the group consisting of, for example, S199, S396, and S404. The "activity of a variant of p38y" refers to an activity of a variant of p38y which is the same as, or substantially similar to, p38y activity. The variant of p38y may be capable of p38y activity without activation (for example, an active variant, such as a constitutively active variant), or may exhibit p38y activity following activation. p38y activity is elevated in a neuron when the amount of p38y activity in the neuron after treatment is increased relative to the amount of p38y activity in the neuron prior to treatment. The activity of a variant of p38y is elevated in a neuron when the amount of activity of the variant in the neuron after treatment is increased relative to the amount of activity of the variant in the neuron prior to treatment. The p38y activity, or the activity of a variant of p38y, is elevated by administering an agent which elevates:

> (a) the amount of endogenous p38yin the neurons, such as increasing expression (transcription and/or translation) of endogenous p38y; and/or
> (b) the amount of exogenous p38y in the neurons; and/or
> (c) the amount of a variant of p38y in the neurons;
> and/or
> (d) the activation of endogenous p38y, exogenous p38y and/or variant of p38y, in the neurons.

In one embodiment, the p38y activity, or the activity of a variant of p38y, is elevated by administering an agent which elevates the amount of exogenous p38y, or a variant thereof, in neurons. The amount of exogenous p38y, or a variant thereof, may be elevated by introducing into neurons p38y, or a variant thereof, or by introducing into neurons a nucleic acid capable of expressing p38y, or a variant thereof.

Thus, in one embodiment, the agent which elevates p38y activity, or the activity of a variant of p38y, in neurons of the subject, may comprise the p38y protein or variant thereof, or a nucleic acid that is capable of expressing p38y, or a variant thereof, in neurons of the subject. The nucleic acid sequence encoding full-length wild-type human p38γ, together with the amino acid sequence of full-length wild-type human p38y, used in the Examples described herein is shown in Figure 43. Naturally occurring isoforms and variants of human p38y are also known (e.g. Genbank accession nos. NP_001290181, CR456515). It is envisaged that natural isoforms or variants of p38y that phosphorylate tau at an amino acid residue of tau which causes disruption of the tau-dependent signalling complex could be used in the methods described herein.

In one embodiment, the agent which elevates p38y activity, or the activity of a variant of p38y, comprises a nucleic acid that encodes p38y or a variant thereof. Those skilled in the art will be able to determine the appropriate nucleic acid sequence which encodes the amino acid sequence of the p38y or variant thereof. For example, a nucleic acid sequence which encodes p38y may comprise a nucleic acid sequence that is in the range of from about 60% to 100% identical to the wild-type coding sequence of human p38y ( SEQ ID NO: 1). For example, the nucleic acid encoding p38y may have a sequence that has at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to the wild-type coding sequence of p38y using one of the alignment programs described herein using standard parameters. Those skilled in the art will recognize that these values can be appropriately adjusted to determine corresponding identity of proteins encoded by a nucleotide sequence by taking into account codon degeneracy, reading frame positioning, and the like.

In one embodiment, the agent which elevates p38y activity, or the activity of a variant of p38y, comprises a variant of p38y. In one embodiment, the agent which elevates p38y activity, or the activity of a variant of p38y, comprises a nucleic acid that encodes a variant of p38y. As used herein, a variant of p38y is a protein which differs from the wild-type human p38y protein by one or more amino acid substitutions, additions or deletion, and which is capable of phosphorylating an amino acid residue of tau which causes disruption of the tau-dependent signalling complex. Typically, the variant of p38y phosphorylates tau at residue T205, and optionally one or more residues selected from the group consisting of S199, S396, S404. In one embodiment, the variant of p38y comprises an amino acid sequence that is at least 60%, 65%, 70%, 75%, 80%, 85%, 90%, 95%, or 99% identical to the amino acid sequence of wild-type human p38y. In one embodiment, the variant of p38y comprises an amino acid sequence that is at least 60%, 65%, 70%, 75%, 80%, 85%, 90%, 95%, or 99% identical to the amino acid sequence represented by SEQ ID NO: 2.

As used herein, "% identity" with reference to a polypeptide, or "% identical to the amino acid sequence of a polypeptide", refers to the percentage of residues in the two sequences that are the same when aligned for maximum correspondence over a specified comparison window, as measured by sequence comparison algorithms or by visual inspection.

Sequence comparison algorithms for determining % identity between two polypeptides are known in the art. Examples of such algorithms are the algorithm of Myers and Miller (1988); the local homology algorithm of Smith et al. (1981); the homology alignment algorithm of Needleman and Wunsch (1970); the search-for-similarity-method of Pearson and Lipman (1988); the algorithm of Karlin and Altschul (1990), modified as in Karlin and Altschul (1993). Computer implementations of these algorithms for determining % identity between two polypeptides include, for example: CLUSTAL (available from Intelligenetics, Mountain View, Calif.) (Pearson et al. (1994)).; the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA).

In some embodiments, the variant of p38y may comprise a part of p38y. The variant of p38y comprises a PDZ interaction motif. PSD-95 comprises a PDZ motif, and p38y is believed to interact with PSD-95, at least in part, through the PDZ interaction motif. The PDZ interaction motif of p38y is a short amino acid sequence in the C-terminal portion of the p38y molecule (see Figure 1A). The PDZ interaction motif comprises the amino acid sequence ETPL or ETAL. In various embodiments, the variant of p38y comprises an amino acid sequence selected from the group consisting of: ETPL (SEQ ID NO: 8), KETPL (SEQ ID NO: 9), SKETPL (SEQ ID NO: 10), VSKETPL (SEQ ID NO: 11), RVSKETPL (SEQ ID NO: 12), ARVSKETPL (SEQ ID NO: 13), GARVSKETPL (SEQ ID NO: 14), LGARVSKETPL (SEQ ID NO: 15), QLGARVSKETPL (SEQ ID NO: 16), RQLGARVSKETPL (SEQ ID NO: 17), PRQLGARVSKETPL (SEQ ID NO: 18), PPRQLGARVSKETPL (SEQ ID NO: 19), KPPRQLGARVSKETPL (SEQ ID NO: 20), FKPPRQLGARVSKETPL (SEQ ID NO: 21), SFKPPRQLGARVSKETPL (SEQ ID NO: 22), LSFKPPRQLGARVSKETPL (SEQ ID NO: 23), VLSFKPPRQLGARVSKETPL (SEQ ID NO: 24), EVLSFKPPRQLGARVSKETPL (SEQ ID NO: 25), KEVLSFKPPRQLGARVSKETPL (SEQ ID NO: 26), YKEVLSFKPPRQLGARVSKETPL(SEQ ID NO: 27), TYKEVLSFKPPRQLGARVSKETPL (SEQ ID NO: 28), VTYKEVLSFKPPRQLGARVSKETPL (SEQ ID NO: 29), RVTYKEVLSFKPPRQLGARVSKETPL (SEQ ID NO: 30), KRVTYKEVLSFKPPRQLGARVSKETPL (SEQ ID NO: 31), ETAL (SEQ ID NO: 32), KETAL (SEQ ID NO: 33), PKETAL (SEQ ID NO: 34), VPKETAL (SEQ ID NO: 35), RVPKETAL (SEQ ID NO: 36), ARVPKETAL (SEQ ID NO: 37), GARVPKETAL (SEQ ID NO: 38), LGARVPKETAL (SEQ ID NO: 39), QLGARVPKETAL (SEQ ID NO: 40), RQLGARVPKETAL (SEQ ID NO: 41), PRQLGARVPKETAL (SEQ ID NO: 42), PPRQLGARVPKETAL (SEQ ID NO: 43), KPPRQLGARVPKETAL (SEQ ID NO: 44), FKPPRQLGARVPKETAL (SEQ ID NO: 45), SFKPPRQLGARVPKETAL (SEQ ID NO: 46), LSFKPPRQLGARVPKETAL (SEQ ID NO: 47), VLSFKPPRQLGARVPKETAL (SEQ ID NO: 48), EVLSFKPPRQLGARVPKETAL (SEQ ID NO: 49), KEVLSFKPPRQLGARVPKETAL (SEQ ID NO: 50), YKEVLSFKPPRQLGARVPKETAL (SEQ ID NO: 51), TYKEVLSFKPPRQLGARVPKETAL (SEQ ID NO: 52), VTYKEVLSFKPPRQLGARVPKETAL (SEQ ID NO: 53), RVTYKEVLSFKPPRQLGARVPKETAL (SEQ ID NO: 54), and KRVTYKEVLSFKPPRQLGARVPKETAL (SEQ ID NO: 55)..

In some embodiments, the variant of p38y may comprise a part of p38y but otherwise differ from the wild-type p38y. In this regard, the inventors envisage that variants of p38y may include protein in which the PDZ interaction motif of p38y is fused to the carboxy-terminus of other kinases, such as MAP kinase or other serine/threonine kinases, or variants of other kinases that carry mutations to modify their activity. For example, the variant of p38y may comprise the PDZ interaction motif of p38y fused to the carboxy-terminus of a kinase selected from the group consisting of p38$\alpha$, p38$\beta$ and p38$\delta$, or variants of p38$\alpha$, p38$\beta$ and p38$\delta$ that carry mutations that modify their activity.

In one embodiment, the variant of p38y is an active variant of p38y. An active variant of p38y is a variant which does not require activation by the MAP kinase kinases MKK3 and MKK6 in order to exhibit p38y activity. In one embodiment, the active variant of p38y is a constitutively active variant of p38y. A constitutively active variant of p38y is a variant of p38y which is continuously active and therefore does not require activation by the MAP kinase kinases MKK3 and MKK6. Typically, a constitutively active variant comprises one or more amino acid substitutions which result in continuous activity. In one embodiment, the constitutively active variant of p38y comprises the amino acid substitution of D179A. The amino acid sequence of an example of a constitutively active variant of p38y is shown in Figure 44 (SEQ ID NO: 3). In another embodiment, the constitutive active variant of p38y may comprise the amino acid substitution of F330L/S. The substitution of F330L/S in p38y corresponds to the substitution of the constitutive active variant of p38$\alpha$ F327L/S.

In one embodiment, there is provided a method of treating Alzheimer's disease in a subject, comprising administering a nucleic acid sequence which expresses p38y or a variant thereof, typically a constitutively active variant of p38y, in neurons of the subject.

In one embodiment, there is provided a method of treating stroke in a subject, comprising administering a nucleic acid sequence which expresses p38y or a variant thereof, typically a constitutively active variant of p38y, in neurons of the subject.

In one embodiment, there is provided a method of treating epilepsy in a subject, comprising administering a nucleic acid sequence which expresses p38y or a variant thereof, typically a constitutively active variant of p38y, in neurons of the subject.

In another embodiment, the subject is treated by administering an agent that introduces into neurons of the subject a variant of tau that causes disruption of the tau-dependent signalling complex. As used herein, a "variant of tau" is a tau protein comprising one or more amino acid substitutions, insertions, or deletions, of the full length wild-type tau, wherein the one or more deletions is not more than 100 contiguous amino acids, typically not more than 90, 80, 70, 60, 50, 40, 30, 20, or 10 contiguous amino acids. In one embodiment, the variant of tau comprises one or more amino acid substitutions or insertions of the wild-type tau. In one embodiment, the variant of tau comprises one or more amino acid substitutions of the wild-type tau. In one embodiment, the variant of tau is a phosphomimetic of tau that causes disruption of the tau-dependent signalling complex. As used herein, a phosphomimetic of tau is a variant of tau comprising one or more amino acid substitutions, and which functions in a manner that is the same as, or substantially the same as, that of unsubstituted tau following phosphorylation of the unsubstituted tau at a particular amino acid. A phosphomimetic comprises a phosphomimetic substitution.

As described in the Examples, the inventors have shown that introduction of a T205E variant of tau into hippocampal neurons lowered Aβ-induced toxicity in the neurons. The T205E variant of Tau is a phosphomimetic of Tau phosphorylated at T205. A phosphomimetic substitution is an amino acid substitution in a protein which results in the protein functioning in a manner which is the same as, or substantially the same as, the unsubstituted protein following phosphorylation of the unsubstituted protein. A phosphomimetic substitution of tau is an amino acid substitution at a site of tau which results in a tau protein that functions in the same, or substantially the same, manner to the wild-type tau following phosphorylation of the wild-type tau, typically at that site.

In one embodiment, the method comprises treating the subject to introduce a phosphomimetic of tau comprising a phosphomimetic substitution of tau that causes disruption of, or reduces formation of, the tau-dependent signalling complex. In one embodiment, the one or more phosphomimetic substitutions are at amino acid residues of the tau protein that are phosphorylated by p38γ. In one embodiment, the phosphomimetic substitution of tau is threonine to glutamic acid at position 205 of tau (T205E), with amino acid numbering based on the longest human tau isoform comprising 441 amino acids. The amino acid sequence of full-length wild-type human tau (SEQ ID NO: 4) and tau T205E (SEQ ID NO: 5) is shown in Figure 45.

Typically, the variant of tau is a variant of human tau. In other embodiments, the variant of tau may be a variant of tau from a non-human mammal. For example, the variant of tau may be a variant of tau from a mouse, dog, cat, pig, cow, rat, non-human primate, goat, sheep.

In one embodiment, there is provided a method of treating Alzheimer's disease in a subject, comprising administering a nucleic acid sequence which expresses tau which differs from wild-type tau in an amino acid substitution of threonine to glutamic acid at position 205 (T205E), in neurons of the subject.

In one embodiment, there is provided a method of treating stroke in a subject, comprising administering a nucleic acid sequence which expresses tau which differs from wild-type tau in an amino acid substitution of threonine to glutamic acid at position 205 (T205E), in neurons of the subject.

In one embodiment, there is provided a method of treating epilepsy in a subject, comprising administering a nucleic acid sequence which expresses tau which differs from wild-type tau in an amino acid substitution of threonine to glutamic acid at position 205 (T205E), in neurons of the subject.

In embodiments in which the agent comprises a nucleic acid that is capable of expressing p38γ or a variant thereof, or the variant of tau, in neurons of the subject, a nucleic acid sequence encoding p38γ or a variant thereof, or the variant of tau, is typically operably linked to regulatory sequence to direct expression of the p38γ, or variant thereof, or the variant of tau, in the neurons of the subject. A nucleic acid that is capable of expressing p38γ or a variant thereof, or a variant of tau, in neurons of a subject may comprise an expression cassette comprising the coding sequence of p38γ or variant thereof, or the variant of tau. An expression cassette is a nucleic acid sequence comprising coding sequence and regulatory sequence which operate together to express a protein encoded by the coding sequence in a cell. "Coding sequence" refers to a DNA or RNA sequence that codes for a specific amino acid sequence. It may constitute an "uninterrupted coding sequence", i.e., lacking an intron, such as in a cDNA, or it may include one or more introns bounded by appropriate splice junctions.

The expression cassette typically includes regulatory sequences. A "regulatory sequence" is a nucleotide sequence located upstream (5' non-coding sequences), within, or downstream (3' non-coding sequences) of a coding sequence, and which influences the transcription, RNA processing or stability, or translation of the associated coding sequence. Regulatory sequences are known in the art and may include, for example, transcriptional regulatory sequences such as promoters, enhancers translation leader sequences, introns, and polyadenylation signal sequences. The coding sequence is typically operably linked to a promoter. A promoter is a DNA region capable under certain conditions of binding RNA polymerase and initiating transcription of a coding sequence usually located downstream (in the 3' direction) from the promoter. The coding sequence may also be operably linked to termination signals. The expression cassette may also include sequences required for proper translation of the coding sequence. The expression cassette including the coding sequence may be chimeric. A "chimeric" vector or expression cassette, as used herein, means a vector or cassette including nucleic acid sequences from at least two different species, or has a nucleic acid sequence from the same species that is linked or associated in a manner that does not occur in the "native" or wild type of the species. The coding sequence in the expression cassette may be under the control of a constitutive promoter or of a regulatable promoter that initiates transcription only in a particular tissue or cell type, or when the host cell is exposed to some particular stimulus. For example, in an expression cassette comprising a nucleic acid encoding p38γ, the coding sequence may be operably linked to a promoter which is not native to the p38γ gene, such as a promoter that expresses the coding sequence in, or is inducible in, neurons. Examples of suitable neural promoters include synapsin (SYN), calcium/calmodulin-dependent protein kinase (CaMKII), tubulin alpha I (Ta1), neuron-specific enolase (NSE), platelet derived growth factor beta chain (PDGF), MfP, dox, GFAP, Preproenkephalin, dopamine β-hydroxylase (dβH), prolactin, chicken beta actin, prion protein, murine Thy1.2, myelin basic promoter, or any of the above combined with an enhancer, such as a partial cytomegaly virus promoter. Examples of other promoters which may be used to express nucleic acid sequence in neurons include, the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, synthetic promoters, hybrid promoters, and the like. Inducible or controllable promoters include, for example, promoters whose transcriptional activity is modified in the presence or absence of mifepristone, doxycycline, tetracycline or tamoxifen.

A nucleic acid encoding a protein (coding sequence) is operably linked to a regulatory sequence when it is arranged relative to the regulatory sequence to permit expression of the protein in a cell. For instance, a promoter is operatively linked to a coding region if the promoter helps initiate transcription of the coding sequence.

As used herein, "expression" of a nucleic acid sequence refers to the transcription and translation of a nucleic acid sequence comprising a coding sequence to produce the polypeptide encoded by the coding sequence.

In one embodiment, the agent is a vector. In such vectors, the nucleic acid sequence encoding p38y or variant thereof, or the variant of tau, or an expression cassette comprising such sequences, is inserted into an appropriate vector sequence. The term "vector" refers to a nucleic acid sequence suitable for transferring genes into a host cell, such as a neuron. The term "vector" includes plasmids, cosmids, naked DNA, viral vectors, etc. In one embodiment, the vector is a plasmid vector. A plasmid vector is a double stranded circular DNA molecule into which additional sequence may be inserted. The plasmid may be an expression vector. Plasmids and expression vectors are known in the art and described in, for example, Sambrook et al. Molecular Cloning: A Laboratory Manual, 4th Ed. Vol. 1-3, Cold Spring Harbor, N.Y. (2012).

In some embodiments, the vector is a viral vector. Viral vectors comprise viral sequence which permits, depending on the viral vector, viral particle production and/or integration into the host cell genome and/or viral replication. Viral vectors which can be utilized with the methods and compositions described herein include any viral vector which is capable of introducing a nucleic acid into neurons, typically neurons of the brain. Examples of viral vectors include adenovirus vectors; lentiviral vectors; adeno-associated viral vectors; Rabiesvirus vectors; Herpes Simplex viral vectors; SV40; polyoma viral vectors; poxvirus vector.

In one embodiment, the viral vector is an adeno-associated viral (AAV) vector for packaging in an adeno-associated virus. In one embodiment, the AAV vector is a serotype selected from the group consisting of AAV1, AAV2, AAV3, AAV4, AAV5, AAV6, AAV6.2, AAV7, AAV8, AAV9, AAVrh10, AAVrh20, AAVrh39, AAVrh43, and AAVcy5 vector or variants thereof. In one embodiment, the viral vector is serotype AAV1, AAV9, AAVrh10 or AAVcy5. In one embodiment, the serotype of the AAV vector is AAV1. In another embodiment, the serotype of the AAV vector is AAV9. In another embodiment, the serotype of the AAV vector is AAVrh10. In another embodiment, the serotype of the AAV vector is AAVcy5. The use of recombinant AAV for introducing nucleic acids into cells is known in the art and described in, for example, US20160038613; Grieger and Samulski (2005) Adeno-associated virus as a gene therapy vector: vector development, production and clinical applications, Advances in Biochemical Engineering/Biotechnology 99: 119-145; Methods for the production of recombinant AAV are known in the art and described in, for example, Harasta et al (2015) Neuropsychopharmacology 40: 1969-1978. An example of an adeno-associated viral vector capable of expressing p38y in neuronal cells is shown in Figures 46 and 48 (SEQ ID NO: 6). An example of an adeno-associated viral vector capable of expressing p38$\gamma^{CA}$ in neuronal cells is shown in Figures 47 and 49 (SEQ ID NO: 7). In one embodiment, the viral vector comprises SEQ ID NO: 6 or 7. In one embodiment, the viral vector comprises SEQ ID NO: 7.

In another embodiment, the viral vector is a lentiviral vector. Methods for production and use of lentiviral vectors are known in the art and described in, for example, Naldini et al. (1996) In vivo gene delivery and stable transduction of nondividing cells by a lentiviral vector, Science, 272:263-267; Lois et al. (2002) Germline transmission and tissue-specific expression of transgenes delivered by lentiviral vectors, Science,295:868-872; Vogel et al (2004), A single lentivirus vector mediates doxycycline-regulated expression of transgenes in the brain. Hum Gene Ther. 2004;15(2):157-165.

Adenoviruses are also contemplated for use in delivery of nucleic acid agents. Thus, in another embodiment, the viral vector is an adenoviral vector. Adenoviral vectors are known in the art and described in, for example, Kozarsky and Wilson, Current Opinion in Genetics and Development 3:499-503 (1993); Southgate et al. (2008) Gene transfer into neural cells in vitro using adenoviral vectors, Current Protocols in Neuroscience, Unit 4 23, Chapter 4; Akli et al. (1993)Transfer of a foreign gene into the brain using adenovirus vectors. Nature genetics, 3(3): 224-228.

Another aspect provides a vector as described herein, typically a viral vector as described herein.

Viral vectors are typically packaged into viral particles using methods known in the art. The viral particles may then be used to transfer cell lines, including neural cell lines, or neural tissue, either *in vitro* or *in vivo*. Thus, another aspect provides a viral particle comprising a vector described herein.

A further aspect provides an agent as described herein.

The agent described herein may be formulated as a pharmaceutical composition. Accordingly, in another aspect, there is provided a pharmaceutical composition comprising the agent described herein. The composition comprises the agent in a pharmaceutically acceptable carrier. Methods for the formulation of agents with pharmaceutical carriers are known in the art and are described in, for example, Remington's Pharmaceutical Science, (17th ed. Mack Publishing Company, Easton, Pa. 1985); Goodman & Gillman's: The Pharmacological Basis of Therapeutics (11th Edition, McGraw-Hill Professional, 2005) .

Acceptable carriers, diluents and adjuvants are nontoxic to recipients and are preferably inert at the dosages and concentrations employed, and include buffers such as phosphate, citrate, or other organic acids; antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics or polyethylene glycol (PEG).

Administration of the agent to subject may be by intracranial, intravenous, intraperitoneal, subcutaneous, intramuscular, intranasal or intrathecal injection. Compositions suitable for intracranial, intravenous, intraperitoneal, subcutaneous, intramuscular, intranasal or intrathecal use include sterile aqueous solutions or dispersions and sterile powders for the extemporaneous preparation of sterile injectable solutions or dispersions. The pharmaceutically acceptable carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride.

In embodiments in which the agent is packaged in a viral particle, the pharmaceutical compositions may comprise viral particles in any concentration that allows the agent to be effective. In such embodiments, the pharmaceutical compositions may comprise the virus particle in an amount of from 0.1% to 99.9% by weight. Pharmaceutically acceptable carriers include water, buffered water, saline solutions such as, for example, normal saline or balanced saline solutions such as Hank's or Earle's balanced solutions), glycine, hyaluronic acid etc.

Titers of viral particles to be administered will vary depending on, for example, the particular vector to be used, the mode of administration, extent of the condition, the individual, and may be determined by methods standard in the art.

The agent described herein may be formulated for introduction into neuronal cells by non-viral methods such as microinjection, electroporation, microparticle bombardment, liposome uptake, nanoparticle-based delivery etc.

In one embodiment, the agents described herein may be formulated in one or more liposomes, lipoplexes, or lipid nanoparticles. In one embodiment, the agents described herein are formulated in liposomes. Liposomes are unilamellar or multilamellar vesicles which have a membrane formed from a lipophilic material and an aqueous interior. The aqueous portion contains the composition to be delivered. Liposome design may include, for example, opsonins or ligands in order to improve the attachment of liposomes to tissue or to activate events such as, for example, endocytosis.

The formation of liposomes may depend on the physicochemical characteristics such as the agent and the liposomal ingredients, the nature of the medium in which the lipid vesicles are dispersed, the effective concentration of the agent, any additional processes involved during the application and/or delivery of the vesicles, the optimization size, polydispersity and the shelf-life of the vesicles for the intended application, and the batch-to-batch reproducibility and possibility of large-scale production of safe and efficient liposomal products.

Methods for the production of liposomes and lipid nanoparticles for delivery of agents are known in the art, and described in, for example, US 5,264,221.

The term "administering" should be understood to mean providing a compound or agent to a subject in need of treatment.

It will be understood that the specific dose level and frequency of dosage for any particular subject may be varied and will depend upon a variety of factors including, for example, the activity of the specific compound or agent employed, the metabolic stability and length of action of that compound or agent, the age, body weight, general health, sex, diet, mode and time of administration, drug combination, the severity of the particular condition, and the host undergoing therapy.

Also provided is a kit, comprising a container comprising the agent. The container may be simply a bottle comprising the agent in parenteral dosage form, each dosage form comprising a unit dose of the agent. The kit will further comprise printed instructions. The article of manufacture will comprise a label or the like, indicating treatment of a subject according to the present method. In one form, the article of manufacture may be a container comprising the agent in a form for parenteral dosage. For example, the agent may be in the form of an injectable solution in a disposable container.

As used herein, "treating" means affecting a subject, tissue or cell to obtain a desired pharmacological and/or physiological effect and includes inhibiting the condition, i.e. arresting its development; or relieving or ameliorating the effects of the condition i.e. cause reversal or regression of the effects of the condition.

As used herein, "preventing" means preventing a condition from occurring in a cell or subject that may be at risk of having the condition, but does not necessarily mean that condition will not eventually develop, or that a subject will not eventually develop a condition. Preventing includes delaying the onset of a condition in a cell or subject.

The inventors envisage that p38y or variants of tau can be used in transgenic animals to assess whether a neurological disease can be treated with the methods described herein.

Accordingly, a further aspect that is not part of the invention, provides a transgenic non-human animal comprising a transgenic nucleic acid sequence which is capable of expressing in neurons of the transgenic animal p38y or a variant thereof, or a variant of tau that causes disruption of the tau-dependent signalling complex.

In one embodiment, the transgenic nucleic acid sequence is a nucleic acid sequence capable of expressing p38y or a variant thereof. In one embodiment, the transgenic nucleic acid sequence is capable of expressing an active variant of p38y. In one embodiment, the active variant of p38y is a constitutively active variant of p38y. In one embodiment, the constitutively active variant of p38y is p38$\gamma^{CA}$.

The regulatory sequences for expressing the transgene in neurons of the animal are described above.

In one embodiment, the transgenic animal is a mouse. However, it will be understood that the transgenic animal may be any animal, including, for example, a rat, cow, sheep, pig or goat.

Another aspect that is not part of the invention provides a method of assessing whether a neurological condition can be treated or prevented by a method described herein, comprising the steps of:

(a) providing a test animal suffering from the neurological condition or exhibiting a phenotype which is a model for the neurological condition;
(b) crossing the test animal with a transgenic animal to obtain progeny, the transgenic animal comprising a transgenic nucleic acid sequence which is capable of expressing in neurons of the animal p38y or a variant thereof, or a variant of tau that causes disruption of the tau-dependent signalling complex; and
(c) assessing the severity of the neurological condition or the phenotype which is a model for the neurological condition in progeny expressing the transgenic nucleic acid sequence.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

The following non-limiting examples are provided.

**Examples**

*Materials and Methods*

*Mice.* APP23 mice expressing human K670N/M671L mutant APP in neurons (C Sturchler-Pierrat et al., Proc Natl Acad Sci U S A 94, 13287-92 (1997)), Alz17 mice expressing human non-mutant tau in neurons (A Probst et al., Acta Neuropathol 99, 469-81 (2000)), neuron-specific Thy1.2-cre transgenic mice (I Dewachter et al., J Neurosci 22, 3445-53 (2002)), *tau*$^{-/-}$ (KL Tucker, M Meyer, YA Barde, Nat Neurosci 4, 29-37 (2001)), *p38$\alpha^{loxP/loxP}$* (FB Engel et al., Genes Dev 19, 1175-87 (2005)), *p38$\beta^{-/-}$* and *p38$\gamma^{-/-}$* (AR Pogozelski et al., PLoS One 4, e7934 (2009)), and *p38$\delta^{-/-}$* mice (G Sumara et al., Cell 136, 235-48 (2009)) were previously described. Knockouts for p38$\beta$, p38y and p386 were global without overt phenotypes, while p38$\alpha$ deletion had to be limited to the CNS due to embryonic mortality of global p38$\alpha$ knockout mice. To obtain *p38$\alpha^{\Delta neu}$* mice, we crossed *p38$\alpha^{loxP/loxP}$* with Thy1.2-cre strain. All lines were maintained on a C57Bl/6 background. Animal experiments were approved by the Animal Ethics Committee of the University of New South Wales. Mice were genotyped by polymerase chain reaction using isopropanol-precipitated DNA from tail biopsies as template. Oligonucleotide primers for genotyping targeted alleles and transgenes by PCR are listed in the following Table 1:

Table 1:

| | Forward primer (5'-3') | SEQ ID NO: | Reverse primer (5'-3') | SEQ ID NO:: |
|---|---|---|---|---|
| APP23 | GTTCTGCTGCATCTTGGACA | 56 | GAATTCCGACATGACTCAGG | 57 |
| Alz17 | GGGTGTCTCCAATGCCTGCTTCTTCAG | 58 | AAGTCACCCAGCAGGGAGGTGCTCAG | 59 |
| $p38\alpha$lox | TCCTACGAGCGTCGGCAAGGTG | 60 | AGTCCCCGAGAGTTCCTGCCTC | 61 |
| $p38\beta$ | AGAAGATGAAGGTGGAGGAGTACAAGC AAG | 62 | TAACCCGGATGGCTGACTGTTCCATTTA G | 63 |
| | | | | |
| $p38\gamma$ | TGGGCTGCGAAGGTAGAGGTG | 64 | GTGTCACGTGCTCAGGGCCTG | 65 |
| $p38\delta$ | ACGTACCTGGGCGAGGCGGCA | 66 | GCTCAGCTTCTTGATGGCCAC | 67 |
| $tau^{WT}$ | CTCAGCATCCCACCTGTAAC | 68 | CCAGTTGTGTATGTCCACCC | 69 |
| $tau^{KO}$ | AAGTTCATCTGCACCACCG | 70 | TGCTCAGGTAGTGGTTGTCG | 71 |
| Thy1.2-Cre | GCGGTCTGGCAGTAAAAACTATC | 72 | GTGAAACAGCATTGCTGTCACTT | 73 |
| Thy1.2-38$\gamma^{CA}$ | AAGTCACCCAGCAGGGAGGTG | 74 | TCGTATGGGTACATGGCCAAAG | 75 |

*Generation of transgenic Thy1.2-p38γ$^{CA}$ mice.*

The human *p38γ* coding sequence carrying the D179A mutation and an N-terminal hemagglutinin (HA)-tag was amplified by PCR and inserted into the XhoI site of the plasmid pEX12 (Ittner, et al. Proc. Natl. Acad. Sci. U.S.A. 105, 15997-16002) (2008)) carrying the *mThy1.2* promoter for neuronal expression using Gibson assembly (Gibson, et al. Nat. Methods 6, 343-345 (2009) (Fig. 30A). The construct was excised by restriction digest and transgenic founder mice were generated on a congenic C57Bl/6 background by pronuclear injection (Ittner et al. Nat. Protoc. 2, 1206-1215 (2007)). Tail DNA from founder mice was screened by PCR for genomic transgene insertion and 2 founder lines (*p38γ*$^{CA}$.3 and *p38γ*$^{CA}$.4) were established by crossing to C57Bl/6 mice. Normal fertility, survival and Mendelian transgene transmission was observed for both *p38γ*$^{CA}$.3 and *p38γ*$^{CA}$.4 lines. Both lines show no overt phenotype. Immunoblots of cortical, hippocampal, and cerebellar brain extracts from transgenic mice confirmed expression of HA-tagged p38γ$^{CA}$ (Fig. 30B) .

*Seizures.*

Seizures were induced with pentylenetetrazole (PTZ, Sigma-Aldrich) as previously described (LM Ittner et al., Cell 142, 387-97 (2010)). Briefly, PTZ was injected i.p. at 30 or 50 mg/kg body weight. Seizures were graded as: 0, no seizures; 1, immobility; 2, tail extension; 3, forelimb clonus; 4, generalized clonus; 5, bouncing seizures; 6, full extension; 7, status epilepticus.

*Spatial learning/memory testing.*

Spatial learning/memory was tested in the Morris Water maze paradigm (CV Vorhees, MT Williams, Nat Protoc 1, 848-58 (2006)). Briefly, a custom-built water tank for mouse Morris Water maze (122 cm diameter, 50 cm height) with white non-reflective interior surface in a room with low-light indirect lighting was filled with water (19 - 22 °C) containing diluted non-irritant white dye. Four different distal cues were placed surrounding the tank at perpendicular positions reflecting 4 quadrants. In the target quadrant, a platform (10cm$^2$) was submerged 1 cm below the water surface. Videos were recorded on CCD camera and analyzed using AnyMaze Software. For spatial acquisition, four trials of each 60 seconds were performed per session. The starting position was randomized along the outer edge of the start quadrant for all trials. To test reference memory, probe trials without platform were performed for a trial duration of 60 seconds, and recordings were analyzed for time spent within each quadrant. For visually-cued control acquisition (to exclude vision impairments), a marker was affixed on top of the platform and four trials (60 s) per session were performed. All mice were age and gender-matched and tested at 4 months of age. Mice that displayed continuous floating behavior were excluded. Genotypes were blinded to staff recording trials and analyzing video tracks. Tracking of swim paths was done using the AnyMaze software (Stölting). Average swimming speed was determined to exclude motor impairments.

Touchscreen operant chambers (Campden Instruments) were used with 2 different paradigms to address spatio-temporal memory and learning (differential paired-associates learning, dPAL) or recognition memory/discrimination learning (pairwise discrimination task, PD). Previously described touchscreen chamber protocols were used (Horner et al. Nat. Protoc. 8, 1961-1984 (2013)). Mice in dPAL schedule underwent pre-testing procedures and training as follows: food deprivation (to 85 - 90% of initial body weight) and adaptation to handling (day 0 - 4), adaptation to touchscreen boxes (day 4), collect reward (strawberry milk shake, Nippy's) (day 5 -8), panel-pushing to collect reward training (day 9), initial stimulus-dependent touch training (day 10), must touch stimulus training (day 11 - 16), must initiate trial training (day 17 - 22), punish incorrect touches (day 23 - 26). Followed by either dPAL acquisition for 21 consecutive days (day 27 - 49) or pairwise discrimination task acquisition (day 27 - 31). Maximum time of sessions was set to 60 minutes. Maximum number of trials was set to 36. All training sessions were repeated until mice reached criterion before next training paradigm was started. Criterion was defined as 36 trials within 60 minutes (initial touch training, must touch training, must initiate training) or 27 out of 36 correct trials (punish incorrect touches). Mice with excessive body weight loss were excluded from the protocol.

Behavior and motor testing

Novelty-induced locomotion and anxiety-related behavior was assessed in the open field test paradigm as previously described (Ke, et al. Acta Neuropathol. 130, 661-678 (2015)). Briefly, mice were placed individually in 40 × 40 cm$^2$ boxes in dimly lit sound-insulated enclosures and movements were recorded for 15 minutes. Mice had not been exposed to open field paradigm before. Boxes were wiped with 70 % ethanol between recordings. Movements were tracked using the AnyMaze software (Stölting). Analysis was either accumulated over entire recording period or split in 1-minute bins.

Motor performance was tested on a 5-wheel Rota-Rod treadmill (Ugo Basile) in acceleration mode (5-60rpm) over 120 (aged) or 180 (young) seconds (van Eersel, et al. Neuropathol. Appl. Neurobiol. 41, 906-925 (2015)). The longest time each mouse remained on the turning wheel out of 3 attempts per session was recorded. Grip strength was determined as previously described (Ke et *al. (2015)*). Briefly, the force required to pull mice off a metal wire was measured using s grip strength meter (Chatillon, AMETEK). Mice were placed such that they had a double grip on a thin metal wire attached to the meter, and they were pulled away from the meter in a horizontal direction until they let go, and a peak force (N) was recorded at the moment when the mice let go. The highest force from three attempts was recorded.

Calcineurin activity assay

Calcineurin activity in cortical extracts of *p38γ*-/- and *p38γ*+/+ littermates was determined by following the manufacturer's instructions (Abcam).

*Electroencephalography.*

Hippocampal EEG recording in freely moving mice was carried out as previously described (AA Ittner, A Gladbach, J Bertz, LS Suh, LM Ittner, Acta Neuropathol Commun 2, 149 (2014)). Briefly, wire EEG electrodes of remote telemetric transmitters (DSI) were implanted in mice anesthetized with ketamine/xylazine. The head was fixed in a stereotactic frame (Kopf instruments) and the bregma was located. Bone openings were drilled using a bone micro-drill (Fine Science Tools, F.S.T.) at positions previously described for the hippocampus (x 2.0, y -2.0, z -2 with reference to bregma). Electrodes were inserted at this position with reference electrode placed above the cerebellum (x 0, y -6.0, z 0 from bregma). Electrodes were fixed in place by polyacrylate followed by wound closure and rehydration. Following 10 days of recovery from the surgery, EEGs were recorded with a DSI wireless receiver setup (DSI) with amplifier matrices using the Dataquest A.R.T. recording software at 500 Hz sampling rate (M Weiergraber, M Henry, J Hescheler, N Smyth, T Schneider, Brain Res Brain Res Protoc 14, 154-64 (2005)). Two days after EEG recordings were completed, animals were transcardially perfused with cold phosphate-buffered saline (PBS) and brains extracted for biochemical and histological analysis. Correct placement of electrodes was confirmed by serial sections of paraffin embedded brain tissue stained with hematoxylin-eosin. Only recordings from mice with correct placement of electrodes were included in further analysis.

Analysis of EEG recordings was performed using the NeuroScore software v3.0 (DSI) with integrated spike detection module, to determine spike train duration, frequency and number of spikes per train were obtained. Recordings were screened manually for movement artefacts and only artefact-free EEG passages were used for analysis. Raw local field potentials (LFP) were noise filtered using a powerline noise filter (Neuroscore, DSI). Spectral analysis (i.e. analysis of signal power at individual frequencies expressed as square of the fast Fourier transform (FFT) magnitude) of intra-ictal sequences was performed using the integrated FFT spectral analysis function of NeuroScore. Frequency bands of theta and gamma wave forms were defined between 4-12 Hz and 25-100 Hz, respectively. Gamma and theta spectral contributions were quantified by area-under-curve (AUC) analysis across the defined frequency band in 8 artefact- and hypersynchronous spike-free sequences per recording (each 1 min in length). Cross-frequency coupling of theta phase and gamma amplitude was performed using MATLAB as previously described (AB Tort, R Komorowski, H Eichenbaum, N Kopell, J Neurophysiol 104, 1195-210 (2010)). Briefly, for cross frequency coupling analysis, raw LFP was noise filtered using a powerline noise filter (Neuroscore, DSI). Noise-filtered LFP was filtered at two frequency ranges of interest for gamma ($f_A$) and theta ($f_p$). The phase time series for theta ($\Phi_{fp}(t)$) and the amplitude envelope time series for gamma ($A_{fA}(t)$) were obtained by Hilbert transformation of the filtered LFPs. The combined series [$\Phi_{fp}(t), A_{fA}(t)$] was then generated. After phase binning, the means $\overline{A}_{fA}(j)$ of $A_{fA}$ for each bin j were calculated and normalized using the sum

$$\sum_{j=1}^{N} \overline{A}_{fA}(j)$$

of $\overline{A}_{fA}(j)$ over N bins to generate phase-amplitude distribution P(j). The modulation index is based on calculating the Kullback-Leibler distance $D_{KL}$ between the non-uniform (i.e. coupled) phase-amplitude distribution P(j). The modulation index is based on calculating the Kullback-Leibler distance $D_{KL}$ between the non-uniform (i.e. coupled) phase-amplitude distribution $P(j)$ over all phase bins and the uniform (i.e. uncoupled) distribution $U(j)$.

$$D_{KL}(P,Q) = \sum_{j=1}^{N} P(j)\log\left[\frac{P(j)}{U(j)}\right]$$

The modulation index *MI* is defined as

$$MI = \frac{D_{KL}(P(j),U(j))}{\log(N)}$$

Phase-amplitude distributions and modulation indices were determined from artefact- and hypersynchronous spike-free 8 sequences (each 1 min) per recording.

Synaptosome and post-synaptic density preparation

Purification of synaptosomes from cortical tissue was performed as previously described (Ittner, et al. Cell 142, 387-397 (2010)). Briefly, cortical tissue was weighed and homogenized in ice-cold sucrose buffer (0.32M sucrose, 1mM NaHCO$_3$, 1mM MgCl$_2$, 0.5mM CaCl$_2$, protease inhibitors (EDTA-free, Roche)) at 30 mg tissue/ml using a pre-cooled dounce homogenizer. After clearing the homogenate by centrifugation (1,400g, 10 minutes, 4°C), pellets were resuspended in sucrose buffer and centrifuged again (1,400g, 10 minutes, 4°C). Combined supernatants were centrifuged again and supernatant (total brain homogenate) was spun at 13,800g for 10 minutes at 4°C. Pellet was resuspended in sucrose buffer and layered on top of 5% Ficoll (Sigma) and centrifuged at 45,000g for 45 minutes at 4°C. Pellet was resuspended in 5% Ficoll and layered on top of 13% Ficoll and centrifuged at 45,000g for 45 minutes at 4°C. The interface (synaptosomes) was collected, diluted in 5% Ficoll and centrifuged at 45,000g for 30 minutes at 4°C. Supernatant (non-synaptic) was collected and pellet was resuspended in pH8 buffer (20mM Tris pH8, 1% Triton- X100, 100mM NaCl, 1mM EGTA, 1mM EDTA, 0.5% sodium deoxycholate, 0.1% sodium dodecyl sulfate (SDS), protease inhibitors (EDTA-free, Roche)). After centrifugation at 40,000g for 30 minutes at 4°C, pellets (post-synaptic densities) were resuspended in 5% SDS. The supernatants constituted synaptic non-PSD associated proteins. Protein concentrations for different fractions was determined before preparing samples for Western blotting.

*Plasmids.*

Plasmids for expression of rat PSD95 (kind gift from Weidong Yao; Addgene plasmid #15463), Fyn kinase (kind gift from Filippo Giancotti; Addgene plasmid #16032) and NR2B (kind gift from Robert Malinow; Addgene plasmid #23998), and were obtained from the Addgene depository. For live cell fluorescence confocal imaging, PSD-95 was internally tagged with mCherry between PDZ domains 2 and 3 by megaprime PCR (Bryksinet al. Biotechniques 48, 463-465 (2010)) and tau variants were tagged with eGFP by cloning into peGFP-C1 (Clontech).

Coding sequences for human p38α, human p38β and human p38γ were cloned into pcDNA3.1 with an N-terminal HA-tag. Coding sequence for human p38δ was cloned in peGFP-C1. Mutations in *p38* coding sequences for generation of active variants (M Avitzour et al., FEBS J 274, 963-75 (2007)) and variants of p38γ lacking the PDZ motif (ΔPDZm) were generated using the Q5 site-directed mutagenesis kit (NEB). Coding sequence for human tau (441 amino acids) was cloned into pcDNA3.2/V5-DEST (Invitrogen). Phosphorylation-site mutants of tau were generated using the Q5 site-directed mutagenesis kit (NEB). Oligonucleootide primers for molecular cloning are listed in Table 2.

Table 2

|  | Forward primer (5'-3' ) | SEQ ID NO: | Reverse primer (5'-3' ) | SEQ ID NO: |
|---|---|---|---|---|
| tauS46D | CCTGAAAGAAgatCCCCTGCAGACCCCC | 76 | CCAGCGTCCGTGTCACCC | 77 |
| tauT50E | TCCCCTGCAGgaaCCCACTGAGG | 78 | GATTCTTTCAGGCCAGCG | 79 |
| tauT52E | GCAGACCCCCgaaGAGGACGGATC | 80 | AGGGGAGATTCTTTCAGG | 81 |
| tauT69E | TGCTAAGAGCgaaCCAACAGCGG | 82 | TCAGAGGTTTCAGAGCCC | 83 |
| tauT71E | GAGCACTCCAgaaGCGGAAGATG | 84 | TTAGCATCAGAGGTTTCAG | 85 |
| tauT111 E | CATTGGAGACgaaCCCAGCCTGG | 86 | CCTGCTTCTTCAGCTGTG | 87 |
| tauT153 E | GAAGATCGCCgaaCCGCGGGGAG | 88 | GTTTTACCATCAGCCCCC | 89 |

(continued)

| | Forward primer (5'-3' ) | SEQ ID NO: | Reverse primer (5'-3' ) | SEQ ID NO: |
|---|---|---|---|---|
| tauT181E | CGCTCCAAAGgaaCCACCCAGCTC | 90 | GGCGGGGTTTTTGCTGGA | 91 |
| TauS199A | CGGCTACAGCGCCCCCGGCTCCC | 92 | CTGCGATCCCCTGATTTTGGAG | 93 |
| TauS199D | CGGCTACAGCGACCCCGGCTCCC | 94 | CTGCGATCCCCTGATTTTGGAG | 95 |
| tauS202A | CAGCCCCGGCgccCCAGGCACTC | 96 | CTGTAGCCGCTGCGATCCCCTG | 97 |
| tauS202D | CAGCCCCGGCgacCCAGGCACTC | 98 | CTGTAGCCGCTGCGATCC | 99 |
| tauS208D | CACTCCCGGCgacCGCTCCCGCAC | 100 | CCTGGGGAGCCGGGGCTG | 101 |
| tauT212E | CCGCTCCCGCgaaCCGTCCCTTCCAAC | 102 | CTGCCGGGAGTGCCTGGG | 103 |
| tauS235D | TCCACCCAAGgacCCGTCTTCCGC | 104 | GTACGGACCACTGCCACC | 105 |
| TauS404A | TGGGGACACGGCTCCACGGCATC | 106 | GACACCACTGGCGACTTGTACACG | 107 |
| TauS404D | TGGGGACACGGATCCACGGCATC | 108 | GACACCACTGGCGACTTG | 109 |
| TauT205 A | CTCCCCAGGCGCTCCCGGCAGCC | 110 | CCGGGGCTGCTGTAGCCGC | 111 |
| TauT205 E | CTCCCCAGGCGAACCCGGCAGCCG | 112 | CCGGGGCTGCTGTAGCCG | 113 |
| TauS199AT205A | CCCAGGCGCTCCCGGCAGCCGCTCCCGC | 114 | GAGCCGGGGGCGCTGTAGCCGCTGCGATCCCC | 115 |
| TauS199DT205E | CCCAGGCGAACCCGGCAGCCGCTCCCGC | 116 | GAGCCGGGGTCGCTGTAGCCGCTGCGATCCCC | 117 |
| TauS396A | CGTGTACAAGGCGCCAGTGGTGT | 118 | ATCTCCGCCCCGTGGTCTG | 119 |
| TauS396D | CGTGTACAAGGACCCAGTGGTGTCTGGGG | 120 | ATCTCCGCCCCGTGGTCT | 121 |

(continued)

| | Forward primer (5'-3' ) | SEQ ID NO: | Reverse primer (5'-3' ) | SEQ ID NO: |
|---|---|---|---|---|
| TauS396 AS404A | TGGGGACACGGCTCCACGGC ATCTCAGCAAT | 122 | GACACCACTGGCGCCTTGTAC ACGATCTCCGC | 123 |
| TauS396 DS404D | TGGGGACACGGACCCACGGC ATCTCAGCAAT | 124 | GACACCACTGGGTCCTTGTAC ACGATCTCCGC | 125 |
| tauS422D | CATGGTAGACgatCCCCAGCTCG CCAC | 126 | TCGATGCTGCCGGTGGAG | 127 |
| tauS199A T205A | CCCAGGCGCTCCCGGCAGCCGCT CCCGC | 128 | GAGCCGGGGGCGCTGTAGCCGCTG CGATCCCC | 129 |
| tauS199D T205E | CCCAGGCGAACCCGGCAGCCGCT CCCGC | 130 | GAGCCGGGGTCGCTGTAGCCGCTG CGATCCCC | 131 |
| tauS396A S404A | TGGGGACACGGCTCCACGGCATC TCAGCAAT | 132 | GACACCACTGGCGCCTTGTACACG ATCTCCGC | 133 |
| tauS396D S404D | TGGGGACACGGACCCACGGCATC TCAGCAAT | 134 | GACACCACTGGGTCCTTGTACACG ATCTCCGC | 135 |
| mCherry PSD-95 | CAAGCCCAGCAATGCCTACCTGA GTGACGTGAGCAAGGGCGAGGAG G | 136 | CGAGGTTGTGATGTCTGGGGGAGC ATAGCTCTTGTACAGCTCGTCCAT GCC | 137 |

Adeno-associated virus vectors (von Jonquieres, et al. PLOS ONE 8, e65646 (2013)) for neuronal expression (pAM-CAG) of wildtype (Figure 46, Figure 48 (SEQ ID NO: 6)) and constitutively active (D179A) p38γ (Figure 47, Figure 49 (SEQ ID NO: 7)) or variants of tau were cloned by conventional restriction enzyme cloning. All plasmids were amplified in *E. coli* DH5α or XL-1blue. AAV vectors were propagated in E. coli Stbl3 to avoid recombination events. Constructs were verified by sequencing.

*Adeno-associated viruses.*

Packaging of rAAV1 vectors was performed as described (AE Harasta et al., Neuropsychopharmacology 40, 1969-78 (2015)). Titres were determined by Quantitative polymerase chain reaction (qPCR). One μl ($1 \times 10^9$ viral particles) of either AAV-SG1-shR or AAV-ctr-shR vector was injected at 3 sites each bilaterally into the brains of cryoanaesthetized neonatal mice as described (G von Jonquieres et al., PLoS One 8, e65646 (2013)).

*Cell culture.*

Primary hippocampal neurons from E16.5 mouse embryos were cultured, using our standard protocol (T Fath, YD Ke, P Gunning, J Gotz, LM Ittner, Nat Protoc 4, 78-85 (2009)). Cytotoxicity was determined by measuring LDH release, using a commercial assay (Promega), or by visualization of EthD1 (Thermo Fisher Scientific) added to the cell culture medium 5 min before fixation with 4% PFA/PBS. 293T cells were cultured in DMEM/10%FBS/1%Glutamate/1%P/S (Life Technologies) and transfected by calcium precipitation (A Ittner et al., J Exp Med 209, 2229-46 (2012)). Primary neurons were transduced by AAV infection (AE Harasta et al., Neuropsychopharmacology 40, 1969-78 (2015)).

*Live cell confocal imaging and FLIM/FRET analysis*

FLIM/FRET measurements were performed using a time resolved, inverted confocal fluorescence microscope (Microtime200, PicoQuant GmbH). Excitation of the donor GFP was via a single-photon fiber coupled pico-second-pulsed diode 473 nm laser (20 MHz repetition rate, 2 ms dwell time, 256 × 256 pixel array) using a 63x water objective (1.25 NA). Fluorescence emission was collected through a 510/32 Semrock BrightLine band pass emission filter onto a single-photon avalanche diode (SPAD) coupled to high speed timing electronics for time-correlated single-photon counting (TCSPC).

Fluorescence images were analysed by phasor plot using the SimFCS software (Globals Software, USA). Briefly, Fourier transformation of the decay curve at each pixel was performed and the resulting transforms were plotted as a 2D histogram. The phasor position for the donor only was determined by measuring the donor in the absence of the acceptor. The FRET samples were measured and the phasor position along the quenching trajectory is calculated according to classical FRET efficiency calculation:

$$E = 1 - \frac{\tau_{DA}}{\tau_D}$$

where E is FRET efficiency, $t_D$ is the fluorescence lifetime of the Donor in absence of acceptor, and $t_{DA}$ is the fluorescence lifetime in the presence of acceptor.

*Cell immunofluorescence staining and microscopy*

Cell staining was done as previously described ((LM Ittner et al., Cell 142, 387-97 (2010))). Briefly, cells were fixed with 4% PFA for 10 min, washed with phosphate buffered saline (PBS), permeabilised with 0.02% NP-40 and blocked with blocking buffer (3% horse serum/1% bovine albumin in PBS). Primary antibodies diluted in blocking buffer were incubated over-night at 4°C or for 1 hour at room temperature. After washing with PBS, secondary antibodies diluted in blocking buffer with or without addition of DAPI to visualize cell nuclei were incubated for 1 hour at room temperature. Cells were then washed and mounted using anti-fade mounting medium (Prolong Gold, Life Technologies). Secondary antibodies used were coupled to Alexa 488, 555, 568 or 647 dyes (Molecular Probes). Confocal images were acquired on a Zeiss LSM780 confocal microscope with a Plan-Apochromatic 100x 1.4 NA objective or on a Zeiss LSM880 Airyscan confocal microscope with a Plan-Apochromatic 100x 1.4 NA objective using the Zen software (Zeiss). Epifluorescence imaging was done on a BX51 bright field/epifluorescence microscope (UPlanFL N lenses [¥/0.17/FN26.5]: 10x/0.3, 20x/0.5, 40x/0.75, 60x/1.25oil and 100x/1.3oil) equipped with a DP70 color camera (Olympus) using CellSens software (Olympus).

*Human brain samples*

Human entorhinal cortex tissue samples were received from the New South Wales Brain Tissue Resource Centre at the University of Sydney and the Sydney Brain Bank at Neuroscience Research Australia, which are supported by The University of New South Wales, Neuroscience Research Australia and Schizophrenia Research Institute. Frozen tissue was lysed in phosphate buffered saline (20%w/v) using a rotating dounce homogeniser followed by five 1s sonication bursts at 20% power (Vibra Cell, Sonics). Lysates were centrifuged at 3,000xg for 10 minutes at 4°C and supernatants were used for analysis. Details on patients are provided in Table 3. Use of human brain samples was approved by the Human Research Ethics Committees of the University of New South Wales and University of Sydney.

Table 3

| Group | Age (y) | gender | PMI | CoD | APOE genotype | Braak |
|---|---|---|---|---|---|---|
| 0 | 93 | F | 21 | cardiac failure | E3/E3 | 0 |
| 0 | 85 | F | 23 | respiratory failure | E3/E3 | 0 |
| 0 | 79 | M | 8 | respiratory failure | E2/E3 | 0 |
| 0 | 89 | F | 23 | metastatic adenocarcinoma | E3/E4 | 0 |
| **0** | **86.5±3.0** | | **18.8±3.6** | | | |
| I/II | 78 | F | 11 | respiratory failure | E3/E3 | I |
| I/II | 80 | M | i< | respiratory failure | E3/E3 | I |
| I/II | 103 | M | 20 | cardiorespiratory failure | E3/E3 | II |
| I/II | 101 | F | 9 | cardiorespiratory failure | E3/E3 | II |

(continued)

| Group | Age (y) | gender | PMI | CoD | APOE genotype | Braak |
|---|---|---|---|---|---|---|
| I/II | 88 | F | 31 | cardiorespiratory failure | E3/E3 | II |
| **I/II** | **90.0±5.2** | | **16.6±4.1** | | | |
| III/IV | 93 | F | 7 | cardiorespiratory failure | E2/E3 | III |
| III/IV | 102 | F | 5 | acute renal failure | E2/E3 | IV |
| III/IV | 92 | F | 5 | infection | E3/E3 | IV |
| III/IV | 76 | F | 3 | cardiac failure | E3/E4 | IV |
| **III/IV** | **90.8±5.4** | | **5.0±0.8** | | | |
| V/VI | 98 | F | 11 | stroke | E3/E3 | VI |
| V/VI | 85 | F | 10 | cardiac failure | E3/E3 | VI |
| V/VI | 100 | F | 4 | pneumonia | E3/E4 | VI |
| V/VI | 100 | F | 3 | aspiration pneumonia | E3/E3 | VI |
| V/VI | 91 | F | 6 | cardiorespiratory failure | E3/E3 | VI |
| **V/VI** | **94.8±3.0** | | **6.8±1.6** | | | |
| PMI, post mortem interval; CoD, cause of death; bold values, mean ± SEM of group | | | | | | |

*Histological Sections and staining*

Mice were transcardially perfused with phosphate-buffered saline followed by 4% paraformaldehyde (PFA) and post-fixing in 4% PFA overnight. Tissue was processed in an Excelsior tissue processor (Thermo) for paraffin embedding. Thioflavin S staining to visualize amyloid plaques were performed following a standard protocol (LM Ittner et al., Cell 142, 387-97 (2010)). Muscle cross-sections were stained with primary antibodies to laminin (Sigma) as previously described (Ke, et al. Acta Neuropathol. 130, 661-678 (2015)). Brain sections from AAV-injected mice were stained with primary antibody to tau (Tau13; Abcam) or HA-tag (HA-7; Sigma-Aldrich) to visualize viral transgene expression. Serial paraffin sections of human entorhinal cortex samples were obtained from the NSW Brain Bank and stained with a standard Nissl protocol for counting. Neuronal counting was done on an Olympus BX51 microscope equipped with agraticulated ocular (U100H6; Olympus). Neurons with the nucleolus, nucleus and cytoplasm visible within a single plane of the section were considered for counting. Forthe CA fields (CA4-1), three random and non-overlapping fields of view were selected. For the entorhinal cortex, three non-overlapping strips of cortex extending from the pial surface and into the grey-white matter junction were marked for counting. Subsequent cortical counts were then performed across three adjacent graticule fields spanning perpendicularly to the pial surface. Mean cell counts across the section were then normalised into cell density values of neurons per mm2. All tissue sections were imaged on a BX51 bright field/epifluorescence microscope (UPlanFL N lenses [¥/0.17/FN26.5]: 10x/0.3, 20x/0.5, 40x/0.75, 60x/1.25oil and 100x/1.3oil) equipped with a DP70 color camera (Olympus).

Western Blotting

Western blotting was performed as previously described (A Ittner et al., J Exp Med 209, 2229-46 (2012)). Bands were visualized by chemiluminescence on X-ray films or ChemiDoc MP (Biorad). Densitometric quantification of Western blot results was performed using ImageJ 2.0.0-rc-49/1.51d (NIH). Antibodies used in this study were: anti-NR1 (Chemicon), anti-NR2B (Santa Cruz), antiphosphoTyrosine1473-NR2B (Affinity BioReagents), anti-PSD95 (Millipore), anti-Fyn (Santa Cruz), anti-phospho-Y418 Fyn (Invitrogen), anti-phospho-Y529 Fyn (Invitrogen), anti-APP (22C11), anti-Aβ (6E10), anti-tau (DAKO), anti-tau (tau-1, Millipore), anti-tau (Tau13, Abcam), anti-phospho-Serine199 tau (Abcam), anti-phospho-Serine202 tau (Abcam), anti-phospho-Threonine205 tau (Abcam), antiphospho-Threonine212 tau (Abcam), anti-phospho-Serine214 tau (Millipore), anti-phospho-Threonine231 tau (Abcam), anti-phospho-Serine235 tau (Abcam), anti-phospho-Serine356 tau (Abcam), anti-phospho-Serine396 tau (Abcam), anti-phospho-Serine404 tau (Millipore), anti-phospho-Serine422 tau (Millipore), PHF-1 (phospho-Serine396-phospho-Serine404 tau; kind gift by P. Davies), anti-p38alpha (Cell Signaling), anti-p38beta (Santa Cruz), anti-p38gamma (R&D), anti-p38delta (R&D), anti-phosphoThreonine180/Tyrosine182-p38 (Cell Signaling Technologies), anti-Flag (M2, Sigma), anti-HA7 (Sigma), anti-V5 (Invitrogen), anti-MAP2 (mouse Abcam), anti-MAP2 (chicken: Abcam), anti-β3 tubulin (Covance), anti-NeuN (Abcam), anti-Debrin (Sigma), anti-Synaptophysin (Abcam), anti-αsynuclein (Sigma), anti-glyceraldehyde dehydrogenase (anti-GAPDH, Millipore).

*Immunoprecipitation.*

Immunoprecipitation was performed from cell or tissue lysates as previously described (LM Ittner et al., Cell 142, 387-97 (2010)). Briefly, cells were lysed in pTNN buffer (20mM Tris pH7.4, 150mM NaCl, 1mM EDTA, 1mM Na3VO4, 1mM NaF, 1mM glycerophosphate, 2.5mM $Na_2H_2P_2O_7$, 1mM PMSF, protease inhibitors (Complete, Roche), 1% NP-40 substitute (Sigma-Aldrich)) on ice. Lysates were cleared by centrifugation (16,000×g/10 min/4°C). Protein concentration was determined (DC Protein Assay, BioRad) and 200ug of lysate incubated with antibody (1:400) for 3 h on a rotator at 4°C. Equilibrated and blocked protein G-beads (Life Technologies) were incubated with lysates for 45 min on a rotator at 4°C. Beads were then washed 3 times and incubated in sample buffer for 5 min at 95°C before SDS-PAGE. Cortical or hippocampal tissues were homogenized in RIPA buffer (20mM Tris pH8.0, 150mM NaCl, 1mM EDTA, 1mM Na3VO4, 1mM NaF, 1mM glycerophosphate, 2.5 mM sodium pyrophosphate, 1mM PMSF, protease inhibitors (Complete, Roche), 1% NP-40 substitute (Sigma-Aldrich), SDS, sodium deoxycholate) and subjected to immunoprecipitation as outlined above. Quantitative densitometric analysis was performed using Image J2.0.0-rc-49/1.5ld (NIH) and levels for immunoprecipiations of PSD-95/tau/Fyn complexes were expressed relative to immunopreciptateed PSD-95 protein levels.

Microscale thermophoresis (MST)

Tau variants were purified as GST-fusion proteins from E. coli BL21DE3pLys (Promega) using glutathione resin (GE Healthcare) followed by concentration and buffer exchange using ultrafiltration spin columns (10,000 molecular weight cut-off; Vivaspin, Sartorius). eGFP-PSD-95 was expressed in 293T cells and lysates were prepared in TNN buffer (20 mM Tris pH7.4, 150 mM sodium chloride, 1% NP40 substitute, sodium orthovanadate, sodium pyrophosphate, glycerophosphate, sodium fluoride, protease inhibitors (Complete; Roche)) 48 h after transfection. Concentrations of fusion proteins were determined by absorbance measurements (Nanodrop 2000C; Thermo-Fisher) using molar extinction coefficients. Thermophoresis of GFP-PSD-95 was measured on a Monolith NT115 (Nanotemper technologies) using 50 % LED power and 20 % MST power with 5 s pre-MST and 30 s MST-time with serials dilutions (1:1) of GST-tau (starting concentration 9 $\mu$M). Thermophoresis and temperature-jump normalized fluorescence curves from three independent experiments were expressed as fraction of the bound state of the fluorophores-tagged protein (Wienken et al. Nat. Commun. 1, 100 (2010)). Thermophoresis was plotted as a function of tau concentration and non-linear curves fitting to determine experimental equilibrium dissociation constants (KD) was performed using sum-of-squares minimization (Marquardt method; Graphpad Prism 6).

*Kinase assay.*

Recombinant proteins were expressed in bacteria and purified as previously described (A Ittner et al., J Exp Med 209, 2229-46 (2012)). Purity of proteins was assessed by SDS-PAGE and Coomassie staining. Kinase assay reactions were performed as previously described (A Ittner et al., J Exp Med 209, 2229-46 (2012)). Briefly, 0.5pg recombinant p38$\gamma$ was mixed with 1$\mu$g of recombinant human tau in kinase reaction buffer (Promega) and incubated for 30 min at 30°C. Kinase reactions were stopped by addition of sample buffer and incubation for 5 min at 95°C.

Mass spectrometry

Phospho-peptide mapping of tau after in vitro p38$\gamma$ kinase reactions was done as previously described (Dolai, et al. Cancer Res. 76, 2766-2777 (2016), Thingholm, et al. Nat. Protoc. 1, 1929-1935 (2006)). Briefly, kinase treated protein extracts containing tau were reduced with 3mM tris(2-carboxyethyl)phosphine (TCEP, 56oC, 10min), alkylated with 6 mM iodoacetamide (ambient temp, 30min), buffer exchanged and concentrated using 100mM ammonium bicarbonate and 3kDa spin-filters (Amicon Ultra-4 centrifugal filters, Merck KGaA, Darmstadt, Germany) followed by trypsin digest (25:1 w/w protein:trypsin ratio, 16h, 37oC). A portion of the material was enriched for phosphopeptides using Titansphere Phos-TiO kit, with TiO2 Spin tips (GL Sciences, Tokyo, Japan), following the manufacturer's protocol. Phosphopeptide enriched and non-enriched samples were analysed by LC-MS/MS using Orbitrap mass spectrometers (LTQ-Orbitrap Velos with CID and ETD activation modes and HCD on the QExactive Plus: Thermo Electron, Bremen, Germany) to maximize identification of phosphopeptides. Chromatography was carried out by nano-LC (Dionex UltiMate 3000 HPLC, Thermo Scientific, Waltham, USA) with autosampler system (Dionex, Amsterdam, Netherlands). Peptides (1-7pL injected) were initially captured on a C18 cartridge (Acclaim PepMap 100, Spm 100 Å, Thermo Scientific Dionex, Waltham, USA), switching to a capillary column (10cm) containing C18 reverse phase packing (Reprosil-Pur, 1.9 um, 200 Å, Dr. Maisch GmbH, Ammerbuch-Entringen, Germany), supported within a column heater (45°C, Sonation GmbH, Germany). Peptides were eluted using a 40min gradient of buffer A ($H_2O$:$CH_3CN$ of 98:2 containing 0.1% formic acid) to 45% buffer B ($H_2O$:$CH_3CN$ of 20:80 containing 0.1% formic acid) at 200nL/min, with high voltage applied at the column inlet. Mass spectrometer settings were: electrospray voltage 2000V, capillary temperature 275 - 300°C, positive ion mode, data dependent acquisition mode with a survey scan acquired (m/z 375-1750) and up to ten multiply charged ions (charge state ≥ 2+) isolated for MS/MS fragmentation (counts > 2500 for CID, >5000 for ETD and intensity threshold of 8.0x104 for HCD). Nitrogen was used as HCD collision gas and fluoranthene anion reagent for ETD. Peak lists were generated from the raw data using MASCOT Distiller (Matrix Science, London, England) and searched using the MASCOT search engine (version 2.5, Matrix Science) and the NCBInr database (downloaded 24-10-15) using *homo sapiens* taxonomy. Search parameters were: peptide tolerance of ± 4ppm and MS/MS tolerances of ± 0.4 Da for CID and ETD or ± 0.05 Da for HCD, variable modifications were carbamidomethyl cys, met oxidation, phospho (ST) and phospho (Y), peptide charge of 2+, 3+, and 4+, enzyme specificity trypsin with up to three missed cleavages allowed.

*Aβ preparation.*

Aβ42 (Bachem) was prepared and pre-aggregated at a concentration of 100 μM as described (MP Lambert et al., Proc Natl Acad Sci U S A 95, 6448-53 (1998)). Briefly, hexafluoro-2-propanol (Sigma) dissolved and evaporated Aβ was reconstituted in dimethyl sulfoxide (Sigma) at 5mM and then diluted in phenol-red free F-12 medium (Invitrogen) to a final concentration of 100μM, followed by brief vortexing and incubation at 4°C for 24 hours. Further dilutions were done in culture medium.

*Aβ levels and pathology.*

Aβ40 and Aβ42 and levels were determined by ELISA as previously described (LM Ittner et al., Cell 142, 387-97 (2010)). Plaque load was determined as previously described (LM Ittner et al., Cell 142, 387-97 (2010)).

*Statistical analysis.*

Statistical analysis was performed using Graphpad Prizm Version 6.0 (Student's t test or ANOVA). Linear regression and correlation analysis was done by sum of-squares minimization. Survival data were analyzed by log-rank Mantel-Cox testing. All values are presented as mean ± standard error of the mean (SEM).

*Results*

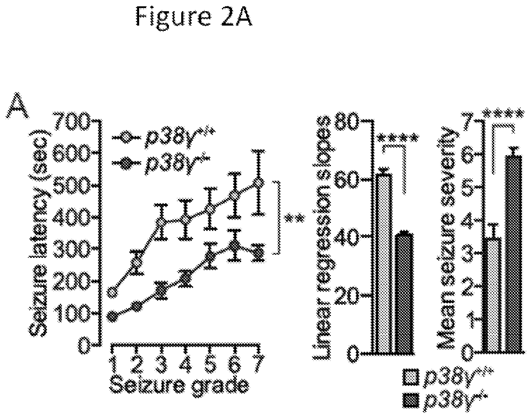

To understand the molecular contributions of p38 kinases to AD, we first challenged mice with individual deletion of *p38α*, *p38β*, *p38γ* or *p38δ* (Fig. 1) by inducing excitotoxic seizures with pentylenetetrazole (PTZ), an approach that has been instrumental in understanding excitotoxicity in AD mouse models (7, 8). The results are shown in Figs. 2A, 6A, 6B and 6C. Surprisingly, neither neuronal deletion of *p38α* (*p38α*Δneu), nor knockout of *p38β* or *p38δ* changed seizure latency and severity after PTZ administration, suggesting they have no modulatory role in acute excitotoxicity. In contrast, *p38γ* depletion (*p38γ*$^{-/-}$) markedly enhanced sensitivity to PTZ-induced seizures (Fig. 2A and fig. 6A, B and C). Pan-p38 inhibition increased severity and reduced latency of PTZ-induced seizures in wild-type mice similar to changes in *p38γ*$^{-/-}$, suggesting p38γ but not p38α/β/δ contribute to acute excitotoxicity. Consistent with a role in post-synaptic signaling, only p38γ localized to dendritic spines and post-synaptic densities of cultured neurons (Fig. 2B). p38α and p38β were found in soma and dendrite shafts, while p38δ was not detectable in neurons. Taken together, only p38γ localizes to the post-synaptic compartment and limits PTZ-induced excitotoxicity.

To test whether the effects of p38γ depletion on PTZ-induced seizures would also impact on Aβ-induced deficits in AD mouse models, we crossed *p38γ*$^{-/-}$ mice with mutant APP expressing APP23 mice. These APP23.p38γ$^{-/-}$ mice were assessed for seizure sensitivity by administering PTZ.

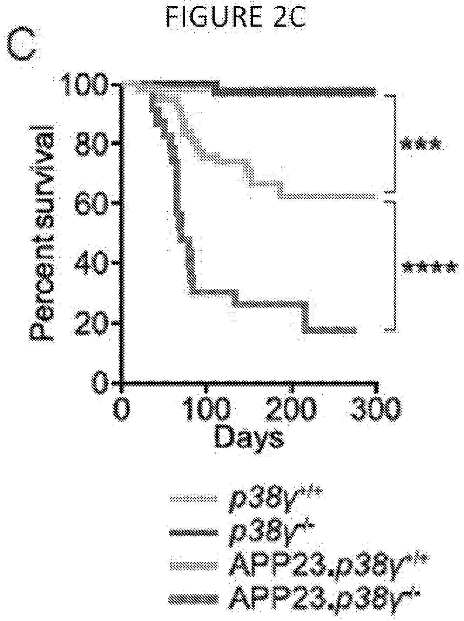

The results are shown in Fig. 7. The increased sensitivity of APP23 mice to PTZ-induced seizures was further augmented in APP23.*p38γ*$^{-/-}$ mice (fig. 7A-C). APP23 mice are characterized by premature mortality, memory deficits, neuronal circuit aberrations with epileptiform brain activity, and Aβ plaque pathology (Ittner et al., Cell 142, 387-397 (2010); ) Ittner, et al., Acta Neuropathol. Commun. 2, 149 (2014); Sturchler-Pierrat et al., Proc. Natl. Acad. Sci. U.S.A. 94, 13287-13292 (1997)). While Aβ formation and plaque pathology were comparable in brains of APP23.*p38γ*$^{-/-}$ and APP23.*p38γ*$^{+/+}$ mice, deletion of *p38γ* aggravated the premature mortality of APP23 mice, and 82% of APP23.*p38γ*$^{-/-}$ mice died by 8 months of age (Fig. 2C). *p38γ*$^{-/-}$ mice showed normal survival (Fig. 2C). Memory deficits in APP23.*p38γ*$^{-/-}$ were significantly more severe compared to those of APP23.*p38γ*$^{+/+}$ mice, as assessed in the Morris-water maze paradigm (Fig. 2D-F, fig. 8A-C), in differential paired associate learning (dPAL) (Fig. 21) and in a pairwise discrimination task (Fig. 22). In contrast, *p38γ*$^{-/-}$ mice showed wild-type-like memory performance and motor function. Memory deficits were associated with neuronal circuit aberrations and hypersynchronous epileptiform brain activity in APP transgenic lines (10), including APP23 (14). Electroencephalography (EEG) of APP23.*p38γ*$^{-/-}$ showed more frequent spontaneous seizure spike trains and interictal hypersynchronous discharges than APP23.*p38γ*$^{+/+}$ recordings (Fig. 2G-I). As can be seen from Fig. 2G, virtually no spike activity was found in $p38\gamma^{-/-}$ and $p38\gamma^{+/+}$ mice. Theta (4-8Hz) and gamma (25-100Hz) oscillations, both critical measures of hippocampal network activity related to learning and memory (18, 19), are altered in APP transgenic mice (14). Accordingly, theta spectral power was shifted to lower frequencies (4-8Hz) in APP23.$p38\gamma^{+/+}$ and more so APP23.$p38\gamma^{-/-}$ mice, while gamma spectral power was increased compared to $p38\gamma^{-/-}$ and $p38\gamma^{+/+}$ mice (fig. 9A-G). Hippocampal cross frequency coupling (CFC) through theta-phase modulation of gamma power (18) correlates with memory performance in rodents and humans (20, 21), and is impaired in APP23 mice (14). Interictal EEG traces showed CFC of similar magnitude at ~8Hz in $p38\gamma^{+/+}$ and $p38\gamma^{-/-}$ mice, but a marked impairment in APP23.$p38\gamma^{+/+}$ and virtual depletion 1 in APP23.$p38\gamma^{-/-}$ littermates (Fig. 2J), suggesting p38$\gamma$ depletion further exacerbates compromised CFC in APP23 mice. Similarly, synchrony of phase-amplitude distribution and theta phase was markedly reduced in APP23.$p38\gamma^{+/+}$, and virtually absent in APP23.$p38\gamma^{-/-}$ mice compared to $p38\gamma^{+/+}$ and $p38\gamma^{-/-}$ mice (fig. 9). Consequently, the modulation index, a robust measure of CFC (21), was significantly lower in APP23.$p38\gamma^{-/-}$ recordings as compared with $p38\gamma^{+/+}$ and $p38\gamma^{-/-}$ or even APP23.$p38\gamma^{+/+}$ (Fig. 2K).

p38$\gamma$ levels were determined in extracts from brains of humans without Alzheimer's disease (Braak 0) and from humans with different neuropatholocial disease stages ranging from Braak I to Braak VI (Table 3). The results are shown in Fig. 23A and 23B. As can be seen from Fig. 23A and 23B, p38$\gamma$ levels were markedly reduced in humans as AD advances.

In summary, p38$\gamma$ modulates excitotoxicity, neuronal circuit synchronicity, premature mortality and memory deficits in APP23 mice, without changes in A$\beta$. In addition, p38$\gamma$ levels are reduced in APP23 mice and humans suffering from AD.

To determine if levels of tau affect the excitotoxicity-limiting effects of p38$\gamma$ in vivo, we crossed non-mutant human tau-expressing Alz17 mice (22) with $p38\gamma^{-/-}$ mice, to challenge these mice with PTZ. The results are shown in Fig. 3A-C. As can be seen from Fig. 3A-C, while tau expression did not affect seizure thresholds in Alz17.$p38\gamma^{+/+}$ mice, Alz17.$p38\gamma^{-/-}$ mice presented with significantly enhanced seizure progression and severity compared to $p38\gamma^{-/-}$ mice (Fig. 3A-C). Conversely, crossing $p38\gamma^{-/-}$ with tau-deficient $tau^{-/-}$ mice, revealed similar protection from PTZ-induced seizures in $tau^{-/-}.p38\gamma^{-/-}$ and $tau^{-/-}.p38\gamma^{+/+}$ mice (Fig. 3D-F) .

To determine whether the A$\beta$ toxicity-limiting effects of p38$\gamma$ were tau-dependent, APP23.p38$\gamma^{-/-}$ mice were crossed with $tau^{-/-}$ mice, and the resulting crosses assessed for survival, memory deficit and neuronal network disfunction. The results are shown in Fig. 3G-3I, 24, 25, 26A, 26B, 27, 28A and B, and 29). The exacerbating effects of p38$\gamma$ loss on reduced survival, memory deficits, and neuronal network dysfunction of APP23 mice were virtually abolished in APP23.p38$\gamma^{-/-}$.tau$^{-/-}$ mice. These data also show that, compared with APP23 mice, APP23.p38$\gamma^{-/-}$ animals had aggravated memory deficits that persisted with aging. In contrast, as noted above, increasing tau levels in p38$\gamma^{-/-}$ mice (brought about by crossing with non-mutant tau-expressing Alz17 mice) significantly enhanced PTZ-induced seizures in Alz17.p38$\gamma^{-/-}$ mice. Conversely, when compared to tau$^{-/-}$.p38$\gamma^{+/+}$ mice, tau$^{-/-}$.p38$\gamma^{-/-}$ animals showed similar protection from PTZ-induced seizures. Taken together, the effects of p38$\gamma$ on excitotoxicity and A$\beta$ toxicity are tau-dependent.

Tau resides in a post-synaptic signaling complex with Fyn and PSD-95 that mediates A$\beta$-induced excitotoxicity (8). Interaction of tau, Fyn and PSD95 in Alz17.$p38\gamma^{-/-}$ brains was enhanced compared to Alz17.$p38\gamma^{+/+}$ mice (Fig. 4A, B), consistent with their increased sensitivity to PTZ-induced seizures. Conversely, no PSD-95/tau/Fyn complexes could be isolated from $tau^{-/-}$ and $tau^{-/-}$ $p38\gamma^{-/-}$ brains. Strikingly, increased p38$\gamma$ levels compromised, and expression of a constitutive active variant of p38$\gamma$ (p38$\gamma^{CA}$) completely disrupted, PSD-95/tau/Fyn interaction in cells (Fig. 4C and D). Pan-p38 inhibition stopped p38$\gamma$ and p38$\gamma^{CA}$-induced disruption of PSD-95/tau/Fyn complexes, furthermore indicating that p38$\gamma$ activity is required (Fig. 4E and F). PSD-95 co-purified more tau and Fyn from $p38\gamma^{-/-}$ than $p38\gamma^{+/+}$ brains, suggesting increased PSD-95/tau/Fyn complex formation in the absence of p38$\gamma$ (Fig. 4G and H). PTZ transiently increased PSD-95/tau/Fyn complex formation in $p38\gamma^{+/+}$ animals, and even further in $p38\gamma^{-/-}$ mice. Similarly, PSD-95/tau/Fyn complex formation was markedly increased in APP23.$p38\gamma^{-/-}$ compared to APP23.$p38\gamma^{+/+}$ and $p38\gamma^{-/-}$ brains (Fig. 4I and J). Consistent with increased PSD-95/tau/Fyn complex formation, Fyn-mediated phosphorylation of NR2B at Y1472, that facilitates interaction of PSD-95 and NR2B (23, 24), was increased in $p38\gamma^{-/-}$ brains. Similarly, p38$\gamma$ and p38$\gamma^{CA}$ expression reduced Y1472-phosphorylation of NR2B.

Importantly, neither p38$\alpha$CA, p38$\beta$CA nor p38$\delta$CA reduced NR2B phosphorylation, indicating that regulation of PSD-95/tau/Fyn complexes is a non-redundant function of p38$\gamma$. Interestingly, both p38$\gamma$ and p38$\gamma^{CA}$ interacted with PSD-95 (Fig. 4C), which was abolished by deleting the C-terminal PDZ interaction motif from p38$\gamma$ and p38$\gamma^{CA}$ (Fig. 10A). Both p38$\gamma$ and p38$\gamma^{CA}$ also interacted with tau (Fig. 10B). Since, p38$\gamma$ and more so p38$\gamma^{CA}$ disrupted PSD-95/tau interaction in the absence of Fyn overexpression (Fig. 10C), but neither disrupted tau/Fyn interaction (Fig. 10D), p38$\gamma$ appears to regulate PSD-95/tau/Fyn complexes at the level of PSD-95/tau interaction.

While p38$\gamma$ phosphorylates tau at multiple epitopes during long-term in vitro kinase assays, possibly contributing to tau hyperphosphorylation (25), the temporal profile of p38$\gamma$-induced tau phosphorylation in acute signaling, including excitotoxicity, remained unknown. Using recombinant tau for short-term in vitro kinase reactions, we tested phosphorylation of a range of SP and TP sites, using available phosphorylation site-specific antibodies (Fig. 11A). Tau was phosphorylated strongly at serine (S) 199 and threonine (T) 205, and less at S396 and S404, but not at other sites tested (Fig. 11B). Site-specificity was confirmed by individually mutating S199, T205, S396 and S404 to alanine, which abolished p38γ-induced tau phosphorylation tau at the mutated sites *in vitro* (Fig. 11C). Mass spectrometric analysis of tau in kinase reactions confirmed these 4 sites, and an additional fourteen low abundant sites. Co-expression of p38γ or p38γ$^{CA}$ and tau revealed that p38γ predominantly phosphorylated tau at T205 and to a lesser degree at S199, but barely at S396 and S404 in cells (Fig. 5A). Similarly, T205 (and less so S199 and S396) were phosphorylated in p38γ$^{CA}$ transgenic mice. Phosphorylated T205 (pT205) increased after PTZ treatment of p38γ$^{+/+}$ animals but was virtually abolished in p38γ$^{-/-}$ mice, whereas pS199, pS396 and pS404 were induced in both p38γ$^{+/+}$ and p38γ$^{-/-}$ mice. Similarly, pT205 was markedly reduced in APP23.p38γ$^{-/-}$ animals compared with APP23.p38γ$^{+/+}$ mice. Consistently, phosphorylation of T205 in primary neurons was markedly reduced by pan-p38 inhibition, while S199 phosphorylation remained unaffected. Taken together, these data indicate that T205 is a primary site in tau phosphorylation by p38γ.

To determine the functional relevance of tau phosphorylation by p38γ at S199 and T205, we generated phosphorylation-mimicking (S199D and T205E) and - preventing (S199A and T205A) tau variants. We also prepared phosphorylation mimicking mutants of all other sites identified by mass spectrometry and assessed all mutants for their ability to co-purify with PSD-95, tau and Fyn. The results are shown in Fig. 5B, 5C and Fig. 31. PSD-95 co-purified with Fyn and all mutants except T205E. In this regard, T205E coprecipitated significantly less with PSD-95 as compared with PSD-95 as compared with non-mutant and T205A tau, while all other phosphorylation mimicking mutants of all other identified sites had no effect on PSD-95/tau/Fyn interaction. Microscale thermophoresis and glutathione S-transferase-pulldown in vitro and fluorescence lifetime imaging microscopy (FLIM)-fluorescence resonance energy transfer (FRET) analysis in live cells confirmed the markedly compromised interaction of T205E tau with PSD-95. The T205E mutation did not hinder tau/Fyn interaction. These data suggests that phosphorylation of tau at T205 is sufficient to disrupt interaction with PSD-95. T205E and T205A mutations did not compromise tau/Fyn interaction. Importantly, p38γ$^{CA}$ disrupted PSD-95/tau/Fyn complexes in the presence of non-mutant tau, but had no effects when T205A tau was co-expressed (Fig. 5D and E). In contrast, phospho-mimicking and -preventing S396 or S404 variants of tau had no effect on PSD-95/tau/Fyn interaction (Fig. 5C). Taken together, this suggests that p38γ regulates PSD-95/tau/Fyn complexes via phosphorylation of tau at T205.

Disruption of NR/PSD-95/tau/Fyn complexes prevented exitotoxicity and Aβ-induced toxicity in primary neurons and APP23 mice (8). Hence, phosphorylation of tau at T205 should mitigate or reduce Aβ-induced neurotoxicity. To test this, we used AAV-mediated gene transfer to express wild-type, T205A or T205E tau at similar levels in primary neurons (fig. 12). Challenge with Aβ induced cell death in wild-type and T205A, but virtually not in T205E human tau-expressing hippocampal neurons, as indicated by increased LDH release (Fig. 5F) or EthD1 uptake (fig. 12A). H$_2$O$_2$-treatment exerted the same level of cytotoxicity in neurons irrespectively of the tau variant expressed. To test whether increasing levels or activity of p38γ in neurons similarly confer protection from Aβ toxicity, we expressed p38γ, p38γ$^{CA}$ or a GFP control in primary neurons (Fig. 5G and Fig. 13). Both, expressed p38γ and p38γ$^{CA}$ enriched in dendritic spines, similar to endogenous p38γ. Neurons expressing p38γ and more so p38γ$^{CA}$ were significantly more resistant to Aβ-induced cell death compared to controls (Fig. 5H). Neither expression of p38γ nor p38γCA limited H$_2$O$_2$-induced cell death. In summary, expression of site-specific phosphorylation-mimicking T205E tau or increasing p38γ activity mitigated the toxic effects of Aβ in hippocampal neurons. Remaining Aβ toxicity in the presence of T205E tau or p38γ$^{CA}$ was possibly due to endogenous tau, or alternative pathways (9) .

To determine if increased neuronal p38γ levels and/or activity limits excitotoxicity *in vivo,* we used AAV-mediated gene transfer to express p38γ, p38γ$^{CA}$ or a GFP control in forebrains of newborn wild-type mice (fig. 14) and challenged them with PTZ at 2 months of age. Expression of p38γ *in vivo* moderately, but significantly decreased progression of PTZ-induced seizures in 2 month-old mice, with a trend towards reduction of mean seizure severity, compared to GFP expressing mice (Fig. 5I, J and fig. 15). p38γ$^{CA}$ expression profoundly increased the latency to develop severe seizures in response to PTZ administration, and significantly decreased the mean seizure severity as compared with control mice (Fig. 5I, J and fig. 15). Expression levels of p38γ and p38γ$^{CA}$ varied between mice as expected from AAV-mediated gene expression, with levels of p38γ being on average higher than those of p38γ$^{CA}$ (Fig. 14B). Interestingly, levels of both p38γ and p38γ$^{CA}$ and seizure latency slopes showed positive linear correlation (p38γ: R2 = 0.483, P = 0.0832, s = 65.23 ± 30.20; p38γCA: R2 = 0.707, P = 0.0023, s = 215.1 ± 48.96), with a significantly pronounced level-dependent protective effect of p38γ$^{CA}$ over p38γ expression (F = 6.8407, P = 0.0214) (Fig. 5K). Thus, levels of active p38γ kinase *in vivo* determine susceptibility to excitotoxic signals.

Memory deficits in APP23.AAV$^{p38γCA}$ were significantly less severe compared to those of APP23.AAV$^{GFP}$ mice, as assessed in the Morris-water maze paradigm (Figs. 16-18).

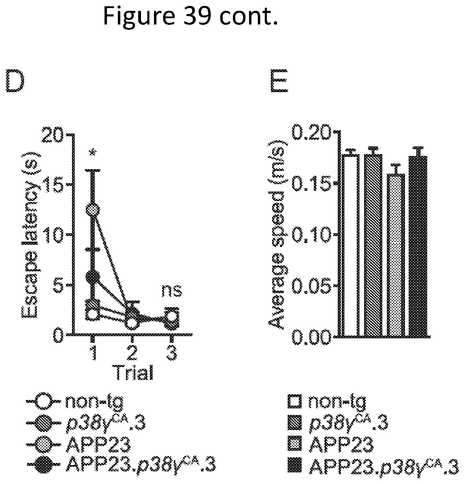

APP23.AAV$^{p38γCA}$ mice showed memory performance similar to wild-type memory performance (AAV$^{GFP}$, AAVP$^{p38γCA}$). Adeno-associated virus (AAV)- mediated expression of WT and T205A, but not T205E tau or green fluorescent protein (GFP), in the forebrains of tau$^{-/-}$ mice enhanced PTZ-induced seizures (Fig. 19). In contrast, expression of p38g$^{CA}$ in WT mice using AAV or in Thy1.2-p38γ$^{CA}$ transgenic mice decreased PTZ-induced seizures. AAV-mediated p38γ$^{CA}$ expression in APP23 mice rescued memory deficits and network aberrations (Fig. 34-38); the same was true for crossing APP23 with Thy.1.2-p38γ$^{CA}$ transgenic mice (Figs. 39-41). In summary, the levels of active p38γ kinase and tau phosphorylation at T205 determined susceptibility to excitotoxicity and Aβ toxicity.

Tau is a key mediator of deficits in APP transgenic mice (7, 8), and tau has been suggested to transmit detrimental signals of Aβ in neurons by becoming aberrantly phosphorylated (4, 27). Here, we show that tau is part of an intrinsic molecular pathway involving phosphorylation at T205 mediated by p38γ to inhibit excito- and Aβ toxicity. While we formally cannot exclude further non-tested sites being phosphorylated by p38γ, our data with T205A/E tau suggest that phosphorylation at T205 is key to modulating post-synaptic PSD-95/tau/Fyn complexes. Tau is required for the toxicity-limiting effects of p38γ, as *p38y* depletion failed to exacerbate seizures in *tau$^{-/-}$.p38γ$^{-/-}$* mice. Although other kinases might target T205 on tau in disease or physiologically (28-30), the very distinct localization of PSD-95, tau and p38γ in a complex at the post-synapse indicates a specific and spatially compartmentalized role of p38γ downstream of synaptic NR activation.

While different roles have been characterized for other p38 kinases, the function of p38γ remained understudied. Here, our study revealed an unprecedented function of p38γ in the brain, by showing its involvement in tau-mediated Aβ toxicity, memory deficits and survival in AD mice. Its distinct spatial expression in post-synapses and unique sequence features, when compared to neuronally expressed p38α/β, likely contribute to this non-redundant function of p38γ in neurons. p38α/β have been described as downstream mediators of excito- (*11*) and Aβ toxicity (12, 13). Therefore and importantly, the p38γ function in excito- and Aβ toxicity we describe here is distinct from and opposite to p38a/β.

In summary, our work suggests that phosphorylation of tau at T205 is part of an Aβ toxicity-inhibiting response. This is contrary to the current view that tau phosphorylation downstream of Aβ toxicity is a purely pathological response (27). However, it is in line with the idea that tau is involved in normal physiologic signaling events in neurons likely involving NR signal transduction (9). Finally, we have identified p38γ as an unprecedented Aβ-toxicity limiting signaling factor, which modulates tau-dependent excitotoxicity by site-specific phosphorylation of tau and controlling post-synaptic PSD-95/tau/Fyn complexes. This provides new insight into post-synaptic processes involved in early AD pathogenesis and may contribute to future drug development.

References

1. C Ballatore, VM Lee, JQ Trojanowski, Nature reviews. Neuroscience 8, 663-72 (2007).
2. C Haass, DJ Selkoe, Nature reviews. Molecular cell biology 8, 101-12 (2007).
3. K Iqbal, F Liu, CX Gong, C Alonso Adel, I Grundke-Iqbal, Acta Neuropathol 118, 53-69 (2009).
4. EM Mandelkow, E Mandelkow, Cold Spring Harb Perspect Med 2, a006247 (2012).
5. ES Musiek, DM Holtzman, Nat Neurosci 18, 800-6 (2015).
6. M Rapoport, HN Dawson, LI Binder, MP Vitek, A Ferreira, Proc Natl Acad Sci USA 99, 6364-9 (2002).
7. ED Roberson et al., Science 316, 750-4 (2007).
8. LM Ittner et al., Cell 142, 387-97 (2010).
9. L Mucke, DJ Selkoe, Cold Spring Harb Perspect Med 2, a006338 (2012).
10. JJ Palop, L Mucke, Nat Neurosci 13, 812-8 (2010).
11. GE Hardingham, H Bading, Nature reviews. Neuroscience 11, 682-96 (2010).
12. Q Wang, DM Walsh, MJ Rowan, DJ Selkoe, R Anwyl, J Neurosci 24, 3370-8 (2004).
13. S Li et al., J Neurosci 31, 6627-38 (2011).
14. AA Ittner, A Gladbach, J Bertz, LS Suh, LM Ittner, Acta Neuropathol Commun 2, 149 (2014).
15. MA Fabian et al., Nat Biotechnol 23, 329-36 (2005).
16. MB Menon, S Dhamija, A Kotlyarov, M Gaestel, Autophagy, 0 (2015).
17. C Sturchler-Pierrat et al., Proc Natl Acad Sci U S A 94, 13287-92 (1997).
18. G Buzsaki, EI Moser, Nat Neurosci 16, 130-8 (2013).
19. R Goutagny, J Jackson, S Williams, Nat Neurosci 12, 1491-3 (2009).
20. RT Canolty et al., Science 313, 1626-8 (2006).
21. AB Tort, RW Komorowski, JR Manns, NJ Kopell, H Eichenbaum, Proc Natl Acad Sci USA 106, 20942-7 (2009).
22. A Probst et al., Acta Neuropathol 99, 469-81 (2000).
23. Y Rong, X Lu, A Bernard, M Khrestchatisky, M Baudry, J Neurochem 79, 382-90 (2001).
24. M Aarts et al., 1 Science 298, 846-50 (2002).
25. M Goedert et al., FEES Lett 409, 57-62 (1997).
26. S Mondragon-Rodriguez et al., J Biol Chem 287, 32040-53 (2012).
27. LM Ittner, J Gotz, Nature reviews. Neuroscience 12, 65-72 (2011).
28. JZ Wang, Q Wu, A Smith, I Grundke-Iqbal, K Iqbal, FEBS Lett 436, 28-34 (1998).
29. V Buee-Scherrer, M Goedert, FEBS Lett 515, 151-4 (2002).
30. A Cavallini et al., J Biol Chem 288, 23331-47 (2013).

**EP 3 423 080 B1**

**Claims**

1. An agent which elevates p38γ activity, or the activity of a variant of p38γ, in neurons of a subject, for use in treating or preventing a neurological condition mediated by a tau-dependent signalling complex in neurons of a subject,

   wherein the agent elevates p38γ activity or the activity of a variant of p38γ is an agent that:

   (a) elevates the amount of p38γ in the neuron; and/or
   (b) elevates the amount of a variant of p38γ in the neuron; and/or
   (c) elevates the amount of p38γ activation in the neuron; and/or
   (d) elevates the amount of activation of the variant of p38γ in the neuron,

   wherein p38γ activity or the activity of a variant of p38γ causes disruption of the tau-dependent signalling complex and is phosphorylation of tau at T205,
   wherein a variant of p38γ is a protein that phosphorylates tau at T205 and comprises a PDZ interaction motif comprising the amino acid sequence EPTL (SEQ ID NO: 8) or ETAL (SEQ ID NO: 32),
   and wherein the agent comprises a nucleic acid sequence, a nucleic acid analogue, a protein, or a peptide.

2. The agent for use of claim 1, wherein the agent comprises p38γ or a variant thereof, or a nucleic acid that is capable of expressing p38γ or a variant thereof in neurons of the subject.

3. The agent for use of claim 1 or 2, wherein the variant of p38γ comprises an amino acid sequence that is at least 85%, 90%, 95 or 99% identical to the amino acid sequence of p38γ (SEQ ID NO: 2).

4. The agent for use of any one of claims 1 to 3, wherein the variant of p38γ is a constitutively active variant of p38γ (p38γ<sup>CA</sup>), preferably comprising an amino acid substitution of aspartic acid to alanine at position 179 of p38γ, such as the amino acid sequence represented by SEQ ID NO: 3.

5. The agent for use of any one of claims 1 to 4, wherein the tau-dependent signalling complex comprises PSD-95 and tau, preferably PSD-95, tau and FYN.

6. The agent for use of any one of claims 1 to 5, wherein the neurological condition is a condition caused by neuronal damage from overactivation of the tau-dependent signalling complex, preferably selected from the group consisting of Alzheimer's disease, frontotemperal dementia, amyotrophic lateral sclerosis, Huntington's disease, Parkinsons's disease, neural damage from stroke, and epilepsy.

7. A vector suitable for treating or preventing a neurological condition mediated by a tau-dependent signalling complex in neurons of a subject, comprising a nucleic acid sequence encoding p38γ or a variant thereof, wherein the nucleic acid sequence is operably linked to a neural promoter for expressing the p38γ or a variant thereof as defined in claim 1 in neurons of the subject.

8. A viral vector suitable for treating or preventing a neurological condition mediated by a tau-dependent signalling complex in neurons of a subject, comprising a nucleic acid sequence encoding p38γ or a variant thereof, wherein the nucleic acid sequence is operably linked to a regulatory sequence for expressing the p38γ or a variant thereof as defined in claim 1 in neurons of the subject.

9. The vector of claim 8, wherein the viral vector is an adeno-associated viral (AAV) vector.

10. The vector of any one of claims 7 to 9, wherein the variant of p38γ comprises an amino acid sequence which is at least 95 or 99% identical to the amino acid sequence of p38γ (SEQ ID NO: 2).

11. The vector of any one of claims 7 to 10, wherein the variant of p38γ is a constitutively active mutant of p38γ, preferably comprising an amino acid substitution of aspartic acid to alanine at position 179 of p38γ, such as the amino acid sequence represented by SEQ ID NO: 3.

12. A vector for use as a medicament, wherein the vector comprises a nucleic acid sequence encoding p38γ or a variant thereof,
    wherein the nucleic acid sequence is operably linked to a regulatory sequence for expressing the p38γ or a variant thereof as defined in claim 1 in neurons of the subject.

13. The vector for use according to claim 12, wherein the medicament is for treating or preventing a neurological condition mediated by a tau-dependent signalling complex in neurons of a subject.

14. A composition comprising the vector of any one of claims 7 to 11.

15. The composition according to claim 14, for use as a medicament.

16. The composition for use according to claim 15, wherein the medicament is for treating or preventing a neurological condition mediated by a tau-dependent signalling complex in neurons of a subject.

**Patentansprüche**

1. Ein Wirkstoff, welcher eine Aktivität von p38$\gamma$, oder eine Aktivität einer Variante von p38$\gamma$, in Neuronen eines Subjekts erhöht, zur Verwendung bei der Behandlung oder Vorbeugung eines neurologischen Zustands, der durch einen Tau-abhängigen Signalkomplex in Neuronen eines Subjekts vermittelt wird, wobei der Wirkstoff, welcher eine Aktivität von p38$\gamma$, oder eine Aktivität einer Variante von p38$\gamma$, erhöht, ein Wirkstoff ist, welcher:

(a) die Menge an p38$\gamma$ in dem Neuron erhöht; und/oder
(b) die Menge einer Variante von p38$\gamma$ in dem Neuron erhöht; und/oder
(c) das Maß an Aktivierung von p38$\gamma$ in dem Neuron erhöht; und/oder
(d) das Maß an Aktivierung einer Variante von p38$\gamma$ in dem Neuron erhöht,

wobei eine Aktivität von p38$\gamma$ oder eine Aktivität einer Variante von p38$\gamma$ eine Unterbrechung des Tau-abhängigen Signalkomplexes verursacht und eine Phosphorylierung von Tau bei T205 ist, wobei eine Variante von p38$\gamma$ ein Protein ist, welches Tau bei T205 phosphoryliert und ein PDZ-Interaktionsmotiv enthält, welches die Aminosäuresequenz EPTL (SEQ ID NO: 8) oder ETAL (SEQ ID NO: 32) enthält, und wobei der Wirkstoff eine Nukleinsäuresequenz, ein Nukleinsäureanalogon, ein Protein oder ein Peptid enthält.

2. Der Wirkstoff zur Verwendung nach Anspruch 1, wobei der Wirkstoff p38$\gamma$ oder eine Variante davon, oder eine Nukleinsäure, welche fähig ist, p38$\gamma$ oder eine Variante davon in Neuronen des Subjekts zu exprimieren, enthält.

3. Der Wirkstoff zur Verwendung nach Anspruch 1 oder 2, wobei die Variante von p38$\gamma$ eine Aminosäuresequenz enthält, welche zu mindestens 85%, 90%, 95 oder 99% identisch ist mit der Aminosäuresequenz von p38$\gamma$ (SEQ ID NO: 2).

4. Der Wirkstoff zur Verwendung nach einem der Ansprüche 1 bis 3, wobei die Variante von p38$\gamma$ eine konstitutiv aktive Variante von p38$\gamma$ (p38$\gamma^{CA}$) ist, die vorzugsweise eine Aminosäuren-Substitution von Asparaginsäure durch Alanin an Position 179 von p38$\gamma$ aufweist, so wie die Aminosäuresequenz, welche durch SEQ ID NO: 3 dargestellt wird.

5. Der Wirkstoff zur Verwendung nach einem der Ansprüche 1 bis 4, wobei der Tauabhängige Signalkomplex PSD-95 und Tau, vorzugsweise PSD-95, Tau und FYN, beinhaltet.

6. Der Wirkstoff zur Verwendung nach einem der Ansprüche 1 bis 5, wobei der neurologische Zustand ein Zustand ist, welcher durch eine neuronale Schädigung aufgrund einer Überaktivierung des Tau-abhängigen Signalkomplexes verursacht wird, vorzugsweise ausgewählt aus der Gruppe bestehend aus Alzheimer-Krankheit, Frontotemporaler Demenz, Amyotropher Lateralsklerose, Huntington-Krankheit, Parkinson-Krankheit, neuronaler Schädigung durch Schlaganfall, und Epilepsie.

7. Ein Vektor, geeignet zur Behandlung oder Vorbeugung eines neurologischen Zustands, der durch einen Tau-abhängigen Signalkomplex in Neuronen eines Subjekts vermittelt wird, wobei der Vektor eine Nukleinsäuresequenz beinhaltet, welche p38$\gamma$ oder eine Variante davon kodiert, wobei die Nukleinsäuresequenz operabel verbunden ist mit einem neuralen Promoter zur Expression des p38$\gamma$, oder einer Variante davon wie in Anspruch 1 definiert, in Neuronen eines Subjekts.

**8.** Ein viraler Vektor, geeignet zur Behandlung oder Vorbeugung eines neurologischen Zustands, der durch einen Tau-abhängigen Signalkomplex in Neuronen eines Subjekts vermittelt wird, wobei der virale Vektor eine Nukleinsäuresequenz beinhaltet, welche p38γ oder eine Variante davon kodiert, wobei die Nukleinsäuresequenz operabel verbunden ist mit einer regulatorischen Sequenz zur Expression des p38γ, oder einer Variante davon wie in Anspruch 1 definiert, in Neuronen eines Subjekts.

**9.** Der Vektor nach Anspruch 8, wobei der virale Vektor ein Adeno-assoziierter viraler (AAV) Vektor ist.

**10.** Der Vektor nach einem der Ansprüche 7 bis 9, wobei die Variante von p38γ eine Aminosäuresequenz enthält, welche zu mindestens 95 oder 99% identisch ist mit der Aminosäuresequenz von p38γ (SEQ ID NO: 2).

**11.** Der Vektor nach einem der Ansprüche 7 bis 10, wobei die Variante von p38γ eine konstitutiv aktive Mutante von p38γ ist, die vorzugsweise eine Aminosäuren-Substitution von Asparaginsäure durch Alanin an Position 179 von p38γ aufweist, so wie die Aminosäuresequenz, welche durch SEQ ID NO: 3 dargestellt wird.

**12.** Ein Vektor zur Verwendung als Medikament, wobei der Vektor eine Nukleinsäuresequenz beinhaltet, welche p38γ oder eine Variante davon kodiert, wobei die Nukleinsäuresequenz operabel verbunden ist mit einer regulatorischen Sequenz zur Expression des p38γ, oder einer Variante davon wie in Anspruch 1 definiert, in Neuronen eines Subjekts.

**13.** Der Vektor zur Verwendung nach Anspruch 12, wobei das Medikament zur Behandlung oder Vorbeugung eines neurologischen Zustands dient, der durch einen Tau-abhängigen Signalkomplex in Neuronen eines Subjekts vermittelt wird.

**14.** Eine Zusammensetzung, welche den Vektor nach einem der Ansprüche 7 bis 11 beinhaltet.

**15.** Die Zusammensetzung nach Anspruch 14 zur Verwendung als Medikament.

**16.** Die Zusammensetzung zur Verwendung nach Anspruch 15, wobei das Medikament zur Behandlung oder Vorbeugung eines neurologischen Zustands vermittelt durch einen Tau-abhängigen Signalkomplex in Neuronen eines Subjekts dient.

**Revendications**

**1.** Agent qui élève l'activité de p38γ, ou l'activité d'un variant de p38γ, dans les neurones d'un sujet, pour l'utilisation dans le traitement ou la prévention d'une affection neurologique médiée par un complexe de signalisation dépendant de tau dans les neurones d'un sujet,

où l'agent qui élève l'activité de p38γ ou l'activité d'un variant de p38γ est un agent qui :

(a) augmente la quantité de p38γ dans le neurone ; et/ou
(b) augmente la quantité d'un variant de p38γ dans le neurone ; et/ou
(c) augmente la quantité d'activation de p38γ dans le neurone ; et/ou
(d) augmente la quantité d'activation du variant de p38γ dans le neurone,

où l'activité de p38γ ou l'activité d'un variant de p38γ causent une perturbation du complexe de signalisation dépendant de tau et est une phosphorylation de tau à T205,
où un variant de p38γ est une protéine qui phosphoryle tau à T205 et comprend un motif d'interaction PDZ comprenant la séquence d'acides aminés EPTL (SEQ ID NO: 8) ou ETAL (SEQ ID NO: 32),
et où l'agent comprend une séquence d'acides nucléiques, un analogue d'acides nucléiques, une protéine, ou un peptide.

**2.** Agent pour l'utilisation selon la revendication 1, où l'agent comprend p38γ ou un variant de celle-ci, ou un acide nucléique qui est capable d'exprimer p38γ ou un variant de celle-ci dans les neurones du sujet.

**3.** Agent pour l'utilisation selon la revendication 1 ou 2, où le variant de p38γ comprend une séquence d'acides aminés qui est au moins 85%, 90%, 95 ou 99 % identique à la séquence d'acides aminés de p38γ (SEQ ID NO: 2).

**4.** Agent pour l'utilisation selon l'une quelconque des revendications 1 à 3, où le variant de p38$\gamma$ est un variant constitutivement actif de p38$\gamma$ (p38$\gamma^{CA}$), comprenant de préférence une substitution d'acide aminé de l'acide aspartique à l'alanine en position 179 de p38$\gamma$, telle que la séquence d'acides aminés représentée par SEQ ID NO: 3.

**5.** Agent pour l'utilisation selon l'une quelconque des revendications 1 à 4, où le complexe de signalisation dépendant de tau comprend PSD-95 et tau, de préférence PSD-95, tau et FYN.

**6.** Agent pour l'utilisation selon l'une quelconque des revendications 1 à 5, où l'affection neurologique est une affection causée par une lésion neuronale due à une suractivation du complexe de signalisation dépendant de tau, de préférence sélectionné dans le groupe consistant en maladie d'Alzheimer, démence frontotempérale, sclérose latérale amyotrophique, maladie d'Huntington, maladie de Parkinson, lésions neuronales causées par un accident vasculaire cérébral et épilepsie.

**7.** Vecteur adapté pour traiter ou prévenir une affection neurologique médiée par un complexe de signalisation dépendant de tau dans les neurones d'un sujet, comprenant une séquence d'acides nucléiques codant p38$\gamma$ ou un variant de celle-ci, où la séquence d'acides nucléiques est liée de manière opérationnelle à un promoteur neuronal pour exprimer la p38$\gamma$ ou un variant de celle-ci comme défini dans la revendication 1 dans les neurones du sujet.

**8.** Vecteur viral adapté pour traiter ou prévenir une affection neurologique médiée par un complexe de signalisation dépendant de tau dans les neurones d'un sujet, comprenant une séquence d'acides nucléiques codant p38$\gamma$ ou un variant de celle-ci, où la séquence d'acides nucléiques est liée de manière opérationnelle à une séquence régulatrice pour exprimer la p38$\gamma$ ou un variant de celle-ci comme défini dans la revendication 1 dans les neurones du sujet.

**9.** Vecteur selon la revendication 8, où le vecteur viral est un vecteur viral adéno-associé (AAV).

**10.** Vecteur selon l'une quelconque des revendications 7 à 9, où le variant de p38$\gamma$ comprend une séquence d'acides aminés qui est au moins 95 ou 99 % identique à la séquence d'acides aminés de p38$\gamma$ (SEQ ID NO: 2).

**11.** Vecteur selon l'une quelconque des revendications 7 à 10, où le variant de p38$\gamma$ est un mutant constitutivement actif de p38$\gamma$, comprenant de préférence une substitution d'acide aminé de l'acide aspartique à l'alanine en position 179 de p38$\gamma$, telle que la séquence d'acides aminés représentée par SEQ ID NO: 3.

**12.** Vecteur pour l'utilisation comme médicament, où le vecteur comprend une séquence d'acides nucléiques codant pour p38$\gamma$ ou un variant de celle-ci,
où la séquence d'acides nucléiques est liée de manière opérationnelle à une séquence régulatrice pour exprimer la p38$\gamma$ ou un variant de celle-ci tel que défini dans la revendication 1 dans les neurones du sujet.

**13.** Vecteur pour l'utilisation selon la revendication 12, où le médicament est pour traiter ou prévenir une affection neurologique médiée par un complexe de signalisation dépendant de tau dans les neurones d'un sujet.

**14.** Composition comprenant le vecteur selon l'une quelconque des revendications 7 à 11.

**15.** Composition selon la revendication 14, pour l'utilisation comme un médicament.

**16.** Composition pour l'utilisation selon la revendication 15, où le médicament est pour traiter ou prévenir une affection neurologique médiée par un complexe de signalisation dépendant de tau dans les neurones d'un sujet.

# Figure 1

Figure 2A

Figure 2B

FIGURE 2C

## Figure 2D

## Figure 2E

Figure 2F

Figure 2G

# Figures 2H and 2I

## Figure 2J

Figure 2K

Figure 3

EP 3 423 080 B1

Figure 3 cont.

# Figure 3 cont.

EP 3 423 080 B1

# Figure 4

# Figure 4 cont.

# Figure 4 cont.

# Figure 4 cont.

# Figure 4 cont.

# Figure 5

# Figure 5 cont.

Figure 5 cont.

# Figure 5 cont.

# Figure 5 cont.

# Figure 5 cont.

# Figure 5 cont.

# Figure 5 cont.

# Figure 6

EP 3 423 080 B1

## Figure 7

EP 3 423 080 B1

Figure 8

EP 3 423 080 B1

Figure 9

Figure 9 cont.

EP 3 423 080 B1

# Figure 9 cont.

Figure 9 cont.

Figure 10

EP 3 423 080 B1

# Figure 11

# Figure 12

A

B

Figure 13

# Figure 14

A

B

Figure 15

Figure 16

# Figure 17

Figure 18

Figure 19

Figure 20

Figure 21

Figure 22

A

B

C

# Figure 23

# Figure 24

Figure 25

## Figure 26

Figure 27

# Figure 28

A

B

Figure 29

A

B

C

EP 3 423 080 B1

# Figure 30

Figure 31

EP 3 423 080 B1

# Figure 32

Figure 33

# Figure 34

EP 3 423 080 B1

# Figure 35

A

B

AAV GFP

5 mV

1 sec

AAV p38γ$^{CA}$

5 mV

1 sec

APP23.AAV GFP

5 mV

1 sec

APP23.AAV p38γ$^{CA}$

5 mV

1 sec

□ AAV GFP
▨ AAV p38γ$^{CA}$
▨ APP23.AAV GFP
■ APP23.AAV p38γ$^{CA}$

Figure 36

Figure 37

A

B

EP 3 423 080 B1

Figure 38

Figure 39

# Figure 39 cont.

D

E

# Figure 40

EP 3 423 080 B1

Figure 40 cont.

Figure 41

A

non-tg

p38γ^CA.3

APP23

APP23.p38γ^CA.3

Amplitude frequ. (Hz)

Phase frequency (Hz)

x10⁻⁴ 12 10 8 6 4 2

B

Normalized amplitude (gamma)

0.070
0.065
0.060
0.055
0.050
0.045

0    180    360
Phase

non-tg
p38γ^CA.3
APP23
APP23.p38γ^CA.3

C

Modulation index

0.003
0.002
0.001
0.000

ns    ns
ns    *

non-tg
p38γ^CA.3
APP23
APP23.p38γ^CA.3

# Figure 42

# Figure 43

## Human p38γ

### A

```
ATGAGCTCTCCGCCGCCCGCCCGCAGTGGCTTTTACCGCCAGGAGGTGACCAAGACGGCCTGGGAGGTGCGCGCCGTGTA
CCGGGACCTGCAGCCCGTGGGCTCGGGCGCCTACGGCGCGGTGTGCTCGGCCGTGGACGGCCGCACCGGCGCTAAGGTGG
CCATCAAGAAGCTGTATCGGCCCTTCCAGTCCGAGCTGTTCGCCAAGCGCGCCTACCGCGAGCTGCGCCTGCTCAAGCAC
ATGCGCCACGAGAACGTGATCGGGCTGCTGGACGTATTCACTCCTGATGAGACCCTGGATGACTTCACGGACTTTTACCT
GGTGATGCCGTTCATGGGCACCGACCTGGGCAAGCTCATGAAACATGAGAAGCTAGGCGAGGACCGGATCCAGTTCCTCG
TGTACCAGATGCTGAAGGGGCTGAGGTATATCCACGCTGCCGGCATCATCCACAGAGACCTGAAGCCCGGCAACCTGGCT
GTGAACGAAGACTGTGAGCTGAAGATCCTGGACTTCGGCCTGGCCAGGCAGGCAGACAGTGAGATGACTGGGTACGTGGT
GACCCGGTGGTACCGGGCTCCCGAGGTCATCTTGAATTGGATGCGCTACACGCAGACGGTGGACATCTGGTCTGTGGGCT
GCATCATGGCGGAGATGATCACAGGCAAGACGCTGTTCAAGGGCAGCGACCACCTGGACCAGCTGAAGGAGATCATGAAG
GTGACGGGGACGCCTCCGGCTGAGTTTGTGCAGCGGCTGCAGAGCGATGAGGCCAAGAACTACATGAAGGGCCTCCCCGA
ATTGGAGAAGAAGGATTTTGCCTCTATCCTGACCAATGCAAGCCCTCTGGCTGTGAACCTCCTGGAGAAGATGCTGGTGC
TGGACGCGGAGCAGCGGGTGACGGCAGGCGAGGCGCTGGCCCATCCCTACTTCGAGTCCCTGCACGACACGGAAGATGAG
CCCCAGGTCCAGAAGTATGATGACTCCTTTGACGACGTTGACCGCACACTGGATGAATGGAAGCGTGTTACTTACAAAGA
GGTGCTCAGCTTCAAGCCTCCCCGGCAGCTGGGGGCCAGGGTCTCCAAGGAGACGCCTCTGTGA
```

### B

```
MSSPPPARSGFYRQEVTKTAWEVRAVYRDLQPVGSGAYGAVCSAVDGRTGAKVAIKKLYR          60
PFQSELFAKRAYRELRLLKHMRHENVIGLLDVFTPDETLDDFTDFYLVMPFMGTDLGKLM         120
KHEKLGEDRIQFLVYQMLKGLRYIHAAGIIHRDLKPGNLAVNEDCELKILDFGLARQADS         180
EMTGYVVTRWYRAPEVILNWMRYTQTVDIWSVGCIMAEMITGKTLFKGSDHLDQLKEIMK         240
VTGTPPAEFVQRLQSDEAKNYMKGLPELEKKDFASILTNASPLAVNLLEKMLVLDAEQRV         300
TAGEALAHPYFESLHDTEDEPQVQKYDDSFDDVDRTLDEWKRVTYKEVLSFKPPRQLGAR         360
VSKETPL                                                             367
```

# Figure 44

p38γ<sup>CA</sup> (D179A)

```
MSSPPPARSGFYRQEVTKTAWEVRAVYRDLQPVGSGAYGAVCSAVDGRTGAKVAIKKLYR        60
PFQSELFAKRAYRELRLLKHMRHENVIGLLDVFTPDETLDDFTDFYLVMPFMGTDLGKLM       120
KHEKLGEDRIQFLVYQMLKGLRYIHAAGIIHRDLKPGNLAVNEDCELKILDFGLARQAAS       180
EMTGYVVTRWYRAPEVILNWMRYTQTVDIWSVGCIMAEMITGKTLFKGSDHLDQLKEIMK       240
VTGTPPAEFVQRLQSDEAKNYMKGLPELEKKDFASILTNASPLAVNLLEKMLVLDAEQRV       300
TAGEALAHPYFESLHDTEDEPQVQKYDDSFDDVDRTLDEWKRVTYKEVLSFKPPRQLGAR       360
VSKETPL                                                          367
```

# Figure 45

Human tau

```
  1 MAEPRQEFEV MEDHAGTYGL GDRKDQGGYT MHQDQEGDTD AGLKESPLQT PTEDGSEEPG
 61 SETSDAKSTP TAEDVTAPLV DEGAPGKQAA AQPHTEIPEG TTAEEAGIGD TPSLEDEAAG
121 HVTQARMVSK SKDGTGSDDK KAKGADGKTK IATPRGAAPP GQKGQANATR IPAKTPPAPK
181 TPPSSGEPPK SGDRSGYSSP GSPGTPGSRS RTPSLPTPPT REPKKVAVVR TPPKSPSSAK
241 SRLQTAPVPM PDLKNVKSKI GSTENLKHQP GGGKVQIINK KLDLSNVQSK CGSKDNIKHV
301 PGGGSVQIVY KPVDLSKVTS KCGSLGNIHH KPGGGQVEVK SEKLDFKDRV QSKIGSLDNI
361 THVPGGGNKK IETHKLTFRE NAKAKTDHGA EIVYKSPVVS GDTSPRHLSN VSSTGSIDMV
421 DSPQLATLAD EVSASLAKQG L
```

Human tau T205E

```
  1 MAEPRQEFEV MEDHAGTYGL GDRKDQGGYT MHQDQEGDTD AGLKESPLQT PTEDGSEEPG
 61 SETSDAKSTP TAEDVTAPLV DEGAPGKQAA AQPHTEIPEG TTAEEAGIGD TPSLEDEAAG
121 HVTQARMVSK SKDGTGSDDK KAKGADGKTK IATPRGAAPP GQKGQANATR IPAKTPPAPK
181 TPPSSGEPPK SGDRSGYSSP GSPGEPGSRS RTPSLPTPPT REPKKVAVVR TPPKSPSSAK
241 SRLQTAPVPM PDLKNVKSKI GSTENLKHQP GGGKVQIINK KLDLSNVQSK CGSKDNIKHV
301 PGGGSVQIVY KPVDLSKVTS KCGSLGNIHH KPGGGQVEVK SEKLDFKDRV QSKIGSLDNI
361 THVPGGGNKK IETHKLTFRE NAKAKTDHGA EIVYKSPVVS GDTSPRHLSN VSSTGSIDMV
421 DSPQLATLAD EVSASLAKQG L
```

# Figure 46

Figure 47

# Figure 48

pAAV-CAG-106-3xHA-p38gamma(MAPK12)-wt

tagctgcgcgctcgctcgctcactgaggccgcccgggcaaagcccgggcgtcgggcga
cctttggtcgcccggcctcagtgagcgagcgagcgcgcagagagggagtggccaactc
catcactaggggttccttgtagttaatgattaacccgccatgctacttatctacgtag
ccatgctctaggtaccgggcccccccctAgaggtAgacggtatcTTCCCATAGTAACGC
CAATAGGGACTTTCCATTGACGTCAATGGGTGGACTATTTACGGTAAACTGCCCACTT
GGCAGTACATCAAGTGTATCATATGCCAAGTACGCCCCCTATTGACGTCAATGACGGT
AAATGGCCCGCCTGGCATTATGCCCAGTACATGACCTTATGGGACTTTCCTACTTGGC
AGTACATCTACGTATTAGTCATCGCTATTACCATGGGTCGAGGTGAGCCCCACGTTCT
GCTTCACTCTCCCCATCTCCCCCCCCTCCCCACCCCCAATTTTGTATTTATTTATTTT
TTAATTATTTTGTGCAGCGATGGGGGCGGGGGGGGGGGGGGGCGCGCGCCAGGCGGGGC
GGGGCGGGGCGAGGGGCGGGGCGGGGCGAGGCGGAGAGGTGCGGCGGCAGCCAATCAG
AGCGGCGCGCTCCGAAAGTTTCCTTTTATGGCGAGGCGGCGGCGGCGGCGGCCCTATA
AAAAGCGAAGCGCGCGGCGGGCGGGAGTCGCTGCGTTGCCTTCGCCCCGTGCCCCGCT
CCGCGCCGCcTCCGCGCCCGCCGCCCCGGCTCTGACTGACCGCGTTACTCCCACAGGT
GAGCGGGCGGGACGGCCCTTCTCCTCCGGGCTGTAATTAGCGCTTGGTTTAATGACGG
CTTGTTTCTTTTCTGTGGCTGCGTGAAAGCCTTGAGGGGCTCCGGGAGGGCCCTTTGT
GCGGGGGGAGCGGCTCGGGGCTGTCCGCGGGGGGACGGCTGCCTTCGGGGGGGACGGG
GCAGGGCGGGGTTCGGCTTCTGGCGTGTGACCGGCGGCTCTAGAGCCTCTGCTAACCA
TGTTCATGCCTTCTTCTTTTTCCTACAGCTCCTGGGCAACGTGCTGGTTATTGTGCTG
TCTCATCATTTTGGCAAAGAATTGGATCCACTCGAGTGGAGCTCGCGACTAGTCGATT
CGAATTCGATagctTATGTACCCATACGATGTTCCAGATTACGCCATGTACCCATACG
ATGTTCCAGATTACGCCATGTACCCATACGATGTTCCAGATTACGCCATGGGGAGCTC
TCCGCCGCCCGCCCGCAGTGGCTTTTACCGCCAGGAGGTGACCAAGACGGCCTGGGAG
GTGCGCGCCGTGTACCGGGACCTGCAGCCCGTGGGCTCGGGCGCCTACGGCGCGGTGT
GCTCGGCCGTGGACGGCCGCACCGGCGCTAAGGTGGCCATCAAGAAGCTGTATCGGCC
CTTCCAGTCCGAGCTGTTCGCCAAGCGCGCCTACCGCGAGCTGCGCCTGCTCAAGCAC
ATGCGCCACGAGAACGTGATCGGGCTGCTGGACGTATTCACTCCTGATGAGACCCTGG
ATGACTTCACGGACTTTTACCTGGTGATGCCGTTCATGGGCACCGACCTGGGCAAGCT
CATGAAACATGAGAAGCTAGGCGAGGACCGGATCCAGTTCCTCGTGTACCAGATGCTG
AAGGGGCTGAGGTATATCCACGCTGCCGGCATCATCCACAGAGACCTGAAGCCCGGCA
ACCTGGCTGTGAACGAAGACTGTGAGCTGAAGATCCTGGACTTCGGCCTGGCCAGGCA
GGCAGACAGTGAGATGACTGGGTACGTGGTGACCCGGTGGTACCGGGCTCCCGAGGTC
ATCTTGAATTGGATGCGCTACACGCAGACGGTGGACATCTGGTCTGTGGGCTGCATCA
TGGCGGAGATGATCACAGGCAAGACGCTGTTCAAGGGCAGCGACCACCTGGACCAGCT
GAAGGAGATCATGAAGGTGACGGGGACGCCTCCGGCTGAGTTTGTGCAGCGGCTGCAG
AGCGATGAGGCCAAGAACTACATGAAGGGCCTCCCCGAATTGGAGAAGAAGGATTTTG
CCTCTATCCTGACCAATGCAAGCCCTCTGGCTGTGAACCTCCTGGAGAAGATGCTGGT
GCTGGACGCGGAGCAGCGGGTGACGGCAGGCGAGGCGCTGGCCCATCCCTACTTCGAG
TCCCTGCACGACACGGAAGATGAGCCCCAGGTCCAGAAGTATGATGACTCCTTTGACG
ACGTTGACCGCACACTGGATGAATGGAAGCGTGTTACTTACAAAGAGGTGCTCAGCTT
CAAGCCTCCCCGGCAGCTGGGGGCCAGGGTCTCCAAGGAGACGCCTCTGTGATctagA
TCAAGCTTATCgataatcaacctctggattacaaaatttgtgaaagattgactggtat
tcttaactatgttgctccttttacgctatgtggatacgctgctttaatgcctttgtat
catgctattgcttcccgtatggctttcattttctcctccttgtataaatcctggttgc

# Figure 48 cont.

```
tgtctctttatgaggagttgtggcccgttgtcaggcaacgtggcgtggtgtgcactgt
gtttgctgacgcaaccccactggttggggcattgccaccacctgtcagctcctttcc
gggactttcgctttcccccctccctattgccacggcggaactcatcgccgcctgccttg
cccgctgctggacaggggctcggctgttgggcactgacaattccgtggtgttgtcggg
gaaGCTGAcgtcctttccAtggctgctcgcctgtgttgccacctggattctgcgcggg
acgtccttctgctacgtcccttcggccctcaatccagcggaccttccttcccgcggcc
tgctgccggctctgcggcctcttccgcgtcttcgccttcgccctcagacgagtcggat
ctccctttgggccgcctccccgcatcgataccgtcgactcgctgatcagcctcgactg
tgccttctagttgccagccatctgttgtttgccctcccccgtgccttccttgaccct
ggaaggtgccactcccactgtcctttcctaataaaatgaggaaattgcatcgcattgt
ctgagtaggtgtcattctattctggggggtggggtggggcaggacagcaaggggggagg
attgggaagacaatagcaggcatgctggggatgcggtgggctctatggcttctgaggc
ggaaagaaccagctggggctcgactagagcatggctacgtagataagtagcatggcgg
gttaatcattaactacaaggaacccctagtgatggagttggccactccctctctgcgc
gctcgctcgctcactgaggccgggcgaccaaaggtcgcccgacgcccgggctttgccc
gggcggcctcagtgagcgagcgagcgcgcagagctttttgcaaaagcctaggcctcca
aaaaagcctcctcactacttctggaatagctcagaggccgaggcggcctcggcctctg
cataaataaaaaaaattagtcagccatggggcggagaatgggcggaactgggcggagt
taggggcgggatgggcggagttaggggcgggactatggttgctgactaattgagatgc
atgctttgcatacttctgcctgctggggagcctggggactttccacacctggttgc
tgactaattgagatgcatgctttgcatacttctgcctgctggggagcctggggacttt
ccacaccctaactgacacacattccacagctgcattaatgaatcggccaacgcgcggg
gagaggcggtttgcgtattgggcgctcttccgcttcctcgctcactgactcgctgcgc
tcggtcgttcggctgcggcgagcggtatcagctcactcaaaggcggtaatacggttat
ccacagaatcaggggataacgcaggaaagaacatgtgagcaaaaggccagcaaaaggc
caggaaccgtaaaaaggccgcgttgctggcgtttttccataggctccgcccccctgac
gagcatcacaaaaatcgacgctcaagtcagaggtggcgaaacccgacaggactataaa
gataccaggcgtttcccctggaagctccctcgtgcgctctcctgttccgaccctgcc
gcttaccggatacctgtccgcctttctcccttcgggaagcgtggcgctttctcatagc
tcacgctgtaggtatctcagttcggtgtaggtcgttcgctccaagctgggctgtgtgc
acgaaccccccgttcagcccgaccgctgcgccttatccggtaactatcgtcttgagtc
caacccggtaagacacgacttatcgccactggcagcagccactggtaacaggattagc
agagcgaggtatgtaggcggtgctacagagttcttgaagtggtggcctaactacggct
acactagaagaacagtatttggtatctgcgctctgctgaagccagttaccttcggaaa
aagagttggtagctcttgatccggcaaacaaaccaccgctggtagcggtggtttttttt
gtttgcaagcagcagattacgcgcagaaaaaaggatctcaagaagatcctttgatct
tttctacggggtctgacgctcagtggaacgaaaactcacgttaagggattttggtcat
gagattatcaaaaaggatcttcacctagatccttttaaattaaaaatgaagttttaaa
tcaatctaaagtatatatgagtaaacttggtctgacagttaccaatgcttaatcagtg
aggcacctatctcagcgatctgtctatttcgttcatccatagttgcctgactccccgt
cgtgtagataactacgatacgggagggcttaccatctggccccagtgctgcaatgata
ccgcgagacccacgctcaccggctccagatttatcagcaataaaccagccagccggaa
gggccgagcgcagaagtggtcctgcaactttatccgcctccatccagtctattaattg
ttgccgggaagctagagtaagtagttcgccagttaatagtttgcgcaacgttgttgcc
attgctacaggcatcgtggtgtcacgctcgtcgtttggtatggcttcattcagctccg
```

# Figure 48 cont.

```
gttcccaacgatcaaggcgagttacatgatcccccatgttgtgcaaaaaagcggttag
ctccttcggtcctccgatcgttgtcagaagtaagttggccgcagtgttatcactcatg
gttatggcagcactgcataattctcttactgtcatgccatccgtaagatgcttttctg
tgactggtgagtactcaaccaagtcattctgagaatagtgtatgcggcgaccgagttg
ctcttgcccggcgtcaatacgggataataccgcgccacatagcagaactttaaaagtg
ctcatcattggaaaacgttcttcggggcgaaaactctcaaggatcttaccgctgttga
gatccagttcgatgtaacccactcgtgcacccaactgatcttcagcatcttttacttt
caccagcgtttctgggtgagcaaaaacaggaaggcaaaatgccgcaaaaaagggaata
agggcgacacggaaatgttgaatactcatactcttccttttcaatattattgaagca
tttatcagggttattgtctcatgagcggatacatatttgaatgtatttagaaaaataa
acaaatagggggttccgcgcacatttccccgaaaagtgccacctgacgtctaagaaacc
attattatcatgacattaacctataaaaataggcgtatcacgaggcccctttcgtctcg
cgcgtttcggtgatgacggtgaaaacctctgacacatgcagctcccggagacggtcac
agcttgtctgtaagcggatgccgggagcagacaagcccgtcagggcgcgtcagcgggt
gttggcgggtgtcggggctggcttaactatgcggcatcagagcagattgtactgagag
tgcaccattcgacgctctcccttatgcgactcctgcattaggaagcagcccagtagta
ggttgaggccgttgagcaccgccgccgcaaggaatggtgcatgcaaggagatggcgcc
caacagtcccccggccacggggcctgccaccatacccacgccgaaacaagcgctcatg
agcccgaagtggcgagcccgatcttccccatcggtgatgtcggcgatataggcgccag
caaccgcacctgtggcgccggtgatgccggccacgatgcgtccggcgtagaggatctg
gctagcgatgaccctgctgattggttcgctgaccatttccgggtgcgggacggcgtta
ccagaaactcagaaggttcgtccaaccaaaccgactctgacggcagtttacgagagag
atgatagggtctgcttcagtaagccagatgctacacaattaggcttgtacatattgtc
gttagaacgcggctacaattaatacataaccttatgtatcatacacatacgatttagg
tgacactatagaatacacggaattaattc
```

# Figure 49

pAAV-CAG-106-3xHA-p38gamma(MAPK12)-ca

```
tagctgcgcgctcgctcgctcactgaggccgcccgggcaaagcccgggcgtcgggcga
cctttggtcgcccggcctcagtgagcgagcgagcgcgcagagagggagtggccaactc
catcactaggggttccttgtagttaatgattaacccgccatgctacttatctacgtag
ccatgctctaggtaccgggccccccctAgaggtAgacggtatcTTCCCATAGTAACGC
CAATAGGGACTTTCCATTGACGTCAATGGGTGGACTATTTACGGTAAACTGCCCACTT
GGCAGTACATCAAGTGTATCATATGCCAAGTACGCCCCCTATTGACGTCAATGACGGT
AAATGGCCCGCCTGGCATTATGCCCAGTACATGACCTTATGGGACTTTCCTACTTGGC
AGTACATCTACGTATTAGTCATCGCTATTACCATGGGTCGAGGTGAGCCCCACGTTCT
GCTTCACTCTCCCCATCTCCCCCCCCTCCCCACCCCCAATTTTGTATTTATTTATTTT
TTAATTATTTTGTGCAGCGATGGGGGCGGGGGGGGGGGGGCGCGCGCCAGGCGGGGC
GGGGCGGGGCGAGGGGCGGGGCGGGGCGAGGCGGAGAGGTGCGGCGGCAGCCAATCAG
AGCGGCGCGCTCCGAAAGTTTCCTTTTATGGCGAGGCGGCGGCGGCGGCGGCCCTATA
AAAAGCGAAGCGCGCGGCGGGCGGGAGTCGCTGCGTTGCCTTCGCCCCGTGCCCCGCT
CCGCGCCGCcTCCGCGCCCGCCGCCCCGGCTCTGACTGACCGCGTTACTCCCACAGGT
GAGCGGGCGGGACGGCCCTTCTCCTCCGGGCTGTAATTAGCGCTTGGTTTAATGACGG
CTTGTTTCTTTTCTGTGGCTGCGTGAAAGCCTTGAGGGGCTCCGGGAGGGCCCTTTGT
GCGGGGGGAGCGGCTCGGGGCTGTCCGCGGGGGGACGGCTGCCTTCGGGGGGGACGGG
GCAGGGCGGGGTTCGGCTTCTGGCGTGTGACCGGCGGCTCTAGAGCCTCTGCTAACCA
TGTTCATGCCTTCTTCTTTTTCCTACAGCTCCTGGGCAACGTGCTGGTTATTGTGCTG
TCTCATCATTTTGGCAAAGAATTGGATCCACTCGAGTGGAGCTCGCGACTAGTCGATT
CGAATTCGATagctTATGTACCCATACGATGTTCCAGATTACGCCATGTACCCATACG
ATGTTCCAGATTACGCCATGTACCCATACGATGTTCCAGATTACGCCATGGGGAGCTC
TCCGCCGCCCGCCCGCAGTGGCTTTTACCGCCAGGAGGTGACCAAGACGGCCTGGGAG
GTGCGCGCCGTGTACCGGGACCTGCAGCCCGTGGGCTCGGGCGCCTACGGCGCGGTGT
GCTCGGCCGTGGACGGCCGCACCGGCGCTAAGGTGGCCATCAAGAAGCTGTATCGGCC
CTTCCAGTCCGAGCTGTTCGCCAAGCGCGCCTACCGCGAGCTGCGCCTGCTCAAGCAC
ATGCGCCACGAGAACGTGATCGGGCTGCTGGACGTATTCACTCCTGATGAGACCCTGG
ATGACTTCACGGACTTTTACCTGGTGATGCCGTTCATGGGCACCGACCTGGGCAAGCT
CATGAAACATGAGAAGCTAGGCGAGGACCGGATCCAGTTCCTCGTGTACCAGATGCTG
AAGGGGCTGAGGTATATCCACGCTGCCGGCATCATCCACAGAGACCTGAAGCCCGGCA
ACCTGGCTGTGAACGAAGACTGTGAGCTGAAGATCCTGGACTTCGGCCTGGCCAGGCA
GGCAGCCAGTGAGATGACTGGGTACGTGGTGACCCGGTGGTACCGGGCTCCCGAGGTC
ATCTTGAATTGGATGCGCTACACGCAGACGGTGGACATCTGGTCTGTGGGCTGCATCA
TGGCGGAGATGATCACAGGCAAGACGCTGTTCAAGGGCAGCGACCACCTGGACCAGCT
GAAGGAGATCATGAAGGTGACGGGGACGCCTCCGGCTGAGTTTGTGCAGCGGCTGCAG
AGCGATGAGGCCAAGAACTACATGAAGGGCCTCCCCGAATTGGAGAAGAAGGATTTTG
CCTCTATCCTGACCAATGCAAGCCCTCTGGCTGTGAACCTCCTGGAGAAGATGCTGGT
GCTGGACGCGGAGCAGCGGGTGACGGCAGGCGAGGCGCTGGCCCATCCCTACTTCGAG
TCCCTGCACGACACGGAAGATGAGCCCCAGGTCCAGAAGTATGATGACTCCTTTGACG
ACGTTGACCGCACACTGGATGAATGGAAGCGTGTTACTTACAAAGAGGTGCTCAGCTT
CAAGCCTCCCCGGCAGCTGGGGGCCAGGGTCTCCAAGGAGACGCCTCTGTGATctagA
TCAAGCTTATCgataatcaacctctggattacaaaatttgtgaaagattgactggtat
tcttaactatgttgctcctttttacgctatgtggatacgctgctttaatgcctttgtat
catgctattgcttcccgtatggctttcattttctcctccttgtataaatcctggttgc
```

# Figure 49 cont.

```
tgtctctttatgaggagttgtggcccgttgtcaggcaacgtggcgtggtgtgcactgt
gtttgctgacgcaaccccccactggttggggcattgccaccacctgtcagctcctttcc
gggactttcgctttcccctccctattgccacggcggaactcatcgccgcctgccttg
cccgctgctggacaggggctcggctgttgggcactgacaattccgtggtgttgtcggg
gaaGCTGAcgtcctttccAtggctgctcgcctgtgttgccacctggattctgcgcggg
acgtccttctgctacgtcccttcggccctcaatccagcggaccttccttccgcggcc
tgctgccggctctgcggcctcttccgcgtcttcgccttcgccctcagacgagtcggat
ctcccttttgggccgcctccccgcatcgataccgtcgactcgctgatcagcctcgactg
tgccttctagttgccagccatctgttgtttgccctccccgtgccttccttgaccct
ggaaggtgccactcccactgtcctttcctaataaaatgaggaaattgcatcgcattgt
ctgagtaggtgtcattctattctgggggtggggtggggcaggacagcaagggggagg
attgggaagacaatagcaggcatgctggggatgcggtgggctctatggcttctgaggc
ggaaagaaccagctggggctcgactagagcatggctacgtagataagtagcatggcgg
gttaatcattaactacaaggaacccctagtgatggagttggccactccctctctgcgc
gctcgctcgctcactgaggccgggcgaccaaaggtcgcccgacgcccgggctttgccc
gggcggcctcagtgagcgagcgagcgcgcagagcttttttgcaaaagcctaggcctcca
aaaaagcctcctcactacttctggaatagctcagaggccgaggcggcctcggcctctg
cataaataaaaaaaattagtcagccatggggcggagaatgggcggaactgggcggagt
taggggcgggatgggcggagttaggggcgggactatggttgctgactaattgagatgc
atgctttgcatacttctgcctgctggggagcctggggactttccacacctggttgctg
actaattgagatgcatgctttgcatacttctgcctgctggggagcctggggactttcc
acaccctaactgacacacattccacagctgcattaatgaatcggccaacgcgcgggga
gaggcggtttgcgtattgggcgctcttccgcttcctcgctcactgactcgctgcgctc
ggtcgttcggctgcggcgagcggtatcagctcactcaaaggcggtaatacggttatcc
acagaatcaggggataacgcaggaaagaacatgtgagcaaaaggccagcaaaaggcca
ggaaccgtaaaaaggccgcgttgctggcgtttttccataggctccgccccctgacga
gcatcacaaaaatcgacgctcaagtcagaggtggcgaaacccgacaggactataaaga
taccaggcgtttccccctggaagctccctcgtgcgctctcctgttccgaccctgccgc
ttaccggataccgtccgccttctcccttcgggaagcgtggcgctttctcatagctc
acgctgtaggtatctcagttcggtgtaggtcgttcgctccaagctgggctgtgtgcac
gaaccccccgttcagcccgaccgctgcgccttatccggtaactatcgtcttgagtcca
acccggtaagacacgacttatcgccactggcagcagccactggtaacaggattagcag
agcgaggtatgtaggcggtgctacagagttcttgaagtggtggcctaactacggctac
actagaagaacagtatttggtatctgcgctctgctgaagccagttaccttcggaaaaa
gagttggtagctcttgatccggcaaacaaaccaccgctggtagcggtggtttttttgt
ttgcaagcagcagattacgcgcagaaaaaaaggatctcaagaagatcctttgatcttt
tctacggggtctgacgctcagtggaacgaaaactcacgttaagggattttggtcatga
gattatcaaaaaggatcttcacctagatccttttaaattaaaaatgaagttttaaatc
aatctaaagtatatatgagtaaacttggtctgacagttaccaatgcttaatcagtgag
gcacctatctcagcgatctgtctatttcgttcatccatagttgcctgactccccgtcg
tgtagataactacgatacgggagggcttaccatctggccccagtgctgcaatgatacc
gcgagacccacgctcaccggctccagatttatcagcaataaaccagccagccggaagg
gccgagcgcagaagtggtcctgcaactttatccgcctccatccagtctattaattgtt
gccgggaagctagagtaagtagttcgccagttaatagtttgcgcaacgttgttgccat
```

# Figure 49 cont.

```
tgctacaggcatcgtggtgtcacgctcgtcgtttggtatggcttcattcagctccggt
tcccaacgatcaaggcgagttacatgatcccccatgttgtgcaaaaaagcggttagct
ccttcggtcctccgatcgttgtcagaagtaagttggccgcagtgttatcactcatggt
tatggcagcactgcataattctcttactgtcatgccatccgtaagatgcttttctgtg
actggtgagtactcaaccaagtcattctgagaatagtgtatgcggcgaccgagttgct
cttgcccggcgtcaatacgggataataccgcgccacatagcagaactttaaaagtgct
catcattggaaaacgttcttcggggcgaaaactctcaaggatcttaccgctgttgaga
tccagttcgatgtaacccactcgtgcacccaactgatcttcagcatctttttactttca
ccagcgtttctgggtgagcaaaaacaggaaggcaaaatgccgcaaaaaagggaataag
ggcgacacggaaatgttgaatactcatactcttccttttttcaatattattgaagcatt
tatcagggttattgtctcatgagcggatacatatttgaatgtatttagaaaaataaac
aaataggggttccgcgcacatttccccgaaaagtgccacctgacgtctaagaaaccat
tattatcatgacattaacctataaaaataggcgtatcacgaggccctttcgtctcgcg
cgtttcggtgatgacggtgaaaacctctgacacatgcagctcccggagacggtcacag
cttgtctgtaagcggatgccgggagcagacaagcccgtcagggcgcgtcagcgggtgt
tggcgggtgtcggggctggcttaactatgcggcatcagagcagattgtactgagagtg
caccattcgacgctctcccttatgcgactcctgcattaggaagcagcccagtagtagg
ttgaggccgttgagcaccgccgccgcaaggaatggtgcatgcaaggagatggcgccca
acagtcccccggccacggggcctgccaccatacccacgccgaaacaagcgctcatgag
cccgaagtggcgagcccgatcttccccatcggtgatgtcggcgatataggcgccagca
accgcacctgtggcgccggtgatgccggccacgatgcgtccggcgtagaggatctggc
tagcgatgaccctgctgattggttcgctgaccatttccgggtgcgggacggcgttacc
agaaactcagaaggttcgtccaaccaaaccgactctgacggcagtttacgagagagat
gataggggtctgcttcagtaagccagatgctacacaattaggcttgtacatattgtcgt
tagaacgcggctacaattaatacataaccttatgtatcatacacatacgatttaggtg
acactatagaatacacggaattaattc
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160038613 A

- US 5264221 A

### Non-patent literature cited in the description

- **SAMBROOK et al.** Molecular Cloning: A Laboratory Manual. Cold Spring Harbor, 2012, vol. 1-3
- **GRIEGER ; SAMULSKI.** Adeno-associated virus as a gene therapy vector: vector development, production and clinical applications. *Advances in Biochemical Engineering/Biotechnology,* 2005, vol. 99, 119-145
- **HARASTA et al.** *Neuropsychopharmacology,* 2015, vol. 40, 1969-1978
- **NALDINI et al.** In vivo gene delivery and stable transduction of nondividing cells by a lentiviral vector. *Science,* 1996, vol. 272, 263-267
- **LOIS et al.** Germline transmission and tissue-specific expression of transgenes delivered by lentiviral vectors. *Science,* 2002, vol. 295, 868-872
- **VOGEL et al.** A single lentivirus vector mediates doxycycline-regulated expression of transgenes in the brain. *Hum Gene Ther.,* 2004, vol. 15 (2), 157-165
- **KOZARSKY ; WILSON.** *Current Opinion in Genetics and Development,* 1993, vol. 3, 499-503
- Gene transfer into neural cells in vitro using adenoviral vectors. **SOUTHGATE et al.** Current Protocols in Neuroscience. 2008
- **AKLI et al.** Transfer of a foreign gene into the brain using adenovirus vectors. *Nature genetics,* 1993, vol. 3 (3), 224-228
- Remington's Pharmaceutical Science. Mack Publishing Company, 1985
- **GOODMAN ; GILLMAN'S.** The Pharmacological Basis of Therapeutics. McGraw-Hill Professional, 2005
- **C STURCHLER-PIERRAT et al.** *Proc Natl Acad Sci U S A,* 1997, vol. 94, 13287-92
- **A PROBST et al.** *Acta Neuropathol,* 2000, vol. 99, 469-81
- **I DEWACHTER et al.** *J Neurosci,* 2002, vol. 22, 3445-53
- **KL TUCKER ; M MEYER ; YA BARDE.** *Nat Neurosci,* 2001, vol. 4, 29-37
- **FB ENGEL et al.** *Genes Dev,* 2005, vol. 19, 1175-87
- **AR POGOZELSKI et al.** *PLoS One,* 2009, vol. 4, e7934

- **G SUMARA et al.** *Cell,* 2009, vol. 136, 235-48
- **ITTNER et al.** *Proc. Natl. Acad. Sci. U.S.A.,* 2008, vol. 105, 15997-16002
- **GIBSON et al.** *Nat. Methods,* 2009, vol. 6, 343-345
- **ITTNER et al.** *Nat. Protoc.,* 2007, vol. 2, 1206-1215
- **LM ITTNER et al.** *Cell,* 2010, vol. 142, 387-97
- **CV VORHEES ; MT WILLIAMS.** *Nat Protoc,* 2006, vol. 1, 848-58
- **HORNER et al.** *Nat. Protoc.,* 2013, vol. 8, 1961-1984
- **KE et al.** *Acta Neuropathol.,* 2015, vol. 130, 661-678
- **VAN EERSEL et al.** *Neuropathol. Appl. Neurobiol.,* 2015, vol. 41, 906-925
- **AA ITTNER ; A GLADBACH ; J BERTZ ; LS SUH ; LM ITTNER.** *Acta Neuropathol Commun,* 2014, vol. 2, 149
- **M WEIERGRABER ; M HENRY ; J HESCHELER ; N SMYTH ; T SCHNEIDER.** *Brain Res Brain Res Protoc,* 2005, vol. 14, 154-64
- **AB TORT ; R KOMOROWSKI ; H EICHENBAUM ; N KOPELL.** *J Neurophysiol,* 2010, vol. 104, 1195-210
- **ITTNER et al.** *Cell,* 2010, vol. 142, 387-397
- **BRYKSINET.** *Biotechniques,* 2010, vol. 48, 463-465
- **M AVITZOUR et al.** *FEBS J,* 2007, vol. 274, 963-75
- **VON JONQUIERES et al.** *PLOS ONE,* 2013, vol. 8, e65646
- **AE HARASTA et al.** *Neuropsychopharmacology,* 2015, vol. 40, 1969-78
- **G VON JONQUIERES et al.** *PLoS One,* 2013, vol. 8, e65646
- **T FATH ; YD KE ; P GUNNING ; J GOTZ ; LM ITTNER.** *Nat Protoc,* 2009, vol. 4, 78-85
- **A ITTNER et al.** *J Exp Med,* 2012, vol. 209, 2229-46
- **WIENKEN et al.** *Nat. Commun.,* 2010, vol. 1, 100

- **DOLAI et al.** *Cancer Res.,* 2016, vol. 76, 2766-2777
- **THINGHOLM et al.** *Nat. Protoc.,* 2006, vol. 1, 1929-1935
- **MP LAMBERT et al.** *Proc Natl Acad Sci U S A,* 1998, vol. 95, 6448-53
- **ITTNER et al.** *Acta Neuropathol. Commun.,* 2014, vol. 2, 149
- **STURCHLER-PIERRAT et al.** *Proc. Natl. Acad. Sci. U.S.A.,* 1997, vol. 94, 13287-13292
- **C BALLATORE ; VM LEE ; JQ TROJANOWSKI.** Nature reviews. *Neuroscience,* 2007, vol. 8, 663-72
- **C HAASS ; DJ SELKOE.** Nature reviews. *Molecular cell biology,* 2007, vol. 8, 101-12
- **K IQBAL ; F LIU ; CX GONG ; C ALONSO ADEL ; I GRUNDKE-IQBAL.** *Acta Neuropathol,* 2009, vol. 118, 53-69
- **EM MANDELKOW ; E MANDELKOW.** *Cold Spring Harb Perspect Med,* 2012, vol. 2, a006247
- **ES MUSIEK ; DM HOLTZMAN.** *Nat Neurosci,* 2015, vol. 18, 800-6
- **M RAPOPORT ; HN DAWSON ; LI BINDER ; MP VITEK ; A FERREIRA.** *Proc Natl Acad Sci USA,* 2002, vol. 99, 6364-9
- **ED ROBERSON et al.** *Science,* 2007, vol. 316, 750-4
- **L MUCKE ; DJ SELKOE.** *Cold Spring Harb Perspect Med,* 2012, vol. 2, a006338
- **JJ PALOP ; L MUCKE.** *Nat Neurosci,* 2010, vol. 13, 812-8
- **GE HARDINGHAM ; H BADING.** Nature reviews. *Neuroscience,* 2010, vol. 11, 682-96
- **Q WANG ; DM WALSH ; MJ ROWAN ; DJ SELKOE ; R ANWYL.** *J Neurosci,* 2004, vol. 24, 3370-8
- **S LI et al.** *J Neurosci,* 2011, vol. 31, 6627-38
- **MA FABIAN et al.** *Nat Biotechnol,* 2005, vol. 23, 329-36
- **MB MENON ; S DHAMIJA ; A KOTLYAROV ; M GAESTEL.** *Autophagy,* 2015, vol. 0
- **G BUZSAKI ; EI MOSER.** *Nat Neurosci,* 2013, vol. 16, 130-8
- **R GOUTAGNY ; J JACKSON ; S WILLIAMS.** *Nat Neurosci,* 2009, vol. 12, 1491-3
- **RT CANOLTY et al.** *Science,* 2006, vol. 313, 1626-8
- **AB TORT ; RW KOMOROWSKI ; JR MANNS ; NJ KOPELL ; H EICHENBAUM.** *Proc Natl Acad Sci USA,* 2009, vol. 106, 20942-7
- **Y RONG ; X LU ; A BERNARD ; M KHRESTCHATISKY ; M BAUDRY.** *J Neurochem,* 2001, vol. 79, 382-90
- **M AARTS et al.** *Science,* 2002, vol. 298, 846-50
- **M GOEDERT et al.** *FEES Lett,* 1997, vol. 409, 57-62
- **S MONDRAGON-RODRIGUEZ et al.** *J Biol Chem,* 2012, vol. 287, 32040-53
- **LM ITTNER ; J GOTZ.** Nature reviews. *Neuroscience,* 2011, vol. 12, 65-72
- **JZ WANG ; Q WU ; A SMITH ; I GRUNDKE-IQBAL ; K IQBAL.** *FEBS Lett,* 1998, vol. 436, 28-34
- **V BUEE-SCHERRER ; M GOEDERT.** *FEBS Lett,* 2002, vol. 515, 151-4
- **A CAVALLINI et al.** *J Biol Chem,* 2013, vol. 288, 23331-47